(19) Europäisches Patentamt
European Patent Office
Office européen des brevets

(11) **EP 4 238 498 B1**

(12) **EUROPEAN PATENT SPECIFICATION**

(45) Date of publication and mention
of the grant of the patent:
**22.10.2025  Bulletin 2025/43**

(21) Application number: **21917602.1**

(22) Date of filing: **02.12.2021**

(51) International Patent Classification (IPC):
***A61B 5/349*** (2021.01)     ***A61B 5/352*** (2021.01)
***A61B 5/355*** (2021.01)     ***A61B 5/366*** (2021.01)
***A61B 5/00*** (2006.01)

(52) Cooperative Patent Classification (CPC):
**A61B 5/352; A61B 5/355; A61B 5/366;**
A61B 5/7253

(86) International application number:
**PCT/JP2021/044316**

(87) International publication number:
**WO 2022/149381 (14.07.2022 Gazette 2022/28)**

(54) **SIGNAL ANALYSIS DEVICE, SIGNAL ANALYSIS METHOD, AND PROGRAM**

SIGNALANALYSEVORRICHTUNG, SIGNALANALYSEVERFAHREN UND PROGRAMM

DISPOSITIF D'ANALYSE DE SIGNAL, PROCÉDÉ D'ANALYSE DE SIGNAL ET PROGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.01.2021  PCT/JP2021/000209**
**09.09.2021  PCT/JP2021/033138**

(43) Date of publication of application:
**06.09.2023  Bulletin 2023/36**

(73) Proprietor: **NTT, Inc.**
**Tokyo 100-8116 (JP)**

(72) Inventor: **TSUKADA, Shingo**
**Musashino-shi, Tokyo 180-8585 (JP)**

(74) Representative: **Brevalex**
**Tour Trinity**
**1 B Place de la Défense**
**92400 Courbevoie (FR)**

(56) References cited:
**JP-A- 2016 533 231     US-A1- 2012 265 086
US-A1- 2012 302 903**

• **BERGELT JULIA, HAMKER FRED H: "Spatial
updating of attention across eye movements: A
neuro-computational approach", JOURNAL OF
VISION, ASSOCIATION FOR RESEARCH IN
VISION AND OPHTHALMOLOGY, US, vol. 19, no.
7, 1 July 2019 (2019-07-01), US
, pages 1 - 23, XP055949178, ISSN: 1534-7362,
DOI: 10.1167/19.7.10**
• **MOHAMMAD SAAD BILLAH ; TAHMIDA BINTE
MAHMUD ; FARHANA SHARMIN SNIGDHA ;
MUHAMMAD ABDULLAH ARAFAT: "A novel
method to model ECG beats using Gaussian
functions", BIOMEDICAL ENGINEERING AND
INFORMATICS (BMEI), 2011 4TH
INTERNATIONAL CONFERENCE ON, IEEE, 15
October 2011 (2011-10-15), pages 612 - 616,
XP032071565, ISBN: 978-1-4244-9351-7, DOI:
10.1109/BMEI.2011.6098409**
• **KARCZEWICZ, M. GABBOUJ, M.: "ECG data
compression by spline approximation", SIGNAL
PROCESSING, vol. 59, no. 1, 1 May 1997
(1997-05-01), pages 43 - 59, XP004075302, ISSN:
0165-1684, DOI: 10.1016/S0165-1684(97)00037-6**
• **DÖSSEL OLAF, LUONGO GIORGIO, NAGEL
CLAUDIA, LOEWE AXEL: "Computer Modeling of
the Heart for ECG Interpretation—A Review",
HEARTS, vol. 2, no. 3, pages 350 - 368,
XP055949184, DOI: 10.3390/hearts2030028**

## Description

TECHNICAL FIELD

The present invention relates to a signal analyzing apparatus, a signal analyzing method, and a program.

The present application claims priority based on PCT/JP2021/000209 filed on January 6, 2021 and PCT/JP2021/033138 filed on September 9, 2021.

Background Art

US 2012/302903 relates to a system for heart performance characterization and abnormality detection involving determining distribution data associated with degree of similarity between first and second signal portions of a heart activity related signal.

An electrocardiogram is useful information for grasping the state of a heart, and for example, if an electrocardiogram is used, whether the target is in a state in which there is a high possibility that heart failure occurs can be determined (Non Patent Document 1).

## CITATION LIST

Non Patent Document

Non Patent Document 1: Hiroshi Tanaka, "On the Inverse Solution of Electrocardiology", Medical Electronics and Biological Engineering, 1985, Vol. 23, No. 3 p.147-158

## SUMMARY

## TECHNICAL PROBLEM

However, a waveform of an electrocardiogram may not necessarily be sufficient for grasping the state of a heart. For example, even if waveforms of electrocardiograms are similar, manners of the onset of a disease related to the heart may be different. As described above, grasping the state of a heart only by viewing a waveform itself of an electrocardiogram may be difficult depending on the disease. For example, in a case of heart failure, the onset can be reduced by the state of a heart being observed using a waveform of an electrocardiogram in daily life. In order to further increase the accuracy of reducing the onset of a disease, it is conceivable to acquire other information using another technique such as collecting blood, but it is not practical to do so in daily life. Therefore, depending on the disease, grasping the state of a heart substantially on the basis of only a waveform of an electrocardiogram may be necessary.

Such circumstances are not limited to the case of grasping the state of a heart on the basis of a waveform of an electrocardiogram. Such circumstances are also common in a case of grasping the state of a heart on the basis of time-series biological information of one channel related to pulsation acquired by a sensor in contact with the body surface, a sensor close to the body surface, a sensor inserted into the body, a sensor embedded in the body, or the like. Note that the time-series biological information related to pulsation of a heart is, for example, a waveform indicating a change in cardiac potential, a waveform indicating a change in pressure of a heart, a waveform indicating a change in blood flow rate, and a waveform indicating a change in heart sound. Note that a waveform of an electrocardiogram is also an example of the time-series biological information related to pulsation of a heart.

In view of the above circumstances, an object of the present disclosure is to provide a technology for obtaining useful information for grasping the state of a heart from time-series biological information of one channel related to pulsation of the heart.

## SOLUTION TO PROBLEM

The invention is defined by the appended claims. An aspect of the present invention is a signal analysis device including a biological information acquisition unit configured to acquire a waveform of a time section of an R wave included in a waveform for one cycle indicating a cardiac cycle of a heart of an analysis target as a target time waveform, and an analysis unit, upon approximating the target time waveform by an approximate time waveform that is a time waveform obtained by a difference or a weighted difference between a first cumulative distribution function that is a cumulative distribution function of a first unimodal distribution and a second cumulative distribution function that is a cumulative distribution function of a second unimodal distribution, or upon approximating the target time waveform by an approximate time waveform that is a time waveform obtained by adding a level value to a difference or a weighted difference between a first cumulative distribution function that is a cumulative distribution function of a first unimodal distribution and a second cumulative distribution function that is a cumulative distribution function of a second unimodal distribution, configured to acquire at least one of at least some parameters of parameters identifying the first unimodal distribution, at least some parameters of parameters identifying the first cumulative distribution function, at least some parameters of parameters identifying the second unimodal distribution, or at least some parameters of parameters identifying the second cumulative distribution function as a parameter indicating activity of a myocardium of the heart.

As a result, a technology for obtaining useful information for grasping the state of a heart from time-series biological information of one channel related to pulsation of the heart can be provided.

An aspect of the present invention is a signal analysis device including a biological information acquisition unit configured to acquire a waveform of a time section of a T wave included in a waveform for one cycle indicating a cardiac cycle of a heart of an analysis target as a target time waveform, and an analysis unit, assuming a waveform obtained by reversing a time axis of the target time waveform as a target inverse time waveform, upon approximating the target inverse time waveform by a waveform obtained by a difference or a weighted difference between a third cumulative distribution function that is a cumulative distribution function of a third unimodal distribution and a fourth cumulative distribution function that is a cumulative distribution function of a fourth unimodal distribution, or upon approximating the target inverse time waveform by a waveform obtained by adding a level value to a difference or a weighted difference between a third cumulative distribution function that is a cumulative distribution function of a third unimodal distribution and a fourth cumulative distribution function that is a cumulative distribution function of a fourth unimodal distribution, configured to acquire at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

An aspect of the present invention is a signal analysis device including a biological information acquisition unit configured to acquire a waveform of a time section of a T wave included in a waveform for one cycle indicating a cardiac cycle of a heart of an analysis target as a target time waveform, and an analysis unit, assuming a cumulative distribution function of a third unimodal distribution as a third cumulative distribution function, a cumulative distribution function of a fourth unimodal distribution as a fourth cumulative distribution function, a function obtained by subtracting the third cumulative distribution function from 1 as a third inverse cumulative distribution function, and a function obtained by subtracting the fourth cumulative distribution function from 1 as a fourth inverse cumulative distribution function, upon approximating the target time waveform by an approximate time waveform that is a time waveform obtained by a difference or a weighted difference between the third inverse cumulative distribution function and the fourth inverse cumulative distribution function, or upon approximating the target time waveform by an approximate time waveform that is a time waveform obtained by adding a level value to a difference or a weighted difference between the third inverse cumulative distribution function and the fourth inverse cumulative distribution function, configured to acquire at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

An aspect of the present invention is a signal analysis method including a biological information acquisition step for acquiring a waveform of a time section of an R wave included in a waveform for one cycle indicating a cardiac cycle of a heart of an analysis target as a target time waveform, and an analysis step for, upon approximating the target time waveform by an approximate time waveform that is a time waveform obtained by a difference or a weighted difference between a first cumulative distribution function that is a cumulative distribution function of a first unimodal distribution and a second cumulative distribution function that is a cumulative distribution function of a second unimodal distribution, or upon approximating the target time waveform by an approximate time waveform that is a time waveform obtained by adding a level value to a difference or a weighted difference between a first cumulative distribution function that is a cumulative distribution function of a first unimodal distribution and a second cumulative distribution function that is a cumulative distribution function of a second unimodal distribution, acquiring at least one of at least some parameters of parameters identifying the first unimodal distribution, at least some parameters of parameters identifying the first cumulative distribution function, at least some parameters of parameters identifying the second unimodal distribution, or at least some parameters of parameters identifying the second cumulative distribution function as a parameter indicating activity of a myocardium of the heart.

As a result, a technology for obtaining useful information for grasping the state of a heart from time-series biological information of one channel related to pulsation of the heart can be provided.

An aspect of the present invention is a signal analysis method including a biological information acquisition step for acquiring a waveform of a time section of a T wave included in a waveform for one cycle indicating a cardiac cycle of a heart of an analysis target as a target time waveform, and an analysis step for setting a waveform obtained by reversing a time axis of the target time waveform as a target inverse time waveform and, upon approximating the target inverse time waveform by a waveform obtained by a difference or a weighted difference between a third cumulative distribution function that is a cumulative distribution function of a third unimodal distribution and a fourth cumulative distribution function that is a cumulative distribution function of a fourth unimodal distribution, or upon approximating the target inverse time waveform by a waveform obtained by adding a level value to a difference or a weighted difference between a third cumulative distribution function that is a cumulative distribution function of a third unimodal distribution and a fourth cumulative distribution function that is a cumulative distribution function of a fourth unimodal distribution, acquiring at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

An aspect of the present invention is a signal analysis method including a biological information acquisition step for acquiring a waveform of a time section of a T wave included in a waveform for one cycle indicating a cardiac cycle of a heart of an analysis target as a target time waveform, and an analysis step for setting a cumulative distribution function of a third unimodal distribution as a third cumulative distribution function, a cumulative distribution function of a fourth unimodal distribution as a fourth cumulative distribution function, a function obtained by subtracting the third cumulative distribution function from 1 as a third inverse cumulative distribution function, and a function obtained by subtracting the fourth cumulative distribution function from 1 as a fourth inverse cumulative distribution function and, upon approximating the target time waveform by an approximate time waveform that is a time waveform obtained by a difference or a weighted difference between the third inverse cumulative distribution function and the fourth inverse cumulative distribution function, or upon approximating the target time waveform by an approximate time waveform that is a time waveform obtained by adding a level value to a difference or a weighted difference between the third inverse cumulative distribution function and the fourth inverse cumulative distribution function, acquiring at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

An aspect of the present invention is a computer program comprising instructions which, when the computer program is executed by a computer, cause the computer to carry out the above-described signal analysis methods.

As a result, a technology for obtaining useful information for grasping the state of a heart from time-series biological information of one channel related to pulsation of the heart can be provided.

Therefore, a computer program for obtaining useful information for grasping the state of a heart from time-series biological information of one channel related to pulsation of the heart can be provided.

## ADVANTAGEOUS EFFECTS OF INVENTION

According to the present invention, a technology for obtaining useful information for grasping the state of a heart from time-series biological information of one channel related to pulsation of the heart can be provided.

## BRIEF DESCRIPTION OF DRAWINGS

Fig. 1 is a diagram illustrating an example of a hardware configuration of a signal analysis device 1 according to an embodiment.

Fig. 2 is a diagram schematically illustrating a function obtained by multiplying a first cumulative distribution function by weight, a function obtained by multiplying a second cumulative distribution function by weight, and an approximate time waveform that is a weighted difference between the first cumulative distribution function and the second cumulative distribution function for a first target time waveform.

Fig. 3 is a diagram schematically illustrating a function obtained by multiplying a third inverse cumulative distribution function by weight, a function obtained by multiplying a fourth inverse cumulative distribution function by weight, and an approximate time waveform that is a weighted difference between the third inverse cumulative distribution function and the fourth inverse cumulative distribution function for a second target time waveform.

Fig. 4 is a diagram illustrating an example of results of fitting a waveform of an electrocardiogram of a target heart by four cumulative distribution functions according to the embodiment.

Fig. 5 is an explanatory diagram for describing that a difference between two cumulative distribution functions in the embodiment can be fitted to substantially the same waveform as a falling waveform of a T wave.

Fig. 6 is a diagram schematically illustrating a function obtained by multiplying a first cumulative distribution function by weight and adding a level value, a function obtained by multiplying a second cumulative distribution function by weight and adding a level value, and an approximate time waveform obtained by adding a level value to a weighted difference between a first cumulative distribution function and a second cumulative distribution function for a first target time waveform.

Fig. 7 is a diagram schematically illustrating a function obtained by multiplying a third inverse cumulative distribution function by weight and adding a level value, a function obtained by multiplying a fourth inverse cumulative distribution function by weight and adding a level value, and an approximate time waveform obtained by adding a level value to a weighted difference between a third inverse cumulative distribution function and a fourth inverse cumulative distribution function for a second target time waveform.

Fig. 8 is a diagram schematically illustrating a Δ wave included in a target time waveform.

Fig. 9 is a diagram illustrating an example of a functional configuration of a control unit 11 according to the embodiment.

Fig. 10 is a flowchart illustrating an example of a flow of processing performed by the signal analysis device 1 according to the embodiment.

Fig. 11 is a first diagram illustrating an example of analysis results of the signal analysis device 1 according to the embodiment.

Fig. 12 is a second diagram illustrating an example of analysis results of the signal analysis device 1 according to the embodiment.

Fig. 13 is a third diagram illustrating an example of analysis results of the signal analysis device 1 according to the embodiment.

Fig. 14 is a fourth diagram illustrating an example of analysis results of the signal analysis device 1 according to the embodiment.

Fig. 15 is a first explanatory diagram of an example in which an electrocardiogram of a ventricular premature contraction is analyzed by the signal analysis device 1 of the embodiment.

Fig. 16 is a second explanatory diagram of the example in which the electrocardiogram of the ventricular premature contraction is analyzed by the signal analysis device 1 of the embodiment.

Fig. 17 is a third explanatory diagram of the example in which the electrocardiogram of the ventricular premature contraction is analyzed by the signal analysis device 1 of the embodiment.

Fig. 18 is a first explanatory diagram in which an electrocardiogram of a target heart in a depolarization period of Brugada syndrome type 1 is analyzed by the signal analysis device 1 according to the embodiment.

Fig. 19 is a second explanatory diagram in which the electrocardiogram of the target heart in the depolarization period of Brugada syndrome type 1 is analyzed by the signal analysis device 1 according to the embodiment.

Fig. 20 is a third explanatory diagram in which the electrocardiogram of the target heart in the depolarization period of Brugada syndrome type 1 is analyzed by the signal analysis device 1 according to the embodiment.

Fig. 21 is a diagram illustrating a first example in which an electrocardiogram is analyzed by the signal analysis device 1 according to the embodiment.

Fig. 22 is a diagram illustrating a second example in which an electrocardiogram is analyzed by the signal analysis device 1 according to the embodiment.

Fig. 23 is a diagram illustrating a third example in which an electrocardiogram is analyzed by the signal analysis device 1 according to the embodiment.

Fig. 24 is a diagram illustrating a fourth example in which an electrocardiogram is analyzed by the signal analysis device 1 according to the embodiment.

Fig. 25 is a diagram illustrating a fifth example in which an electrocardiogram is analyzed by the signal analysis device 1 according to the embodiment.

Fig. 26 is a diagram illustrating a sixth example in which an electrocardiogram is analyzed by the signal analysis device 1 according to the embodiment.

Fig. 27 is a diagram illustrating a seventh example in which an electrocardiogram is analyzed by the signal analysis device 1 according to the embodiment.

Fig. 28 is a diagram illustrating an eighth example in which an electrocardiogram is analyzed by the signal analysis device 1 according to the embodiment.

Fig. 29 is a diagram illustrating a ninth example in which an electrocardiogram is analyzed by the signal analysis device 1 according to the embodiment.

Fig. 30 is a diagram illustrating a tenth example in which an electrocardiogram is analyzed by the signal analysis device 1 according to the embodiment.

Fig. 31 is a diagram illustrating an eleventh example in which an electrocardiogram is analyzed by the signal analysis device 1 according to the embodiment.

Fig. 32 is a diagram illustrating a twelfth example in which an electrocardiogram is analyzed by the signal analysis device 1 according to the embodiment.

Fig. 33 is a diagram illustrating a thirteenth example in which an electrocardiogram is analyzed by the signal analysis device 1 according to the embodiment.

Fig. 34 is a diagram illustrating a fourteenth example in which an electrocardiogram is analyzed by the signal analysis device 1 according to the embodiment.

Fig. 35 is a diagram illustrating a fifteenth example in which an electrocardiogram is analyzed by the signal analysis device 1 according to the embodiment.

Fig. 36 is a diagram illustrating a sixteenth example in which an electrocardiogram is analyzed by the signal analysis device 1 according to the embodiment.

Fig. 37 is a diagram illustrating a seventeenth example in which an electrocardiogram is analyzed by the signal analysis device 1 according to the embodiment.

Fig. 38 is a diagram illustrating an eighteenth example in which an electrocardiogram is analyzed by the signal analysis device 1 according to the embodiment.

Fig. 39 is a diagram illustrating a nineteenth example in which an electrocardiogram is analyzed by the signal analysis device 1 according to the embodiment.

Fig. 40 is a diagram illustrating a twentieth example in which an electrocardiogram is analyzed by the signal analysis device 1 according to the embodiment.

Fig. 41 is a diagram illustrating a twenty-first example in which an electrocardiogram is analyzed by the signal analysis device 1 according to the embodiment.

Fig. 42 is a diagram illustrating a twenty-second example in which an electrocardiogram is analyzed by the signal analysis device 1 according to the embodiment.

Fig. 43 is a diagram illustrating a twenty-third example in which an electrocardiogram is analyzed by the signal analysis device 1 according to the embodiment.

Fig. 44 is a diagram illustrating a twenty-fourth example in which an electrocardiogram is analyzed by the signal analysis device 1 according to the embodiment.

Fig. 45 is a diagram illustrating a twenty-fifth example in which an electrocardiogram is analyzed by the signal analysis device 1 according to the embodiment.

Fig. 46 is a diagram illustrating a twenty-sixth example in which an electrocardiogram is analyzed by the signal analysis device 1 according to the embodiment.

Fig. 47 is a diagram illustrating a twenty-seventh example in which an electrocardiogram is analyzed by the signal analysis device 1 according to the embodiment.

Fig. 48 is a diagram illustrating a twenty-eighth example in which an electrocardiogram is analyzed by the signal analysis device 1 according to the embodiment.

Fig. 49 is a diagram illustrating a twenty-ninth example in which an electrocardiogram is analyzed by the signal analysis device 1 according to the embodiment.

Fig. 50 is a diagram illustrating a thirtieth example in which an electrocardiogram is analyzed by the signal analysis device 1 according to the embodiment.

Fig. 51 is a diagram illustrating a thirty-first example in which an electrocardiogram is analyzed by the signal analysis device 1 according to the embodiment.

Fig. 52 is a diagram illustrating a thirty-second example in which an electrocardiogram is analyzed by the signal analysis device 1 according to the embodiment.

Fig. 53 is a diagram illustrating a thirty-third example in which an electrocardiogram is analyzed by the signal analysis device 1 according to the embodiment.

Fig. 54 is a diagram illustrating a thirty-fourth example in which an electrocardiogram is analyzed by the signal analysis device 1 according to the embodiment.

Fig. 55 is a diagram illustrating a thirty-fifth example in which an electrocardiogram is analyzed by the signal analysis device 1 according to the embodiment.

Fig. 56 is a diagram illustrating a thirty-sixth example in which an electrocardiogram is analyzed by the signal analysis device 1 according to the embodiment.

Fig. 57 is a diagram illustrating a thirty-seventh example in which an electrocardiogram is analyzed by the signal analysis device 1 according to the embodiment.

Fig. 58 is a diagram illustrating a thirty-eighth example in which an electrocardiogram is analyzed by the signal analysis device 1 according to the embodiment.

Fig. 59 is a diagram illustrating a thirty-ninth example in which an electrocardiogram is analyzed by the signal analysis device 1 according to the embodiment.

## DESCRIPTION OF EMBODIMENTS

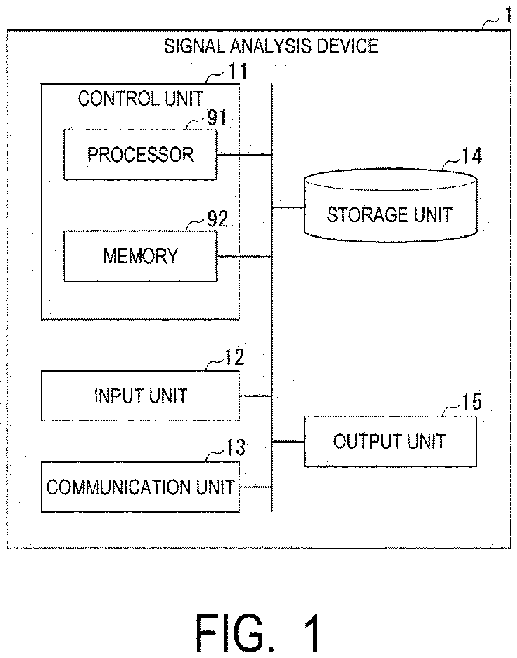

Fig. 1 is a diagram illustrating an example of a hardware configuration of a signal analysis device 1 according to an embodiment. Hereinafter, for simplicity of description, the signal analysis device 1 will be described by a case where analysis is performed on the basis of a waveform of one channel of an electrocardiogram being exemplified. However, the signal analysis device 1 can perform similar analysis on the basis of not only a waveform of an electrocardiogram but also time-series biological information related to pulsation of a heart. Note that the time-series biological information related to pulsation of a heart is, for example, a waveform indicating a change in cardiac potential, a waveform indicating a change in pressure of a heart, a waveform indicating a change in blood flow rate, and a waveform indicating a change in heart sound. Therefore, the signal analysis device 1 may use not only a waveform of an electric signal acquired from a body surface but also any waveform as long as the waveform indicates a cardiac cycle acquired from any point regardless of whether the point is on the body surface or in the body using a sensor in contact with the body surface, a sensor close to the body surface, a sensor inserted into the body, a sensor embedded in the body, or the like.

That is, the signal analysis device 1 may use a waveform indicating a change in pressure of a heart as the time-series biological information related to pulsation of a heart instead of a waveform of an electrocardiogram. Furthermore, the signal analysis device 1 may use a waveform indicating a change in blood flow rate as the time-series biological information related to pulsation of a heart instead of a waveform of an electrocardiogram. Furthermore, the signal analysis device 1 may use a waveform indicating a change in heart sound as the time-series biological information related to pulsation of a heart instead of a waveform of an electrocardiogram. Note that a waveform of an electrocardiogram is also an example of the time-series biological information related to pulsation of a heart. Note that the time-series biological information related to pulsation of a heart may be time-series biological information related to cyclical pulsation of a heart.

The signal analysis device 1 acquires a waveform of an electrocardiogram of a heart that is an analysis target (hereinafter, referred to as a "target heart"). On the basis of the acquired waveform of the electrocardiogram, the signal analysis device 1 acquires a parameter indicating activity of a myocardium of the target heart (hereinafter referred to as a "myocardial activity parameter") including at least one of a parameter indicating activity of an outer layer of the myocardium (hereinafter, referred to as a "myocardial outer layer") of the target heart (hereinafter, the parameter is referred to as a "myocardial outer layer parameter") or a parameter indicating activity of an inner layer of the myocardium (hereinafter, referred to as a "myocardial inner layer") of the target heart (hereinafter, the parameter is referred to as a "myocardial inner layer parameter").

Here, a relationship between activity of a myocardium and a waveform of an electrocardiogram will be described. In the medical field, a model for describing a relationship between movement of a myocardium and an electrocardiogram called an electromotive force dipole model (Reference Document 1) is known. According to the electromotive force dipole model, a myocardium is modeled using two layers of a myocardial outer layer and a myocardial inner layer.

Reference Document 1: Yoshifumi Tanaka, "Understand from the constitution, electrocardiogram waveform, reading activity potential of myocardium", Gakken Medical Shujunsha Co., Ltd. (2012)

In the electromotive force dipole model, the myocardial outer layer and the myocardial inner layer are modeled as different electromotive force generation sources. According to the electromotive force dipole model, a composite wave of an epicardial myocardial activity potential and an endocardial myocardial activity potential substantially corresponds to a temporal change in potential of a body surface observed on the body surface. A graph representing the temporal change in potential of a body surface is a waveform of an electrocardiogram. The epicardial myocardial activity potential is a result obtained by directly measuring a change in electromotive force generated by pulsation of a myocardial outer layer by a catheter electrode being inserted. The endocardial myocardial activity potential is a result obtained by directly measuring a change in electromotive force generated by pulsation of a myocardial inner layer by a catheter electrode being inserted. The above is a schematic description of the electromotive force dipole model.

Meanwhile, the myocardial outer layer in the electromotive force dipole model is a population of cells. Therefore, pulsation timing of cells in a myocardial outer layer in one-time pulsation of the myocardial outer layer is not necessarily the same in all the cells, and there is a possibility that there is a distribution in the pulsation timing. The same applies to a myocardial inner layer. That is, pulsation timing of cells in a myocardial inner layer in one-time pulsation of the myocardial inner layer is not necessarily the same in all the cells, and there is a possibility that there is a distribution in the pulsation timing. However, such a possibility that there is a distribution in pulsation timing of cells is not assumed in the electromotive force dipole model.

Furthermore, there is also a distribution in a distance between each cell and an electrode on a body surface, and a configuration of body tissue between the each cell and the electrode on the body surface is not the same. Therefore, conversion efficiency at which excitation of cells in a myocardial outer layer is reflected in a waveform of an electrocardiogram is not necessarily the same in all the cells, and there is a possibility that there is a distribution in the conversion efficiency at which pulsation is reflected in the waveform of the electrocardiogram. Similarly, conversion efficiency at which excitation of cells in a myocardial inner layer is reflected in a waveform of an electrocardiogram is not necessarily the same in all the cells, and there is a possibility that there is a distribution in the conversion efficiency at which pulsation is reflected in the waveform of the electrocardiogram. However, such a possibility that there is a distribution in conversion efficiency at which pulsation of cells is reflected in a waveform of an electrocardiogram is not assumed in the electromotive force dipole model.

In the signal analysis device 1, in consideration of the possibility that there is a distribution in pulsation timing of cells and the possibility that there is a distribution in the conversion efficiency at which pulsation of cells is reflected in a waveform of an electrocardiogram, analysis is performed assuming that each of a distribution of timing at which the start of pulsation of each cell of a myocardial outer layer appears in a waveform of an electrocardiogram, a distribution of timing at which the start of pulsation of each cell of a myocardial inner layer appears in the waveform of the electrocardiogram, a distribution of timing at which the end of the pulsation of the each cell of the myocardial outer layer appears in the waveform of the electrocardiogram, and a distribution of timing at which the end of the pulsation of the each cell of the myocardial inner layer appears in the waveform of the electrocardiogram is a Gaussian distribution. That is, in the signal analysis device 1, analysis is performed assuming that the start of activity of a myocardial inner layer by all cells of the myocardial inner layer is included in a waveform of an electrocardiogram as a cumulative Gaussian distribution, the start of activity of a myocardial outer layer by all cells of the myocardial outer layer is included in the waveform of the electrocardiogram as a cumulative Gaussian distribution, the end of the activity of the myocardial inner layer by all the cells of the myocardial inner layer is included in the waveform of the electrocardiogram as a cumulative Gaussian distribution, and the end of the activity of the myocardial outer layer by all the cells of the myocardial outer layer is included in the waveform of the electrocardiogram as a cumulative Gaussian distribution.

Note that, although a cumulative Gaussian distribution function is preferably used in the signal analysis device 1, a sigmoid function, a Gompertz function, a logistic function, or the like may be used instead of the cumulative Gaussian distribution function. That is, the signal analysis device 1 may use, instead of the cumulative Gaussian distribution function, a cumulative distribution function of a unimodal distribution, that is, a cumulative distribution function corresponding to a distribution in which a value monotonically increases until time at which the value is the maximum value and monotonically decreases after the time at which the value is the maximum value. However, the cumulative distribution function used by the signal analysis device 1 needs to be a cumulative distribution function having a shape that can be identified by a parameter representing the shape of the cumulative distribution function or a parameter representing the shape of a unimodal distribution that is a cumulative source of the cumulative distribution function. Hereinafter, a parameter representing the shape of a cumulative distribution function (that is, parameter identifying a cumulative distribution function) is referred to as a shape parameter of a cumulative distribution function, and a parameter representing the shape of a unimodal distribution (that is, parameter identifying a unimodal distribution) is referred to as a shape parameter of a unimodal distribution. However, as a matter of course, the shape parameter of a cumulative distribution function and the shape parameter of a unimodal distribution are substantially the same. For example, in a case where a cumulative distribution function used by the signal analysis device 1 is a cumulative Gaussian distribution function, a standard deviation (or variance) and an average value of a Gaussian distribution that is a cumulative source of the cumulative Gaussian distribution function are the shape parameter of a unimodal distribution and also the shape parameter of a cumulative distribution function.

The signal analysis device 1 sets a waveform of a time section of one of an R wave and a T wave included in a waveform for one cycle of an acquired electrocardiogram of a target heart as a target time waveform and, upon approximating the target time waveform by a time waveform obtained by a difference or a weighted difference between a first cumulative distribution function that is a cumulative distribution function of a first unimodal distribution and a second cumulative distribution function that is a cumulative distribution function of a second unimodal distribution (hereinafter, the time waveform is referred to as an "approximate time waveform"), acquires a parameter identifying the first unimodal distribution or a parameter identifying the first cumulative distribution function and a parameter identifying the second unimodal distribution or a parameter identifying the second cumulative distribution function, as parameters representing characteristics of the target time waveform, that is, acquires them as myocardial activity parameters. Hereinafter, approximating a target time waveform by an approximate time waveform, that is, identifying the approximate time waveform is referred to as "fitting", and a first cumulative distribution function and a second cumulative distribution function included in the approximate time waveform are referred to as "fitting results". Note that, in a case where approximation is performed using a weighted difference, the signal analysis device 1 may also acquire weight given to a first cumulative distribution function and weight given to a second cumulative distribution function as parameters representing characteristics of a target time waveform (that is, myocardial activity parameters), or may also acquire a ratio between the weight given to the first cumulative distribution function and the weight given to the second cumulative distribution function as a parameter representing characteristics of the target time waveform (that is, myocardial activity parameter).

In a case where a target time waveform is approximated by an approximate time waveform obtained by a difference between a first cumulative distribution function and a second cumulative distribution function, for example, the signal analysis device 1 generates time waveforms each obtained by a difference between a first cumulative distribution function and a second cumulative distribution function (hereinafter, the time waveforms are referred to as "possible time waveforms") using respective combinations ($M \times N$) of parameters each identifying a cumulative distribution function for a plurality of possibilities (M) of a first cumulative distribution function and parameters each identifying a cumulative distribution function for a plurality of possibilities (N) of a second cumulative distribution function, identifies a possible time waveform closest to the target time waveform among the generated $M \times N$ possible time waveforms as an approximate time waveform, and acquires a parameter identifying a possibility of the first cumulative distribution function and a parameter identifying a possibility of the second cumulative distribution function used for generation of the identified approximate time waveform as parameters representing characteristics of the target time waveform. Processing of identifying a possible time waveform closest to a target time waveforms as an approximate time waveform may be performed by, for example, processing of identifying a possible time waveform having the smallest square error between the possible time waveform and the target time waveform.

Alternatively, for example, the signal analysis device 1, by repeating obtaining a possible time waveform that is a time waveform obtained by a difference between a possibility of a first cumulative distribution function and a possibility of a second cumulative distribution function that approximates a target time waveform, and updating at least one of parameters each identifying a cumulative distribution function in a direction in which the square error between the possible time waveform and the target time waveform decreases until the square error is equal to or less than a predetermined standard, or a predetermined number of times, identifies a finally obtained possible time waveform as an approximate time waveform, and acquires a parameter identifying a possibility of the first cumulative distribution function and a parameter identifying a possibility of the second cumulative distribution function used for generation of the identified approximate time waveform as parameters representing characteristics of the target time waveform.

In a case where a target time waveform is approximated by an approximate time waveform obtained by a weighted difference between a first cumulative distribution function and a second cumulative distribution function, for example, the signal analysis device 1 generates possible time waveforms that are time waveforms each obtained by a weighted difference between a first cumulative distribution function and a second cumulative distribution function using respective combinations (K × L × M × N) of parameters each identifying a cumulative distribution function for a plurality of possibilities (M) of a first cumulative distribution function, parameters each identifying a cumulative distribution function for a plurality of possibilities (N) of a second cumulative distribution function, a plurality of possibilities (K) of weight given to a first cumulative distribution function, and a plurality of possibilities (L) of weight given to a second cumulative distribution function, identifies a possible time waveform closest to the target time waveform among the generated K × L × M × N possible time waveforms as an approximate time waveform, and acquires a parameter identifying a possibility of the first cumulative distribution function, a parameter identifying a possibility of the second cumulative distribution function, weight given to the first cumulative distribution function, and weight given to the second cumulative distribution function used for generation of the identified approximate time waveform as parameters representing characteristics of the target time waveform.

Alternatively, for example, the signal analysis device 1, by repeating obtaining a possible time waveform that is a time waveform obtained by a weighted difference between a possibility of a first cumulative distribution function and a possibility of a second cumulative distribution function that approximates a target time waveform, and updating at least one of parameters each identifying a cumulative distribution function or weight each given to a cumulative distribution function in a direction in which the square error between the possible time waveform and the target time waveform decreases until the square error is equal to or less than a predetermined standard, or a predetermined number of times, identifies a finally obtained possible time waveform as an approximate time waveform, and acquires a parameter identifying a possibility of the first cumulative distribution function, a parameter identifying a possibility of the second cumulative distribution function, weight given to the first cumulative distribution function, and weight given to the second cumulative distribution function used for generation of the identified approximate time waveform as parameters representing characteristics of the target time waveform.

Hereinafter, processing of acquiring parameters representing characteristics of a target time waveform included in a waveform for one cycle of an acquired electrocardiogram of a target heart is referred to as myocardial activity information parameter acquisition processing.

Assuming that information indicating time is x, in a case where Gaussian distributions are used as a first unimodal distribution and a second unimodal distribution, the first unimodal distribution is represented by following Formula (1), a first cumulative distribution function $f_1(x)$ is represented by Formula (2), the second unimodal distribution is represented by Formula (3), and a second cumulative distribution function $f_2(x)$ is represented by Formula (4).

[Math. 1]

$$\frac{1}{\sqrt{2\pi\sigma_1{}^2}} exp\left(-\frac{(x-\mu_1)^2}{2\sigma_1{}^2}\right) \qquad \cdots (1)$$

[Math. 2]

$$f_1(x) = \frac{1}{2}\left(1 + erf\left(\frac{x-\mu_1}{\sqrt{2\sigma_1{}^2}}\right)\right) \qquad \cdots (2)$$

[Math. 3]

$$\frac{1}{\sqrt{2\pi\sigma_2{}^2}} exp\left(-\frac{(x-\mu_2)^2}{2\sigma_2{}^2}\right) \qquad \cdots (3)$$

[Math. 4]

$$f_2(x) = \frac{1}{2}\left(1 + erf\left(\frac{x-\mu_2}{\sqrt{2\sigma_2{}^2}}\right)\right) \qquad \cdots (4)$$

Formula (1) is a Gaussian distribution (normal distribution) having an average of $\mu_1$ and a standard deviation of $\sigma_1$ (variance of $\sigma_1{}^2$). Formula (3) is a Gaussian distribution (normal distribution) having an average of $\mu_2$ and a standard deviation of $\sigma_2$ (variance of $\sigma_2{}^2$). Formula (2) is a cumulative distribution function of Formula (1). Formula (4) is a cumulative distribution function of Formula (3). "erf" is a sigmoid function (error function). The unit of the information x indicating time is any unit, and for example, a sample number or relative time having a waveform of one cycle of an electrocardiogram as a starting end may be used as the information x indicating time.

The difference between a first cumulative distribution function and a second cumulative distribution function is expressed by, for example, following Formula (5). The function expressed by following Formula (5) is a function obtained by subtracting a second cumulative distribution function from a first cumulative distribution function.

[Math. 5]

$$f_1(x) - f_2(x)$$

$$= \frac{1}{2}\left(1 + erf\left(\frac{x-\mu_1}{\sqrt{2\sigma_1{}^2}}\right)\right) - \frac{1}{2}\left(1 + erf\left(\frac{x-\mu_2}{\sqrt{2\sigma_2{}^2}}\right)\right) \qquad \cdots (5)$$

That is, in a case where a target time waveform is approximated by an approximate time waveform that is a difference between a first cumulative distribution function and a second cumulative distribution function, the average $\mu_1$ and the standard deviation $\sigma_1$ that are parameters identifying a first unimodal distribution or parameters identifying the first cumulative distribution function, and the average $\mu_2$ and the standard deviation $\sigma_2$ that are parameters identifying a second unimodal distribution or the second cumulative distribution function are acquired as parameters representing characteristics of the target time waveform. Note that instead of acquiring a standard deviation as a parameter, a variance may be acquired as a parameter. The same applies to the following description of acquiring a standard deviation as a parameter.

The weighted difference between a first cumulative distribution function and a second cumulative distribution function is expressed by, for example, following Formula (6) assuming weight of a first cumulative distribution function as $k_1$ and weight of a second cumulative distribution function as $k_2$. The function expressed by following Formula (6) is a function obtained by subtracting a function obtained by multiplying a second cumulative distribution function by weight $k_2$ from a function obtained by multiplying a first cumulative distribution function by weight $k_1$.

[Math. 6]

$$k_1 f_1(x) - k_2 f_2(x)$$

$$= k_1 \frac{1}{2}\left(1 + erf\left(\frac{x-\mu_1}{\sqrt{2\sigma_1{}^2}}\right)\right) - k_2 \frac{1}{2}\left(1 + erf\left(\frac{x-\mu_2}{\sqrt{2\sigma_2{}^2}}\right)\right)$$

$$\cdots (6)$$

That is, in a case where a target time waveform is approximated by an approximate time waveform that is a weighted difference between a first cumulative distribution function and a second cumulative distribution function, the average $\mu_1$ and the standard deviation $\sigma_1$, that are parameters identifying a first unimodal distribution or parameters identifying the first cumulative distribution function, and the average $\mu_2$ and the standard deviation $\sigma_2$ that are parameters

identifying a second unimodal distribution or parameters identifying the second cumulative distribution function are at least acquired as parameters representing characteristics of the target time waveform. Note that the weight $k_1$ of the first cumulative distribution function and the weight $k_2$ of the second cumulative distribution function or a ratio between the weight $k_1$ of the first cumulative distribution function and the weight $k_2$ of the second cumulative distribution function ($k_1/k_2$ or $k_2/k_1$) may also be acquired as parameters representing the characteristics of the target time waveform.

In consideration that the function of Formula (6) is a function obtained by subtracting a function obtained by multiplying a second cumulative distribution function by weight $k_2$ from a function obtained by multiplying a first cumulative distribution function by weight $k_1$, both the weight $k_1$ and the weight $k_2$ are positive values. However, in a case where the target heart is in an abnormal state, a possibility that at least one of the weight $k_1$ or the weight $k_2$ obtained by fitting is not a positive value cannot be denied. Therefore, the signal analysis device 1 may perform fitting such that both weight $k_1$ and weight $k_2$ have positive values, but performing the fitting such that both weight $k_1$ and weight $k_2$ have positive values is not essential.

Note that, assuming an R wave as a first target time waveform and a T wave as a second target time waveform among the R wave and the T wave included in a waveform for one cycle of an acquired electrocardiogram of a target heart, the signal analysis device 1 preferably acquires parameters representing characteristics of a target time waveform described above for each of the first target time waveform and the second target time waveform.

For example, in a case where the first target time waveform (that is, R wave) is approximated by a difference between a first cumulative distribution function and a second cumulative distribution function, the first target time waveform is approximated by an approximate time waveform of Formula (9) that is a function obtained by subtracting a second cumulative distribution function $f_b(x)$ expressed by Formula (8) from a first cumulative distribution function $f_a(x)$ expressed by Formula (7), and an average $\mu_a$ and a standard deviation $\sigma_a$ that are parameters identifying the first cumulative distribution function, and an average $\mu_b$ and a standard deviation $\sigma_b$ that are parameters identifying the second cumulative distribution function are acquired as parameters representing the characteristics of the first target time waveform (that is, R wave).

[Math. 7]

$$f_a(x) = \frac{1}{2}\left(1 + erf\left(\frac{x - \mu_a}{\sqrt{2\sigma_a{}^2}}\right)\right) \quad \cdots (7)$$

[Math. 8]

$$f_b(x) = \frac{1}{2}\left(1 + erf\left(\frac{x - \mu_b}{\sqrt{2\sigma_b{}^2}}\right)\right) \quad \cdots (8)$$

[Math. 9]

$$f_a(x) - f_b(x) = \frac{1}{2}\left(1 + erf\left(\frac{x - \mu_a}{\sqrt{2\sigma_a{}^2}}\right)\right) - \frac{1}{2}\left(1 + erf\left(\frac{x - \mu_b}{\sqrt{2\sigma_b{}^2}}\right)\right)$$
$$\cdots (9)$$

For example, in a case where the first target time waveform (that is, R wave) is approximated by a weighted difference between a first cumulative distribution function and a second cumulative distribution function, the first target time waveform is approximated by an approximate time waveform of Formula (10) that is a function obtained by subtracting a function obtained by multiplying a second cumulative distribution function $f_b(x)$ expressed by Formula (8) by weight $k_b$ from a function obtained by multiplying a first cumulative distribution function $f_a(x)$ expressed by Formula (7) by weight $k_a$, and an average $\mu_a$ and a standard deviation $\sigma_a$ that are parameters identifying the first cumulative distribution function, and an average $\mu_b$ and a standard deviation $\sigma_b$ that are parameters identifying the second cumulative distribution function are at least acquired as parameters representing the characteristics of the first target time waveform (that is, R wave). Note that the weight $k_a$ of the first cumulative distribution function and the weight $k_b$ of the second cumulative distribution function or a ratio between the weight $k_a$ of the first cumulative distribution function and the weight $k_b$ of the second cumulative distribution function ($k_a/k_b$ or $k_b/k_a$) may also be acquired as parameters representing the characteristics of a parameter representing the characteristics of the first target time waveform (that is, R wave).

[Math. 10]

$$k_a f_a(x) - k_b f_b(x)$$

$$= k_a \frac{1}{2}\left(1 + erf\left(\frac{x - \mu_a}{\sqrt{2\sigma_a^2}}\right)\right) - k_b \frac{1}{2}\left(1 + erf\left(\frac{x - \mu_b}{\sqrt{2\sigma_b^2}}\right)\right)$$

$$\cdots (10)$$

In consideration that the function of Formula (10) is a function obtained by subtracting a function obtained by multiplying a second cumulative distribution function by weight $k_b$ from a function obtained by multiplying a first cumulative distribution function by weight $k_a$, both the weight $k_a$ and the weight $k_b$ are positive values. However, in a case where a target heart is in an abnormal state, a possibility that at least one of the weight $k_a$ or the weight $k_b$ obtained by fitting is not a positive value cannot be denied. Therefore, the signal analysis device 1 may perform fitting such that both weight $k_a$ and weight $k_b$ have positive values, but performing the fitting such that both weight $k_a$ and weight $k_b$ have positive values is not essential.

Since an R wave corresponds to sequential start of excitation of all cells of a myocardium according to Gaussian distributions, as for the R wave, a target time waveform in the forward direction of time needs to be approximated by an approximate time waveform obtained by a difference or a weighted difference between two cumulative Gaussian distributions as described above. On the other hand, since a T wave corresponds to sequential awakening of excitation of all cells of a myocardium according to the Gaussian distributions, the T wave can be interpreted as a phenomenon in the opposite direction of an R wave in the time axis. That is, as for the T wave, a waveform obtained by reversing the time axis of a target time waveform needs to be approximated by a difference or a weighted difference of cumulative Gaussian distributions. Hereinafter, this is referred to as a first method. Furthermore, since a T wave corresponds to awakening from the state in which all cells of a myocardium are excited according to Gaussian distributions, it can be said that a target time waveform in the forward direction of time needs to be approximated by a difference or a weighted difference between two functions (functions obtained by subtracting cumulative Gaussian distributions from 1) as for the T wave. Hereinafter, this is referred to as a second method. Specific examples of the first method and the second method will be described below, but in order to avoid confusion between cumulative distribution functions for an R wave described above and cumulative distribution functions for a T wave described below, as for the T wave, a first cumulative distribution function described above is referred to as a third cumulative distribution function, and a second cumulative distribution function described above is referred to as a fourth cumulative distribution function.

In a case where the second target time waveform (that is, T wave) is approximated by the difference between the third cumulative distribution function and the fourth cumulative distribution function using the first method, assuming that information indicating time in the opposite direction is x' and a waveform obtained by reversing the time axis of the second target time waveform is referred to as a second target inverse time waveform, the second target inverse time waveform is approximated by an approximate inverse time waveform of Formula (13) that is a function obtained by subtracting a fourth cumulative distribution function $f_g(x')$ expressed by Formula (12) from a third cumulative distribution function $f_e(x')$ expressed by Formula (11), and an average $\mu_e$ and a standard deviation $\sigma_e$ that are parameters identifying the third cumulative distribution function, and an average $\mu_g$ and a standard deviation $\sigma_g$ that are parameters identifying the fourth cumulative distribution function are acquired as parameters representing the characteristics of the second target time waveform (that is, T wave).

[Math. 11]

$$f_e(x') = \frac{1}{2}\left(1 + erf\left(\frac{x' - \mu_e}{\sqrt{2\sigma_e^2}}\right)\right) \qquad \cdots (11)$$

[Math. 12]

$$f_g(x') = \frac{1}{2}\left(1 + erf\left(\frac{x' - \mu_g}{\sqrt{2\sigma_g^2}}\right)\right) \qquad \cdots (12)$$

[Math. 13]

$$f_e(x') - f_g(x')$$

$$= \frac{1}{2}\left(1 + erf\left(\frac{x' - \mu_e}{\sqrt{2\sigma_e{}^2}}\right)\right) - \frac{1}{2}\left(1 + erf\left(\frac{x' - \mu_g}{\sqrt{2\sigma_g{}^2}}\right)\right)$$

$$\cdots (1\,3)$$

For example, in a case where a second target time waveform (that is, T wave) is approximated by a weighted difference between a third cumulative distribution function and a fourth cumulative distribution function using the first method, the second target inverse time waveform is approximated by an approximate inverse time waveform of Formula (14) that is a function obtained by subtracting a function obtained by multiplying a fourth cumulative distribution function $f_g(x')$ expressed by Formula (12) by weight $k_g$ from a function obtained by multiplying a third cumulative distribution function $f_e(x')$ expressed by Formula (11) by weight $k_e$, and an average $\mu_e$ and a standard deviation $\sigma_e$ that are parameters identifying the third cumulative distribution function, and an average $\mu_g$ and a standard deviation $\sigma_g$ that are parameters identifying the fourth cumulative distribution function are at least acquired as parameters representing characteristics of the second target time waveform (that is, T wave). Note that the weight $k_e$ of the third cumulative distribution function and the weight $k_g$ of the fourth cumulative distribution function or a ratio between the weight $k_e$ of the third cumulative distribution function and the weight $k_g$ of the fourth cumulative distribution function ($k_e/k_g$ or $k_g/k_e$) may also be acquired as parameters representing the characteristics of the second target time waveform (that is, T wave).
[Math. 14]

$$k_e f_e(x') - k_g f_g(x')$$

$$= k_e \frac{1}{2}\left(1 + erf\left(\frac{x' - \mu_e}{\sqrt{2\sigma_e{}^2}}\right)\right) - k_g \frac{1}{2}\left(1 + erf\left(\frac{x' - \mu_g}{\sqrt{2\sigma_g{}^2}}\right)\right)$$

$$\cdots (1\,4)$$

In consideration that the function of Formula (14) is a function obtained by subtracting a function obtained by multiplying a fourth cumulative distribution function by weight $k_g$ from a function obtained by multiplying a third cumulative distribution function by weight $k_e$, both the weight $k_e$ and the weight $k_g$ are positive values. However, in a case where a target heart is in an abnormal state, a possibility that at least one of the weight $k_e$ or the weight $k_g$ obtained by fitting is not a positive value cannot be denied. Therefore, the signal analysis device 1 may perform fitting such that both weight $k_e$ and weight $k_g$ have positive values, but performing the fitting such that both weight $k_e$ and weight $k_g$ have positive values is not essential.

For example, in a case where a second target time waveform (that is, T wave) is approximated by a difference between a function f'$_c$(x) obtained by subtracting a third cumulative distribution function f$_c$(x) expressed by Formula (15) from 1 (hereinafter, the function is referred to as a "third inverse cumulative distribution function") and a function f'$_d$(x) obtained by subtracting a fourth cumulative distribution function f$_d$(x) expressed by Formula (16) from 1 (hereinafter, the function is referred to as a "fourth inverse cumulative distribution function") using the second method, the second target time waveform is approximated by an approximate time waveform of Formula (17) that is a function obtained by subtracting the fourth cumulative distribution function f'$_d$(x) from the third inverse cumulative distribution function f'$_c$(x), and an average $\mu_c$ and a standard deviation $\sigma_c$ that are parameters identifying the third cumulative distribution function, and an average $\mu_d$ and a standard deviation $\sigma_d$ that are parameters identifying the fourth cumulative distribution function are acquired as parameters representing characteristics of the second target time waveform (that is, T wave).
[Math. 15]

$$f_c(x) = \frac{1}{2}\left(1 + erf\left(\frac{x - \mu_c}{\sqrt{2\sigma_c{}^2}}\right)\right) \qquad \cdots (1\,5)$$

[Math. 16]

$$f_d(x) = \frac{1}{2}\left(1 + erf\left(\frac{x - \mu_d}{\sqrt{2\sigma_d^2}}\right)\right) \qquad \cdots (16)$$

[Math. 17]

$$f'_c(x) - f'_d(x) = \left(1 - f_c(x)\right) - \left(1 - f_d(x)\right)$$

$$= f_d(x) - f_c(x) = \frac{1}{2}\left(1 + erf\left(\frac{x - \mu_d}{\sqrt{2\sigma_d^2}}\right)\right) - \frac{1}{2}\left(1 + erf\left(\frac{x - \mu_c}{\sqrt{2\sigma_c^2}}\right)\right)$$

$$\cdots (17)$$

For example, in a case where a second target time waveform (that is, T wave) is approximated by a weighted difference between a third inverse cumulative distribution function and a fourth inverse cumulative distribution function using the second method, the second target time waveform is approximated by an approximate time waveform of Formula (18) that is a function obtained by subtracting a function obtained by multiplying a fourth inverse cumulative distribution function $f'_d(x)$ by weight $k_d$ from a function obtained by multiplying a third inverse cumulative distribution function $f'_c(x)$ by weight $k_c$, and an average $\mu_c$ and a standard deviation $\sigma_c$ that are parameters identifying the third cumulative distribution function, and an average $\mu_d$ and a standard deviation $\sigma_d$ that are parameters identifying the fourth cumulative distribution function are acquired as parameters representing characteristics of the second target time waveform (that is, T wave). Note that the weight $k_c$ of the third inverse cumulative distribution function and the weight $k_d$ of the fourth inverse cumulative distribution function or a ratio between the weight $k_c$ of the third inverse cumulative distribution function and the weight $k_d$ of the fourth inverse cumulative distribution function ($k_c/k_d$ or $k_d/k_c$) may also be acquired as parameters representing the characteristics of the second target time waveform (that is, T wave).

[Math. 18]

$$k_c f'_c(x) - k_d f'_d(x)$$

$$= k_c\left(1 - f_c(x)\right) - k_d\left(1 - f_d(x)\right) = k_d f_d(x) - k_c f_c(x) + k_c - k_d$$

$$= k_d \frac{1}{2}\left(1 + erf\left(\frac{x - \mu_d}{\sqrt{2\sigma_d^2}}\right)\right) - k_c \frac{1}{2}\left(1 + erf\left(\frac{x - \mu_c}{\sqrt{2\sigma_c^2}}\right)\right) + k_c - k_d$$

$$\cdots (18)$$

In consideration that the function of Formula (18) is a function obtained by subtracting the function obtained by multiplying a fourth inverse cumulative distribution function by weight $k_d$ from a function obtained by multiplying a third inverse cumulative distribution function by weight $k_c$, both the weight $k_c$ and the weight $k_d$ are positive values. However, in a case where a target heart is in an abnormal state, a possibility that at least one of the weight $k_c$ or the weight $k_d$ obtained by fitting is not a positive value cannot be denied. Therefore, the signal analysis device 1 may perform fitting such that both weight $k_c$ and weight $k_d$ have positive values, but performing the fitting such that both weight $k_c$ and weight $k_d$ have positive values is not essential.

Note that an approximate time waveform of Formula (17) is a function obtained by subtracting a third cumulative distribution function $f_c(x)$ from a fourth cumulative distribution function $f_d(x)$, that is, a difference between the third cumulative distribution function $f_c(x)$ and the fourth cumulative distribution function $f_d(x)$. Furthermore, an approximate time waveform of Formula (18) is obtained by adding a constant term to a weighted difference between a third cumulative distribution function $f_c(x)$ and a fourth cumulative distribution function $f_d(x)$, and the shape of a curved portion is the same as a weighted difference between a third cumulative distribution function $f_c(x)$ and a fourth cumulative distribution function $f_d(x)$. Furthermore, as described above, a T wave can be interpreted as a phenomenon in the opposite direction of an R wave in the time axis, and as for the T wave, a waveform obtained by reversing the time axis of a target time waveform can be approximated by a difference or a weighted difference between a third cumulative distribution function $f_e(x)$ and a fourth cumulative distribution function $f_g(x)$. For these reasons, in the following description of a T wave, a cumulative distribution function and an inverse cumulative distribution function are not described together, and only a cumulative distribution function may be simply used.

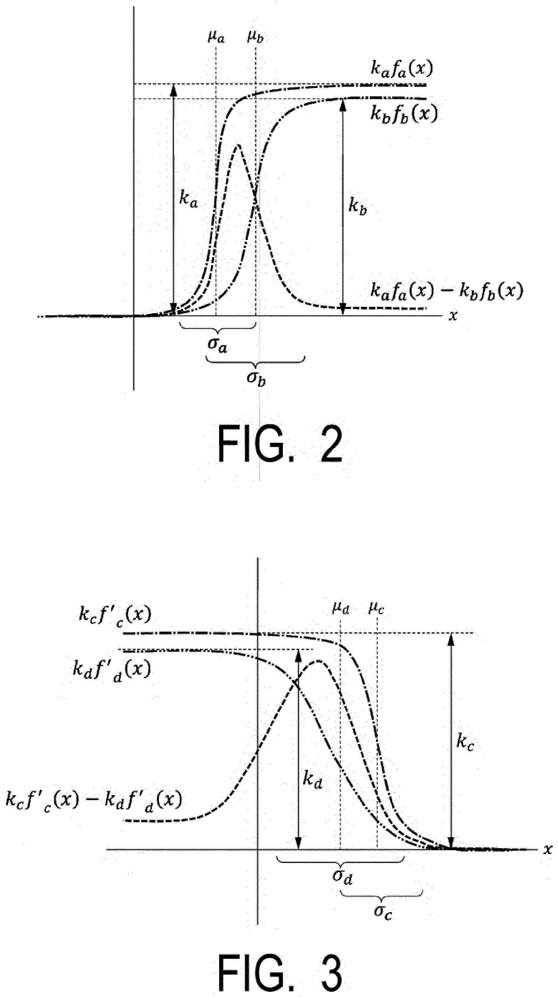

Fig. 2 is a diagram schematically illustrating a function $k_a f_a(x)$ obtained by multiplying a first cumulative distribution function $f_a(x)$ by weight $k_a$, a function $k_b f_b(x)$ obtained by multiplying a second cumulative distribution function $f_b(x)$ by weight $k_b$, and an approximate time waveform $k_a f_a(x) - k_b f_b(x)$ that is a weighted difference between the first cumulative distribution function and the second cumulative distribution function for a first target time waveform (that is, R wave). A one-dot chain line is the function $k_a f_a(x)$ obtained by multiplying the first cumulative distribution function $f_a(x)$ by the weight $k_a$, a two-dot chain line is the function $k_b f_b(x)$ obtained by multiplying the second cumulative distribution function $f_b(x)$ by the weight $k_b$, and a broken line is the approximate time waveform $k_a f_a(x) - k_b f_b(x)$. The approximate time waveform $k_a f_a(x) - k_b f_b(x)$ is a waveform obtained by approximating the first target time waveform (that is, R wave).

Fig. 3 is a diagram schematically illustrating a function $k_c f'_c(x)$ obtained by multiplying a third inverse cumulative distribution function $f'_c(x) = 1 - f_c(x)$ by weight $k_c$, a function $k_d f'_d(x)$ obtained by multiplying a fourth inverse cumulative distribution function $f'_d(x) = 1 - f_d(x)$ by weight $k_d$, and an approximate time waveform $k_c f'_c(x) - k_d f'_d(x)$ that is a weighted difference between the third inverse cumulative distribution function and the fourth inverse cumulative distribution function for a second target time waveform (that is, T wave). A one-dot chain line is the function $k_c f'_c(x)$ obtained by multiplying the third inverse cumulative distribution function $f'_c(x) = 1 - f_c(x)$ by the weight $k_c$, a two-dot chain line is the function $k_d f'_d(x)$ obtained by multiplying the fourth inverse cumulative distribution function $f'_d(x) = 1 - f_d(x)$ by the weight $k_d$, and a broken line is the approximate time waveform $k_c f'_c(x) - k_d f'_d(x)$. The approximate time waveform $k_c f'_c(x) - k_d f'_d(x)$ is a waveform obtained by approximating the second target time waveform (that is, T wave).

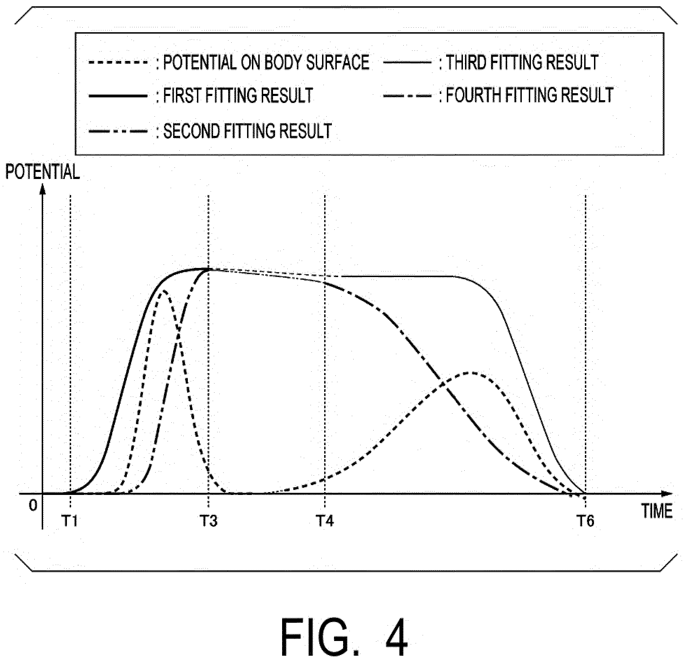

Fig. 4 is a diagram schematically illustrating results of fitting each of a first target time waveform and a second target time waveform by a difference between two cumulative distribution functions, assuming an R wave included in a waveform for one cycle of an electrocardiogram of a target heart according to the embodiment as the first target time waveform and a T wave as the second target time waveform. In Fig. 4, the horizontal axis represents time and the vertical axis represents a potential. Both the horizontal axis and the vertical axis are arbitrary units.

Specifically, Fig. 4 illustrates an example in which the first target time waveform (that is, R wave) is fitted by a difference between a first cumulative distribution function and a second cumulative distribution function, and the second target time waveform (that is, T wave) is fitted by a difference between a third cumulative distribution function and a fourth cumulative distribution function. The domain of the first cumulative distribution function and the domain of the second cumulative distribution function are the same, and are time T1 to time T3 that are time sections of the first target time waveform (that is, R wave). The domain of the third cumulative distribution function and the domain of the fourth cumulative distribution function are the same, and are from time T4 to time T6.

A "first fitting result" and a "second fitting result" in Fig. 4 are results of fitting for the R wave. A "third fitting result" and a "fourth fitting result" in Fig. 4 are results of fitting for the T wave.

In Fig. 4, the "first fitting result" indicates the first cumulative distribution function among results of fitting to the first target time waveform (that is, R wave) of the electrocardiogram. In Fig. 4, the "second fitting result" indicates the second cumulative distribution function among the results of fitting to the first target time waveform (that is, R wave) of the electrocardiogram. In Fig. 4, the "third fitting result" indicates the third cumulative distribution function among results of fitting to the second target time waveform (that is, T wave) of the electrocardiogram. In Fig. 4, the "fourth fitting result" indicates the fourth cumulative distribution function among the results of fitting to the second target time waveform (that is, T wave) of the electrocardiogram. In Fig. 4, a "potential on body surface" represents a waveform of the electrocardiogram to be fitted.

Note that the signal analysis device 1 does not perform fitting on a period from the time T3 to the time T4 that does not belong to a time section of the first target time waveform (that is, R wave) or a time section of the second target time waveform (that is, T wave). Note that the period on which fitting is not performed in the signal analysis device 1 is expressed by a line connecting the first fitting result at the time T3 and the third fitting result at the time T4 and a line connecting the second fitting result at the time T3 and the fourth fitting result at the time T4 in Fig. 4. That is, in a case where the signal analysis device 1 displays the fitting results, as illustrated in Fig. 4, the signal analysis device 1 needs to display lines obtained by connecting the first fitting result at the time T3 and the third fitting result at the time T4, and the second fitting result at the time T3 and the fourth fitting result at the time T4 by predetermined functions such as constant functions or linear functions.

Note that in a case where the signal analysis device 1 displays the fitting results, a weight value may be corrected so that the first fitting result at the time T3 and the third fitting result at the time T4 can be displayed using the same value. That is, although the actual first fitting result at the time T3 is $k_a f_a(T3)$ and the actual third fitting result at the time T4 is $k_c f'_c(T4)$, $\alpha_1$ that satisfies $k_a f_a(T3) = \alpha_1 k_c f'_c(T4)$ may be obtained and fitting results may be displayed using $\alpha_1 k_c$ instead of weight $k_c$, or the fitting results may be displayed using $k_a / \alpha_1$ instead of weight $k_a$. Similarly, in a case where the signal analysis device 1 displays the fitting results, a weight value may be corrected so that the second fitting result at the time T3 and the fourth fitting result at the time T4 can be displayed using the same value. That is, although the second fitting result at the time T3 is $k_b f_b(T3)$ and the fourth fitting result at the time T4 is $k_d f'_d(T4)$, $\alpha_2$ that satisfies $k_b f_b(T3) = \alpha_2 k_d f'_d(T4)$ is obtained and fitting results may be displayed using $\alpha_2 k_d$ instead of weight $k_d$, or the fitting results may be displayed using $k_b/\alpha_2$ instead of weight $k_b$.

Note that fitting for the first target time waveform and fitting for the second target time waveform may not be performed individually. That is, fitting for the first target time waveform and the second target time waveform may be collectively performed as fitting for both the first target time waveform and the second target time waveform. For example, in a case of collectively performing the fitting for the first target time waveform and the second target time waveform, the signal analysis device 1 preferably performs fitting in consideration of reducing a difference between the first fitting result at the time T3 and the third fitting result at the time T4 and reducing a difference between the second fitting result at the time T3 and the fourth fitting result at the time T4.

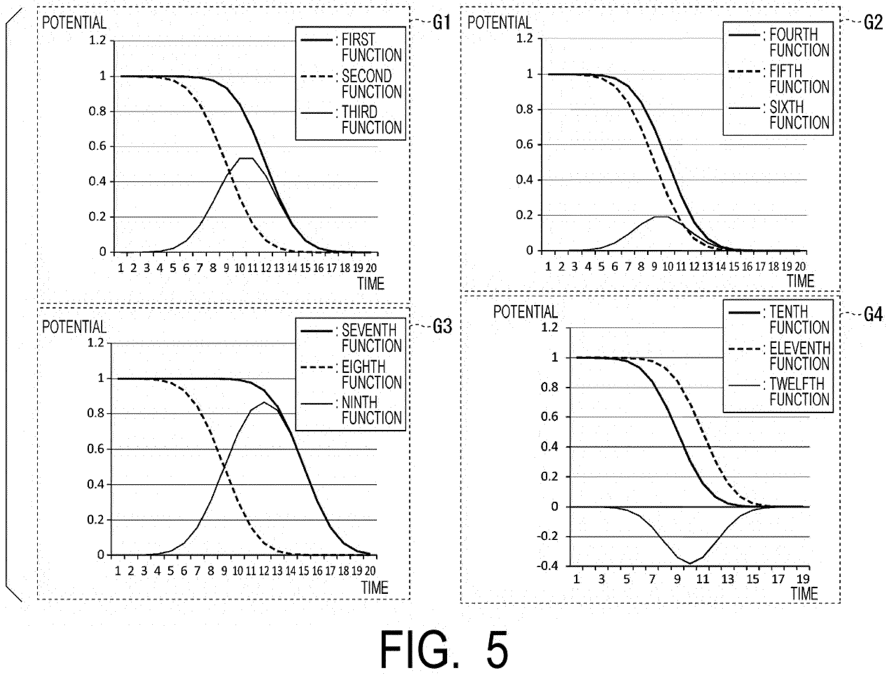

Fig. 5 is an explanatory diagram illustrating that typical characteristics of a T wave can be visualized by the T wave being approximated by a function obtained by subtracting a fourth inverse cumulative distribution function from a third inverse cumulative distribution function and the third inverse cumulative distribution function and the fourth inverse cumulative distribution function being displayed or parameters each identifying a cumulative distribution function being displayed. Fig. 5 illustrates four images of an image G1, an image G2, an image G3, and an image G4. Each of the images G1 to G4 illustrates a graph in which the horizontal axis represents time and the vertical axis represents a potential. The units of the horizontal axis and the vertical axis of each of the images G1 to G4 in Fig. 5 are arbitrary units.

A "first function" in Fig. 5 is an example of the third inverse cumulative distribution function. A "second function" in Fig. 5 is an example of the fourth inverse cumulative distribution function. A "third function" in Fig. 5 represents a function obtained by subtracting the "second function" from the "first function", that is, a function obtained by subtracting the fourth inverse cumulative distribution function from the third inverse cumulative distribution function. The "third function" in Fig. 5 has substantially the same shape as the shape of a T wave of a normal heart.

A "fourth function" in Fig. 5 is an example of the third inverse cumulative distribution function. A "fifth function" in Fig. 5 is an example of the fourth inverse cumulative distribution function. A "sixth function" in Fig. 5 represents a function obtained by subtracting the "fifth function" from the "fourth function", that is, a function obtained by subtracting the fourth inverse cumulative distribution function from the third inverse cumulative distribution function. The "sixth function" in Fig. 5 is substantially the same shape as the shape of decrease in height of a T wave of one of abnormal typical three patterns of a T wave. The interval between a falling portion of the third inverse cumulative distribution function and a falling portion of the fourth inverse cumulative distribution function in the image G2 of Fig. 5 is narrower than the interval between a falling portion of the third inverse cumulative distribution function and a falling portion of the fourth inverse cumulative distribution function in the image G1 of a normal heart, thereby visualizing that delay of activity of a myocardial outer layer from activity of a myocardial inner layer is small in decrease in height of a T wave.

A "seventh function" in Fig. 5 is an example of the third inverse cumulative distribution function. An "eighth function" in Fig. 5 is an example of the fourth inverse cumulative distribution function. A "ninth function" in Fig. 5 represents a function obtained by subtracting the "eighth function" from the "seventh function", that is, a function obtained by subtracting the fourth inverse cumulative distribution function from the third inverse cumulative distribution function. The "ninth function" in Fig. 5 is substantially the same shape as the shape of increase in height of a T wave of one of the abnormal typical three patterns of a T wave. The interval between a falling portion of the third inverse cumulative distribution function and a falling portion of the fourth inverse cumulative distribution function in the image G3 of Fig. 5 is wider than the interval between the falling portion of the third inverse cumulative distribution function and the falling portion of the fourth inverse cumulative distribution function in the image G1 of the normal heart, thereby visualizing that delay of activity of a myocardial outer layer from activity of a myocardial inner layer is large in increase in height of a T wave.

A "tenth function" in Fig. 5 is an example of the third inverse cumulative distribution function. An "eleventh function" in Fig. 5 is an example of the fourth inverse cumulative distribution function. A "twelfth function" in Fig. 5 represents a function obtained by subtracting the "eleventh function" from the "tenth function", that is, a function obtained by subtracting the fourth inverse cumulative distribution function from the third inverse cumulative distribution function. The "twelfth function" in Fig. 5 is substantially the same shape as the shape of a negative T wave of one of the abnormal typical three patterns of a T wave. The order between a falling portion of the third inverse cumulative distribution function and a falling portion of the fourth inverse cumulative distribution function in the image G4 of Fig. 5 is opposite to the order between the falling portion of the third inverse cumulative distribution function and the falling portion of the fourth inverse cumulative distribution function in the image G1 of the normal heart, thereby visualizing that a myocardial outer layer terminates activity earlier than a myocardial inner layer in a negative T wave.

The function obtained by subtracting the fourth inverse cumulative distribution function from the third inverse cumulative distribution function can express waves having different widths in the vertical axis direction and in the horizontal axis direction including the "third function", the "sixth function", and the "ninth function". Furthermore, the function obtained by subtracting the fourth inverse cumulative distribution function from the third inverse cumulative distribution function can express a negative wave including the "twelfth function". That is, by a T wave being approximated by the function obtained by subtracting the fourth inverse cumulative distribution function from the third inverse cumulative distribution function, or by a T wave being approximated by the function obtained by reversing the time axis and subtracting a fourth cumulative distribution function from a third cumulative distribution function, activity of each of a myocardial inner layer and a myocardial outer layer included in the T wave and a relationship between activity of the myocardial inner layer and the myocardial outer layer can be expressed. The same applies to a case where an R wave is approximated by a function obtained by subtracting a second cumulative distribution function from a first cumulative distribution function.

In this manner, the signal analysis device 1 performs fitting for a waveform of an R wave or a T wave of an electrocardiogram of a target heart by a difference or a weighted difference between two cumulative distribution functions. Then, the signal analysis device 1 acquires parameters identifying an approximate time waveform identified by fitting as parameters indicating activity of a myocardium.

[Approximation by Adding Value to Difference or Weighted Difference Between Two Cumulative Distribution Functions]

The signal analysis device 1 may set a waveform of a time section of an R wave or a T wave included in a waveform for one cycle of an acquired electrocardiogram of a target heart as a target time waveform, and set a time waveform obtained by adding a value to a difference or a weighted difference between a first cumulative distribution function that is a cumulative distribution function of a first unimodal distribution and a second cumulative distribution function that is a cumulative distribution function of a second unimodal distribution (hereinafter, the value is referred to as a "level value") as an approximate time waveform. In this case, upon approximating the target time waveform by the approximate time waveform, the level value is also acquired as a parameter representing characteristics of the target time waveform in addition to a parameter identifying the first unimodal distribution or a parameter identifying the first cumulative distribution function and a parameter identifying the second unimodal distribution or a parameter identifying the second cumulative distribution function. Of course, in a case where approximation is performed using a weighted difference, weight given to the first cumulative distribution function and weight given to the second cumulative distribution function may also be acquired as parameters representing the characteristics of the target time waveform, or a ratio between the weight given to the first cumulative distribution function and the weight given to the second cumulative distribution function may also be acquired as a parameter representing the characteristics of the target time waveform.

In a case where a weighted difference is used, for example, the signal analysis device 1 generates possible time waveforms that are time waveforms each obtained by adding a level value to a weighted difference between a first cumulative distribution function and a second cumulative distribution function using respective combinations ($J \times K \times L \times M \times N$) of parameters each identifying a cumulative distribution function for a plurality of possibilities (M) of a first cumulative distribution function, parameters each identifying a cumulative distribution function for a plurality of possibilities (N) of a second cumulative distribution function, a plurality of possibilities (K) of weight given to a first cumulative distribution function, a plurality of possibilities (L) of weight given to a second cumulative distribution function, and a plurality of possibilities (J) of a level value, identifies a possible time waveform closest to a target time waveform among the generated $J \times K \times L \times M \times N$ possible time waveforms as an approximate time waveform, and acquires a parameter identifying a possibility of the first cumulative distribution function, a parameter identifying a possibility of the second cumulative distribution function, weight given to the first cumulative distribution function, weight given to the second cumulative distribution function, and a level value used for generation of the identified approximate time waveform as parameters representing characteristics of the target time waveform.

Alternatively, for example, the signal analysis device 1 repeats obtaining a possible time waveform that is a time waveform obtained by adding a level value to a weighted difference between a possibility of a first cumulative distribution function and a possibility of a second cumulative distribution function that approximate the target time waveform, and updating at least one of parameters each identifying a cumulative distribution function, weight each given to a cumulative distribution function, and the level value in a direction in which the square error between the possible time waveform and the target time waveform decreases until the square error is equal to or less than a predetermined standard, or a predetermined number of times, identifies a finally obtained possible time waveform as an approximate time waveform, and acquires a parameter identifying a possibility of the first cumulative distribution function, a parameter identifying a possibility of the second cumulative distribution function, weight given to the first cumulative distribution function, weight given to the second cumulative distribution function, and a level value used for generation of the identified approximate time waveform as parameters representing characteristics of the target time waveform.

Note that a level value may be determined before fitting. In this case, the signal analysis device 1 first acquires a potential at the starting end of a target time waveform (corresponding to the time T1 in Fig. 4) as a level value in a case where a target time waveform is an R wave, and acquires a potential at the end of a target time waveform as a level value (corresponding to the time T6 in Fig. 4) in a case where the target time waveform is a T wave. Then, the signal analysis device 1 generates possible time waveforms that are time waveforms each obtained by adding the level value to a weighted difference between a first cumulative distribution function and a second cumulative distribution function using respective combinations ($K \times L \times M \times N$) of parameters each identifying a cumulative distribution function for a plurality of possibilities (M) of a first cumulative distribution function, parameters each identifying a cumulative distribution function for a plurality of possibilities (N) of a second cumulative distribution function, a plurality of possibilities (K) of weight given to a first cumulative distribution function, and a plurality of possibilities (L) of weight given to a second cumulative distribution function, and identifies a possible time waveform closest to a target time waveform among the generated $K \times L \times M \times N$ possible time waveforms as an approximate time waveform, and acquires a parameter identifying a possibility of the first cumulative distribution function, a parameter identifying a possibility of the second cumulative distribution function, weight given to the first cumulative distribution function, weight given to the second cumulative distribution function, and the level value determined in first processing used for generation of the identified approximate time waveform as parameters representing characteristics of the target time waveform.

Alternately, for example, the signal analysis device 1 first acquires a potential at the starting end of a target time waveform (corresponding to the time T1 in Fig. 4) as a level value in a case where the target time waveform is an R wave, and acquires a potential at the end of a target time waveform as a level value (corresponding to the time T6 in Fig. 4) in a case where the target time waveform is a T wave. Then, the signal analysis device 1 repeats obtaining a possible time waveform that is a time waveform obtained by adding the level value to a weighted difference between a possibility of a first cumulative distribution function and a possibility of a second cumulative distribution function that approximate a target time waveform, and updating at least one of parameters each identifying a cumulative distribution function or weight each given to a cumulative distribution function in a direction in which the square error between the possible time waveform and the target time waveform decreases until the square error is equal to or less than a predetermined standard, or a predetermined number of times, identifies a finally obtained possible time waveform as an approximate time waveform, and acquires a parameter identifying a possibility of the first cumulative distribution function, a parameter identifying a possibility of the second cumulative distribution function, weight given to the first cumulative distribution function, weight given to the second cumulative distribution function, and the level value determined in first processing used for generation of the identified approximate time waveform as parameters representing characteristics of the target time waveform.

Assuming a level value is $\beta$, a value obtained by adding the level value to a weighted difference between a first cumulative distribution function and a second cumulative distribution function is expressed by, for example, following Formula (19). The function expressed by following Formula (19) is a function obtained by subtracting a function obtained by multiplying a second cumulative distribution function by weight $k_2$ from a function obtained by multiplying a first cumulative distribution function by weight $k_1$ and adding the level value $\beta$.

[Math. 19]

$$k_1 f_1(x) - k_2 f_2(x) + \beta$$

$$= k_1 \frac{1}{2}\left(1 + erf\left(\frac{x - \mu_1}{\sqrt{2\sigma_1{}^2}}\right)\right) - k_2 \frac{1}{2}\left(1 + erf\left(\frac{x - \mu_2}{\sqrt{2\sigma_2{}^2}}\right)\right) + \beta$$

$$\cdots (19)$$

In a case where a target time waveform is approximated by an approximate time waveform of Formula (19) obtained by adding a level value to a weighted difference between a first cumulative distribution function and a second cumulative distribution function, an average $\mu_1$ and a standard deviation $\sigma_1$ that are parameters identifying a first unimodal distribution or parameters identifying the first cumulative distribution function, an average $\mu_2$ and a standard deviation $\sigma_2$ that are parameters identifying a second unimodal distribution or parameters identifying the second cumulative distribution function, and the level value $\beta$ are at least acquired as parameters representing characteristics of the target time waveform. Note that the weight $k_1$ of the first cumulative distribution function and the weight $k_2$ of the second cumulative distribution function or a ratio between the weight $k_1$ of the first cumulative distribution function and the weight $k_2$ of the second cumulative distribution function ($k_1/k_2$ or $k_2/k_1$) may also be acquired as parameters representing the characteristics of the target time waveform.

In consideration that the function of Formula (19) is a function obtained by subtracting a function obtained by multiplying a second cumulative distribution function by weight $k_2$ from a function obtained by multiplying a first cumulative distribution function by weight $k_1$ and adding the level value $\beta$, both the weight $k_1$ and the weight $k_2$ are positive values. However, in a case where the target heart is in an abnormal state, a possibility that at least one of the weight $k_1$ or the weight $k_2$ obtained by fitting is not a positive value cannot be denied. Therefore, the signal analysis device 1 may perform fitting such that both weight $k_1$ and weight $k_2$ have positive values, but performing the fitting such that both weight $k_1$ and weight $k_2$ have positive values is not essential.

Assuming an R wave as a first target time waveform and a T wave as a second target time waveform among the R wave and the T wave included in a waveform for one cycle of an acquired electrocardiogram of a target heart, the signal analysis device 1 may acquire above-described parameters representing characteristics of a target time waveform for each of the first target time waveform and the second target time waveform.

For example, in a case where a first target time waveform (that is, R wave) is approximated by a function obtained by adding a level value to a weighted difference between a first cumulative distribution function and a second cumulative distribution function, assuming a potential at the starting end of the first target time waveform as a level value $\beta_R$, the first target time waveform is approximated by an approximate time waveform of Formula (20) that is a function obtained by subtracting a function obtained by multiplying a second cumulative distribution function $f_b(x)$ expressed by Formula (8) by weight $k_b$ from a function obtained by multiplying a first cumulative distribution function $f_a(x)$ expressed by Formula (7) by weight $k_a$ and adding the level value $\beta_R$, and an average $\mu_a$ and a standard deviation $\sigma_a$ that are parameters identifying the first cumulative distribution function, an average $\mu_b$ and a standard deviation $\sigma_b$ that are parameters identifying the second cumulative distribution function, and the level value $\beta_R$ are at least acquired as parameters representing characteristics of the first target time waveform (that is, R wave). Note that the weight $k_a$ of the first cumulative distribution function and the weight $k_b$ of the second cumulative distribution function or a ratio between the weight $k_a$ of the first cumulative distribution function and the weight $k_b$ of the second cumulative distribution function ($k_a/k_b$ or $k_b/k_a$) may also be acquired as parameters representing the characteristics of a parameter representing the characteristics of the first target time waveform (that is, R wave).

[Math. 20]

$$k_a f_a(x) - k_b f_b(x) + \beta_R$$

$$= k_a \frac{1}{2} \left( 1 + erf \left( \frac{x - \mu_a}{\sqrt{2\sigma_a{}^2}} \right) \right) - k_b \frac{1}{2} \left( 1 + erf \left( \frac{x - \mu_b}{\sqrt{2\sigma_b{}^2}} \right) \right) + \beta_R$$

$$\cdots (20)$$

In consideration that the function of Formula (20) is a function obtained by subtracting a function obtained by multiplying a second cumulative distribution function by weight $k_b$ from a function obtained by multiplying a first cumulative distribution function by weight $k_a$ and adding the level value $\beta_R$, both the weight $k_a$ and the weight $k_b$ are positive values. However, in a case where a target heart is in an abnormal state, a possibility that at least one of the weight $k_a$ or the weight $k_b$ obtained by fitting is not a positive value cannot be denied. Therefore, the signal analysis device 1 may perform fitting such that both weight $k_a$ and weight $k_b$ have positive values, but performing the fitting such that both weight $k_a$ and weight $k_b$ have positive values is not essential.

For example, in a case where a second target inverse time waveform that is a waveform obtained by reversing the time axis of a second target time waveform (that is, T wave) is approximated by a function obtained by adding a level value to a weighted difference between a third cumulative distribution function and a fourth cumulative distribution function, assuming a potential at the end of the second target time waveform as a level value $\beta_T$, the second target inverse time waveform is approximated by an approximate inverse time waveform of Formula (21) that is a function obtained by subtracting a function obtained by multiplying a fourth inverse cumulative distribution function $f_g(x')$ expressed by Formula (12) by weight $k_g$ from a function obtained by multiplying a third cumulative distribution function $f_e(x')$ expressed by Formula (11) by weight $k_e$ and adding the level value $\beta_T$, and an average $\mu_e$ and a standard deviation $\sigma_e$ that are parameters identifying the third cumulative distribution function, an average $\mu_g$ and a standard deviation $\sigma_g$ that are parameters identifying the fourth cumulative distribution function, and the level value $\beta_T$ are at least acquired as parameters representing characteristics of the second target time waveform (that is, T wave). Note that the weight $k_e$ of the third cumulative distribution function and the weight $k_g$ of the fourth cumulative distribution function or a ratio between the weight $k_e$ of the third cumulative distribution function and the weight $k_g$ of the fourth cumulative distribution function ($k_e/k_g$ or $k_g/k_e$) may also be acquired as parameters representing characteristics of a parameter representing the characteristics of the second target time waveform (that is, T wave).

[Math. 21]

$$k_e f_e(x') - k_g f_g(x') + \beta_T$$

$$= k_e \frac{1}{2} \left( 1 + erf \left( \frac{x' - \mu_e}{\sqrt{2\sigma_e{}^2}} \right) \right) - k_g \frac{1}{2} \left( 1 + erf \left( \frac{x' - \mu_g}{\sqrt{2\sigma_g{}^2}} \right) \right) + \beta_T$$

$$\cdots (21)$$

In consideration that the function of Formula (21) is a function obtained by subtracting a function obtained by multiplying a fourth cumulative distribution function by weight $k_g$ from a function obtained by multiplying a third cumulative distribution function by weight $k_e$ and adding the level value $\beta_T$, both the weight $k_e$ and the weight $k_g$ are positive values. However, in a case where a target heart is in an abnormal state, a possibility that at least one of the weight $k_e$ or the weight $k_g$ obtained by fitting is not a positive value cannot be denied. Therefore, the signal analysis device 1 may perform fitting such that both weight $k_e$ and weight $k_g$ have positive values, but performing the fitting such that both weight $k_e$ and weight $k_g$ have positive values is not essential.

For example, in a case where a second target time waveform (that is, T wave) is approximated by a function obtained by adding a level value to a weighted difference between a function obtained by subtracting a third cumulative distribution function from 1 (that is, third inverse cumulative distribution function) and a function obtained by subtracting a fourth cumulative distribution function from 1 (that is, fourth inverse cumulative distribution function), assuming a potential at the end of the second target time waveform as the level value $\beta_T$, the second target time waveform is approximated by an approximate time waveform of Formula (22) that is a function obtained by subtracting a function obtained by multiplying a fourth inverse cumulative distribution function $f'_d(x)$ by weight $k_d$ from a function obtained by multiplying a third inverse cumulative distribution function $f'_c(x)$ by weight $k_c$ and adding the level value $\beta_T$, and an average $\mu_c$ and a standard deviation $\sigma_c$ that are parameters identifying the third cumulative distribution function, an average $\mu_d$ and a standard deviation $\sigma_d$ that are parameters identifying the fourth cumulative distribution function, and the level value $\beta_T$ are acquired as parameters representing characteristics of the second target time waveform (that is, T wave). Note that the weight $k_c$ of the third inverse cumulative distribution function and the weight $k_d$ of the fourth inverse cumulative distribution function or a ratio between the weight $k_c$ of the third inverse cumulative distribution function and the weight $k_d$ of the fourth inverse cumulative distribution function ($k_c/k_d$ or $k_d/k_c$) may also be acquired as parameters representing the characteristics of the second target time waveform (that is, T wave).

[Math. 22]

$$k_c f'_c(x) - k_d f'_d(x) + \beta_T = k_c\left(1 - f_c(x)\right) - k_d\left(1 - f_d(x)\right) + \beta_T$$
$$= k_d f_d(x) - k_c f_c(x) + k_c - k_d + \beta_T$$
$$= k_d \frac{1}{2}\left(1 + erf\left(\frac{x - \mu_d}{\sqrt{2\sigma_d{}^2}}\right)\right) - k_c \frac{1}{2}\left(1 + erf\left(\frac{x - \mu_c}{\sqrt{2\sigma_c{}^2}}\right)\right) + k_c - k_d + \beta_T$$
$$\cdots (2\,2)$$

In consideration that the function of Formula (22) is a function obtained by subtracting a function obtained by multiplying a fourth inverse cumulative distribution function by weight $k_d$ from a function obtained by multiplying a third inverse cumulative distribution function by weight $k_c$ and adding the level value $\beta_T$, both the weight $k_c$ and the weight $k_d$ are positive values. However, in a case where a target heart is in an abnormal state, a possibility that at least one of the weight $k_c$ or the weight $k_d$ obtained by fitting is not a positive value cannot be denied. Therefore, the signal analysis device 1 may perform fitting such that both weight $k_c$ and weight $k_d$ have positive values, but performing the fitting such that both weight $k_c$ and weight $k_d$ have positive values is not essential.

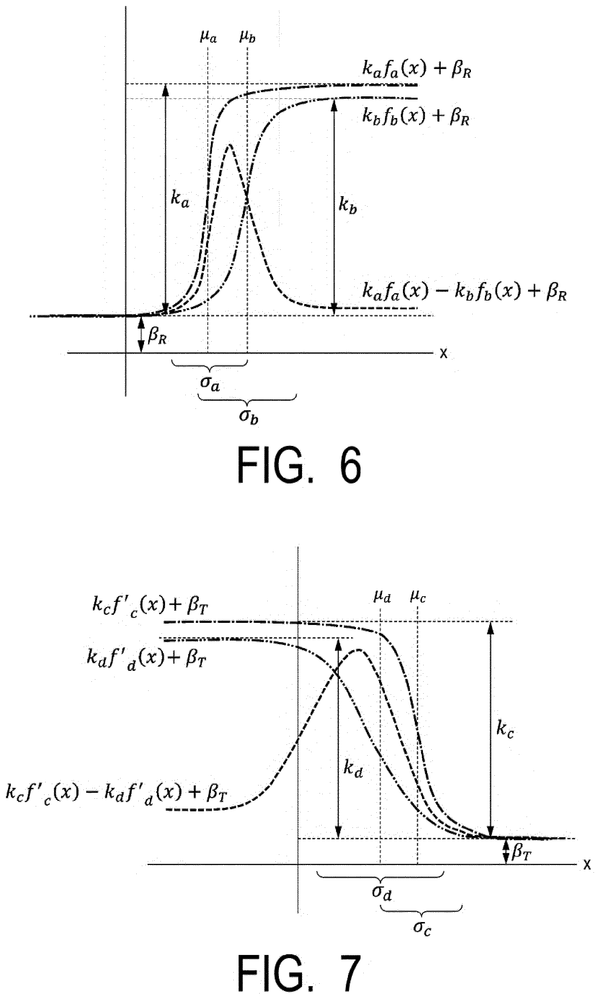

Fig. 6 is a diagram schematically illustrating a function $k_a f_a(x) + \beta_R$ obtained by multiplying a first cumulative distribution function $f_a(x)$ by weight $k_a$ and adding a level value $\beta_R$, a function $k_b f_b(x) + \beta_R$ obtained by multiplying a second cumulative distribution function $f_b(x)$ by weight $k_b$ and adding the level value $\beta_R$, and an approximate time waveform $k_a f_a(x) - k_b f_b(x) + \beta_R$ obtained by adding the level value $\beta_R$ to a weighted difference between the first cumulative distribution function and the second cumulative distribution function for a first target time waveform (that is, R wave). A one-dot chain line is the function $k_a f_a(x) + \beta_R$ obtained by multiplying the first cumulative distribution function $f_a(x)$ by the weight $k_a$ and adding the level value $\beta_R$, a two-dot chain line is the function $k_b f_b(x) + \beta_R$ obtained by multiplying the second cumulative distribution function $f_b(x)$ by the weight $k_b$ and adding the level value $\beta_R$, and a broken line is the approximate time waveform $k_a f_a(x) - k_b f_b(x) + \beta_R$. The approximate time waveform $k_a f_a(x) - k_b f_b(x) + \beta_R$ is a waveform obtained by approximating the first target time waveform (that is, R wave).

Fig. 7 is a diagram schematically illustrating a function $k_c f'_c(x) + \beta_T$ obtained by multiplying a third inverse cumulative distribution function $f'_c(x) - 1 - f_c(x)$ by weight $k_c$ and adding a level value $\beta_T$, a function $k_d f'_d(x) + \beta_T$ obtained by multiplying a fourth inverse cumulative distribution function $f'_d(x) = 1 - f_d(x)$ by weight $k_d$ and adding the level value $\beta_T$, and an approximate time waveform $k_c f'_c(x) - k_d f'_d(x) + \beta_T$ obtained by adding the level value $\beta_T$ to a weighted difference between the third inverse cumulative distribution function and the fourth inverse cumulative distribution function for a second target time waveform (that is, T wave). A one-dot chain line is the function $k_c f'_c(x) + \beta_T$ obtained by multiplying the third inverse cumulative distribution function $f'_c(x) = 1 - f_c(x)$ by the weight $k_c$ and adding the level value $\beta_T$, a two-dot chain line is the function $k_d f'_d(x) + \beta_T$ obtained by multiplying the fourth inverse cumulative distribution function $f'_d(x) = 1 - f_d(x)$ by the weight $k_d$ and adding the level value $\beta_T$, and a broken line is the approximate time waveform $k_c f'_c(x) - k_d f'_d(x) + \beta_T$. The approximate

time waveform $k_c f'_c(x) - k_d f'_d(x) + \beta_T$ is a waveform obtained by approximating the second target time waveform (that is, T wave).

Note that the level value $\beta_R$ that is a potential at the starting end of an R wave (more precisely, QRS wave) is a value representing the magnitude of a DC component at the starting end of the R wave, and in a case where there is an abnormality in the coronary artery, the level value $\beta_R$ may fall to the negative side. Furthermore, the level value $\beta_T$ that is a potential at the end of a T wave is a value representing the magnitude of a DC component at the end of the T wave, and in a case where there is an abnormality in repolarization of a myocardium, the level value $\beta_T$ may rise to the positive side.

[Approximation of Difference Between Target Time Waveform And Approximate Time Waveform]

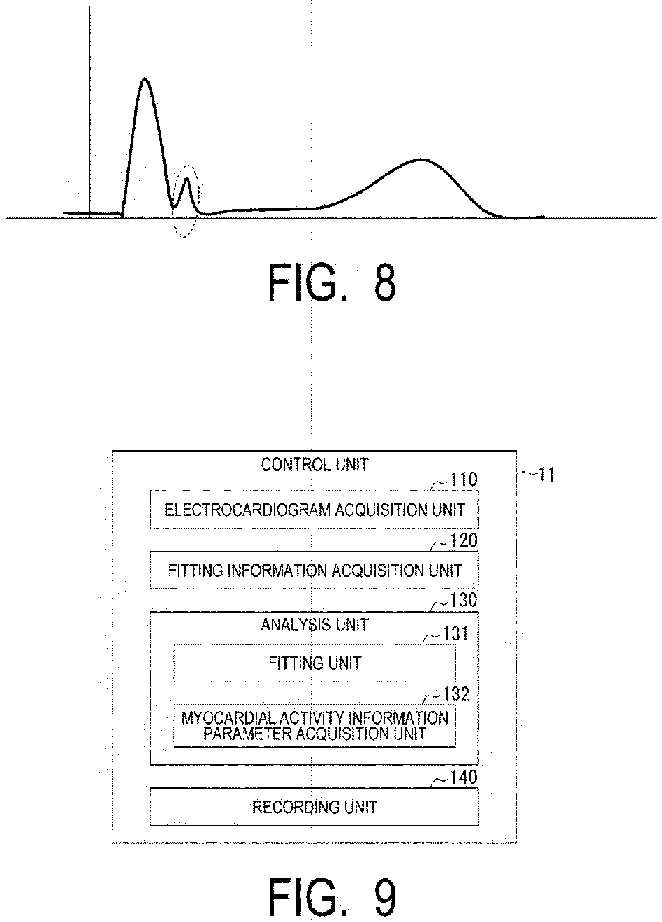

In a case where an R wave or a T wave in a particular state is used as a target time waveform, a portion in which the target time waveform cannot be approximated by an above-described approximate time waveform (hereinafter, the portion referred to as a "residual portion") may be left. For example, in a case where early repolarization or conduction disturbance (such as accessory pathway) occurs in a target heart, a $\Delta$ wave as indicated using a broken line in Fig. 8 may be included in the target time waveform (R wave). This $\Delta$ wave portion is left as a residual portion as the target time waveform cannot be approximated by the above-described approximate time waveform. Since the residual portion is also a time waveform caused by certain activity of the heart, the signal analysis device 1 may perform analysis assuming that the residual portion is a cumulative Gaussian distribution, a function obtained by multiplying a cumulative Gaussian distribution by weight, a difference between cumulative Gaussian distributions, or a weighted difference between cumulative Gaussian distributions.

That is, as for a residual time waveform that is a difference between a target time waveform and an approximate time waveform, upon approximating the residual time waveform by a cumulative distribution function (for convenience, referred to as a "fifth cumulative distribution function") of a certain unimodal distribution (for convenience, referred to as a "fifth unimodal distribution") or a function obtained by multiplying the fifth cumulative distribution function by weight, the signal analysis device 1 may acquire a parameter identifying the fifth unimodal distribution or a parameter identifying the fifth cumulative distribution function also as a parameter representing characteristics of the target time waveform.

Alternatively, as for a residual time waveform that is a difference between a target time waveform and an approximate time waveform, upon approximating the residual time waveform by a time waveform (hereinafter, referred to as an "approximate residual time waveform") obtained by a difference or a weighted difference between a cumulative distribution function (for convenience, referred to as a "fifth cumulative distribution function") of a certain unimodal distribution (for convenience, referred to as a "fifth unimodal distribution") and a cumulative distribution function (for convenience, referred to as a "sixth cumulative distribution function") of a unimodal distribution different from the fifth unimodal distribution (for convenience, referred to as a "sixth unimodal distribution"), the signal analysis device 1 may acquire a parameter identifying the fifth unimodal distribution or a parameter identifying the fifth cumulative distribution function, and a parameter identifying the sixth unimodal distribution or a parameter identifying the sixth cumulative distribution function also as parameters representing characteristics of the target time waveform.

More specifically, in a case where a residual time waveform is approximated by a fifth cumulative distribution function that is a cumulative distribution function of a fifth unimodal distribution expressed by Formula (23), the signal analysis device 1 approximates the residual time waveform by an approximate time waveform $f_5(x)$ of a fifth cumulative distribution function expressed by Formula (24) for the residual time waveform that is a difference between a target time waveform and an approximate time waveform, and acquires an average $\mu_5$ and a standard deviation $\sigma_5$ that are parameters identifying the fifth cumulative distribution function as parameters representing characteristics of the target time waveform in addition to above-described parameters representing the characteristics of the target time waveform.
[Math. 23]

$$\frac{1}{\sqrt{2\pi\sigma_5{}^2}} exp\left(-\frac{(x-\mu_5)^2}{2\sigma_5{}^2}\right) \quad \cdots (23)$$

[Math. 24]

$$f_5(x) = \frac{1}{2}\left(1 + erf\left(\frac{x-\mu_5}{\sqrt{2\sigma_5{}^2}}\right)\right) \quad \cdots (24)$$

In a case of approximating a residual time waveform by a function obtained by multiplying a fifth cumulative distribution function by weight, the signal analysis device 1 may approximate the residual time waveform by an approximate time waveform $k_5f_5(x)$ of a function obtained by multiplying a fifth cumulative distribution function $f_5(x)$ expressed by Formula (24) by weight $k_5$ for the residual time waveform that is a difference between a target time waveform and an approximate time waveform, and acquire an average $\mu_5$ and a standard deviation $\sigma_5$ that are parameters identifying the fifth cumulative distribution function as parameters representing characteristics of the target time waveform in addition to above-described parameters representing the characteristics of the target time waveform. The signal analysis device 1 may further acquire the weight $k_5$ as a parameter representing the characteristics of the target time waveform.

In a case where a residual time waveform is approximated by a difference between a fifth cumulative distribution function and a sixth cumulative distribution function that is a cumulative distribution function of a sixth unimodal distribution expressed by Formula (25), for example, the signal analysis device 1 approximates the residual time waveform by an approximate time waveform $f_5(x) - f_6(x)$ that is a waveform obtained by subtracting a sixth cumulative distribution function expressed by Formula (26) from a fifth cumulative distribution function expressed by Formula (24) for the residual time waveform that is a difference between a target time waveform and an approximate time waveform, and acquires an average $\mu_5$ and a standard deviation $\sigma_5$ that are parameters identifying the fifth cumulative distribution function and an average $\mu_6$ and a standard deviation $\sigma_6$ that are parameters identifying the sixth cumulative distribution function as parameters representing characteristics of the target time waveform in addition to above-described parameters representing the characteristics of the target time waveform.

[Math. 25]

$$\frac{1}{\sqrt{2\pi\sigma_6{}^2}} exp\left(-\frac{(x-\mu_6)^2}{2\sigma_6{}^2}\right) \qquad \cdots (2\,5)$$

[Math. 26]

$$f_6(x) = \frac{1}{2}\left(1 + erf\left(\frac{x-\mu_6}{\sqrt{2\sigma_6{}^2}}\right)\right) \qquad \cdots (2\,6)$$

In a case of approximating a residual time waveform by a weighted difference between a fifth cumulative distribution function and a sixth cumulative distribution function, the signal analysis device 1 approximates the residual time waveform by an approximate time waveform $k_5f_5(x) - k_6f_6(x)$ that is a waveform obtained by subtracting a function $k_6f_6(x)$ obtained by multiplying a sixth cumulative distribution function $f_6(x)$ by weight $k_6$ from a function $k_5f_5(x)$ obtained by multiplying a fifth cumulative distribution function $f_5(x)$ by weight $k_5$ for the residual time waveform that is a difference between a target time waveform and an approximate time waveform, and acquires an average $\mu_5$ and a standard deviation $\sigma_5$ that are parameters identifying the fifth cumulative distribution function and an average $\mu_6$ and a standard deviation $\sigma_6$ that are parameters identifying the sixth cumulative distribution function as parameters representing characteristics of the target time waveform in addition to above-described parameters representing the characteristics of the target time waveform. The signal analysis device 1 may further acquire the weight $k_5$ and the weight $k_6$ or a ratio of the weight $k_5$ and the weight $k_6$ ($k_5/k_6$ or $k_6/k_5$) as parameters representing the characteristics of the target time waveform.

In consideration that a residual time waveform is approximated by an approximate time waveform that is a waveform obtained by subtracting a function obtained by multiplying a sixth cumulative distribution function by weight $k_6$ from a function obtained by multiplying a fifth cumulative distribution function by weight $k_5$, both the weight $k_5$ and the weight $k_6$ are positive values. However, in a case where a target heart is in an abnormal state, a possibility that at least one of the weight $k_5$ or the weight $k_6$ obtained by fitting is not a positive value cannot be denied. Therefore, the signal analysis device 1 may perform fitting such that both weight $k_5$ and weight $k_6$ have positive values, but performing the fitting such that both weight $k_5$ and weight $k_6$ have positive values is not essential.

The description returns to Fig. 1. The signal analysis device 1 includes a control unit 11 including a processor 91 such as a central processing unit (CPU) and a memory 92 connected by a bus, and executes a program. The signal analysis device 1 functions as a device including the control unit 11, an input unit 12, a communication unit 13, a storage unit 14, and an output unit 15 by executing the program.

More specifically, the processor 91 reads the program stored in the storage unit 14, and stores the read program in the memory 92. The processor 91 executes the program stored in the memory 92, whereby the signal analysis device 1 functions as a device including the control unit 11, the input unit 12, the communication unit 13, the storage unit 14, and the output unit 15.

The control unit 11 controls operation of various functional units included in the signal analysis device 1. The control unit 11 performs, for example, myocardial activity information parameter acquisition processing. The control unit 11 controls operation of the output unit 15, for example, and causes the output unit 15 to output an acquisition result of the myocardial activity information parameter acquisition processing. The control unit 11 records, for example, various types of information generated by execution of the myocardial activity information parameter acquisition processing in the storage unit 14.

The input unit 12 includes an input device such as a mouse, a keyboard, and a touch panel. The input unit 12 may be configured as an interface that connects these input devices to the signal analysis device 1. The input unit 12 receives input of various types of information to the signal analysis device 1.

For example, information indicating the shape of a distribution represented by each cumulative distribution function (hereinafter, the information is referred to as "distribution shape designation information") is input to the input unit 12 for a plurality of possibilities of each cumulative distribution function used for fitting.

Note that the distribution shape designation information may be stored in the storage unit 14 in advance. In such a case, the distribution shape designation information stored in the storage unit 14 does not need to be input from the input unit 12. Hereinafter, the signal analysis device 1 will be described by taking, as an example, a case where the storage unit 14 has stored the distribution shape designation information in advance for simplicity of description.

The communication unit 13 includes a communication interface for connecting the signal analysis device 1 to an external device. The communication unit 13 communicates with the external device in a wired or wireless manner. The external device is, for example, a device of a transmission source of a waveform of an electrocardiogram of a target heart. The device of a transmission source of a waveform of an electrocardiogram of a target heart is, for example, an electrocardiogram measurement device. For example, in a case where the external device is the electrocardiogram measurement device, the communication unit 13 acquires a waveform of an electrocardiogram from the electrocardiogram measurement device by communication. Note that the waveform of the electrocardiogram may be input to the input unit 12.

The storage unit 14 is configured using a non-transitory computer-readable storage medium device such as a magnetic hard disk device or a semiconductor storage device. The storage unit 14 stores various types of information related to the signal analysis device 1. The storage unit 14 stores, for example, information input via the input unit 12 or the communication unit 13. The storage unit 14 stores, for example, an electrocardiogram input via the input unit 12 or the communication unit 13. The storage unit 14 stores, for example, various types of information generated by execution of the myocardial activity information parameter acquisition processing.

The output unit 15 outputs various types of information. The output unit 15 includes, for example, a display device such as a cathode ray tube (CRT) display, a liquid crystal display, or an organic electro-luminescence (EL) display. The output unit 15 may be configured as an interface that connects these display devices to the signal analysis device 1. The output unit 15 outputs, for example, information input to the input unit 12. The output unit 15 may display, for example, an electrocardiogram input to the input unit 12 or the communication unit 13. The output unit 15 may display, for example, an execution result of the myocardial activity information parameter acquisition processing.

Fig. 9 is a diagram illustrating an example of a functional configuration of the control unit 11 according to the embodiment. The control unit 11 includes an electrocardiogram acquisition unit 110, a fitting information acquisition unit 120, an analysis unit 130, and a recording unit 140.

The electrocardiogram acquisition unit 110 acquires a waveform for one cycle from an electrocardiogram waveform of a target heart input to the input unit 12 or the communication unit 13, and outputs the waveform to the analysis unit 130. The waveform of an electrocardiogram of a target heart is a waveform in which waveforms of a plurality of beats (a plurality of cycles) are arranged in time series. Even in a case where an abnormality occurs in the target heart, a waveform of all beats included in the waveform of the electrocardiogram is not a particular waveform, and only a waveform of any small number of beats included in the waveform of the electrocardiogram is often a characteristic waveform. In the myocardial activity information parameter acquisition processing, this characteristic waveform is preferably targeted. Therefore, the electrocardiogram acquisition unit 110 acquires a waveform for one cycle that is a characteristic waveform from a waveform of an electrocardiogram of a target heart. For example, the electrocardiogram acquisition unit 110 needs to acquire a waveform for one cycle that is a characteristic waveform from a waveform of an electrocardiogram of a target heart using a known technique for determining similarity and specificity. Furthermore, for example, the electrocardiogram acquisition unit 110 may cause the output unit 15 to display a waveform of an electrocardiogram, cause the input unit 12 to receive designation of a waveform for one cycle by a user such as a doctor, and acquire a waveform for one cycle corresponding to the designation received by the input unit 12 from the waveform of the electrocardiogram.

The electrocardiogram acquisition unit 110 outputs a waveform for one cycle as digital time-series data sampled at a predetermined sampling frequency. The predetermined sampling frequency is a sampling frequency of a signal used in processing in the fitting information acquisition unit 120, and is, for example, 250 Hz. In a case where the input waveform of an electrocardiogram is sampled at a predetermined sampling frequency, the electrocardiogram acquisition unit 110 needs to cut out digital time-series data of a waveform for one cycle from digital time-series data of the input waveform of the electrocardiogram and output the digital time-series data. In a case where the input waveform of the electrocardiogram is sampled at a sampling frequency different from the predetermined sampling frequency, the electrocardiogram acquisition unit 110 needs to cut out digital time-series data of a waveform for one cycle from digital time-series data of the input waveform of the electrocardiogram, convert the digital time-series data to the predetermined frequency, and then output the digital time-series data.

Furthermore, the electrocardiogram acquisition unit 110 identifies a time section of an R wave and a time section of a T wave included in the waveform of one cycle of the electrocardiogram, acquires information identifying the time section of the R wave and information identifying the time section of the T wave, and outputs the information to the analysis unit 130. For example, the electrocardiogram acquisition unit 110 needs to identify the starting end of the R wave, the end of the R wave, the starting end of the T wave, and the end of the T wave by a known technique, and acquire sample numbers corresponding to the identified starting end of the R wave, end of the R wave, starting end of the T wave, and end of the T wave, relative time from the starting ends of waveforms, and the like as information identifying the time section of the R wave and information identifying the time section of the T wave. Note that an R wave in the present specification accurately indicates a QRS wave. Although it is a fact that there are various interpretations as to which point of a waveform the starting end of an R wave (that is, starting end of a QRS wave) is, the electrocardiogram acquisition unit 110 needs to assume a point identified by any known technique as the starting end of the R wave.

Note that, in a waveform of an electrocardiogram of a target heart, a characteristic waveform that changes as time passes may appear. Therefore, the electrocardiogram acquisition unit 110 may acquire waveforms for a plurality of cycles from a waveform of an electrocardiogram of a target heart as a target waveform on which the myocardial activity information parameter acquisition processing is performed. That is, the electrocardiogram acquisition unit 110 may acquire a predetermined longterm time-series waveform (trend graph) from a waveform of an electrocardiogram of a target heart, and output, for waveforms of respective cycles included in the acquired waveform, waveforms, information identifying time sections of R waves included in the waveforms, and information identifying time sections of T waves included in the waveforms to the analysis unit 130.

The fitting information acquisition unit 120 acquires distribution shape designation information. In a case where distribution shape designation information is stored in the storage unit 14, the fitting information acquisition unit 120 reads the distribution shape designation information from the storage unit 14.

The analysis unit 130 includes a fitting unit 131 and a myocardial activity information parameter acquisition unit 132.

Using possibilities of a cumulative distribution function indicated by distribution shape designation information, the fitting unit 131 performs fitting on a target time waveform that is a waveform in a time section of at least one of an R wave or a T wave included in a waveform for one cycle of an electrocardiogram acquired by the electrocardiogram acquisition unit 110.

The myocardial activity information parameter acquisition unit 132 acquires parameters representing characteristics of a target time waveform on the basis of fitting results by the fitting unit 131. For example, upon approximating a target time waveform by an approximate time waveform that is a time waveform obtained by a difference or a weighted difference between a first cumulative distribution function that is a cumulative distribution function of a first unimodal distribution and a second cumulative distribution function that is a cumulative distribution function of a second unimodal distribution, the myocardial activity information parameter acquisition unit 132 acquires a parameter identifying the first unimodal distribution or a parameter identifying the first cumulative distribution function and a parameter identifying the second unimodal distribution or a parameter identifying the second cumulative distribution function, as parameters representing characteristics of the target time waveform. The parameters representing characteristics of a target time waveform acquired by the myocardial activity information parameter acquisition unit 132 are examples of myocardial activity parameters.

As described above, the analysis unit 130 acquires myocardial activity parameters on the basis of a target time waveform that is a waveform in a time section of at least one of an R wave or a T wave included in a waveform for one cycle of an electrocardiogram of a target heart and distribution possibility information.

The storage unit 14 records various types of information generated by processing executed by the control unit 11 in the storage unit 14.

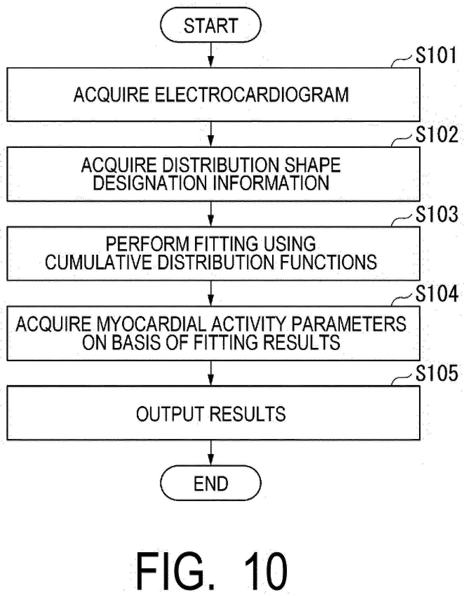

Fig. 10 is a flowchart illustrating an example of a flow of processing executed by the signal analysis device 1 according to the embodiment. The electrocardiogram acquisition unit 110 acquires a waveform for one cycle of an electrocardiogram of a target heart, information identifying a time section of an R wave, and information identifying a time section of a T wave via the input unit 12 or the communication unit 13 (step S101). Next, the fitting information acquisition unit 120 acquires distribution shape designation information (step S102). Next, using possibilities of a cumulative distribution function indicated by the distribution shape designation information, the fitting unit 131 performs fitting on a target time waveform that is a waveform in a time section of at least one of the R wave or the T wave included in the waveform for one cycle of the electrocardiogram acquired in step S101 (step S103). Next, the myocardial activity information parameter acquisition unit 132 acquires myocardial activity parameters on the basis of fitting results (step S104). The acquired myocardial activity parameters are output to the output unit 15 (step S105).

In step S105, a graph of fitting results for each target time waveform may be displayed. Furthermore, processing of step S102 only needs to be executed before processing of step S103 is executed, and may be executed before execution of step S101. Processing in steps S103 and S104 is an example of processing executed by the analysis unit 130.

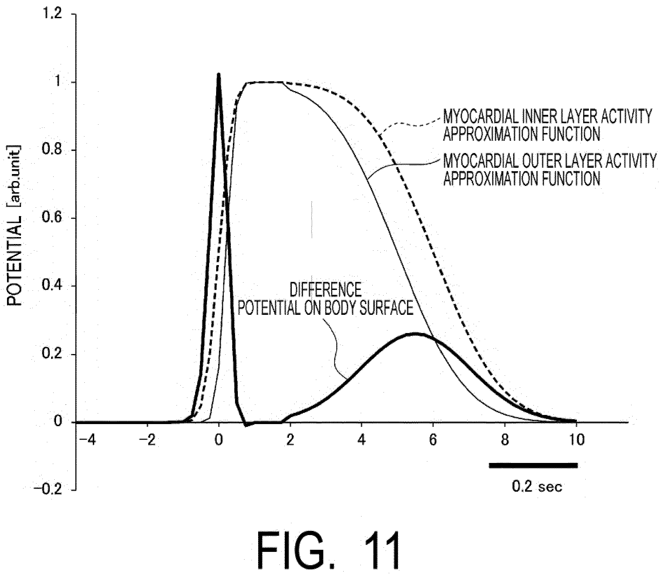

Fig. 11 is a first diagram illustrating an example of analysis results of the signal analysis device 1 according to the embodiment. More specifically, Fig. 11 is an example of analysis results by the signal analysis device 1 for a waveform of an electrocardiogram of a target heart in which the motion is normal. In Fig. 11, the horizontal axis represents time and the vertical axis represents a potential. The unit of the vertical axis is an arbitrary unit.

Fig. 11 illustrates an example in which a result of performing a first fitting and a second fitting on an R wave of depolarization of the electrocardiogram of the target heart in which the motion is normal and a result of performing a fourth fitting and a third fitting on a T wave in a repolarization phase of the electrocardiogram of the target heart in which the motion is normal are displayed using $k_a/\alpha_1$ instead of weight $k_a$ so that the first fitting result at time T3 and the third fitting result at time T4 are the same value, and using $k_b/\alpha_2$ instead of weight $k_b$ so that the second fitting result at the time T3 and the fourth fitting result at the time T4 are the same value. Hereinafter, a result obtained by connecting the first fitting result and the third fitting result having changed weight via a straight line is referred to as a myocardial inner layer activity approximation function, and a result obtained by connecting the second fitting result and the fourth fitting result having changed weight via a straight line is referred to as a myocardial outer layer activity approximation function.

Fig. 11 corresponds to a fact that, in depolarization in a normal heart, activity of a myocardial inner layer (that is, activity of ion channels) starts earlier and proceeds rapidly than that of a myocardial outer layer, activity of the myocardial outer layer starts slightly later than timing at which the activity of the ion channels of the myocardial inner layer starts, and a difference between the timing at which the activity of the ion channels of the myocardial inner layer starts and timing at which the activity of the myocardial outer layer starts is a positive and sharp R wave. That is, an average value and a standard deviation of a partial myocardial inner layer activity approximation function of the first fitting result in the myocardial inner layer activity approximation function and an average value and a standard deviation of a portion of the second fitting result in the myocardial outer layer activity approximation function are parameters representing timing and progress of the activity of the ion channels in depolarization of the normal heart.

Furthermore, Fig. 11 corresponds to a fact that, in a repolarization phase of the normal heart, deactivation of ion channels in the myocardial outer layer starts earlier than in the myocardial inner layer, deactivation of the myocardial inner layer starts later than deactivation of the myocardial outer layer, both deactivation of the myocardial outer layer and deactivation of the myocardial inner layer proceed slowly, and a difference between deactivation of the myocardial inner layer and deactivation of the myocardial outer layer is a positive and gentle T wave. That is, an average value and a standard deviation of a portion of the fourth fitting result in the myocardial outer layer activity approximation function and an average value and a standard deviation of a portion of the third fitting result in the myocardial inner layer activity approximation function are parameters representing timing and progress of deactivation of activity of ion channels in the repolarization phase of the normal heart. As described above, the myocardial inner layer activity approximation function and the myocardial outer layer activity approximation function obtained by the first to fourth fitting correspond to timing and progress of collective activation of ion channels of depolarization and collective deactivation of ion channels in a repolarization phase, and average values and standard deviations of respective fitting results of each function are parameters representing activity of the myocardium.

The shapes of the myocardial inner layer activity approximation function and the myocardial outer layer activity approximation function in Fig. 11 substantially correspond to measurement results of electromotive force of the myocardium obtained by directly measuring by inserting a catheter electrode into the myocardium of the target heart in which the motion is normal. This indicates that, according to the signal analysis device 1, information indicating the activity of the myocardium can be acquired only from the electrocardiogram without a catheter electrode being inserted.

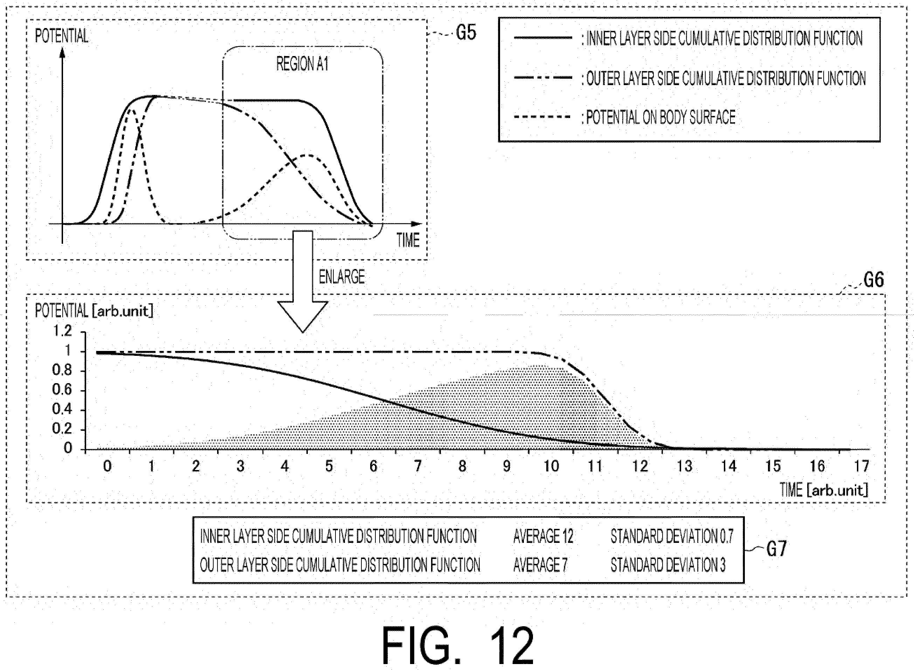

Fig. 12 is a second diagram illustrating an example of analysis results of the signal analysis device 1 according to the embodiment. More specifically, Fig. 12 is an example of analysis results by the signal analysis device 1 for a waveform of an electrocardiogram of a target heart in which the motion is normal.

Fig. 12 illustrates three results of a graph G5, a graph G6, and a result G7. In Fig. 12, an "inner layer side cumulative distribution function" represents a fitting result of a function representing a collective channel activity timing distribution of ion channels existing in a myocardial inner layer. In Fig. 12, an "outer layer side cumulative distribution function" represents a fitting result of a function representing a collective channel activity timing distribution of ion channels existing in a myocardial outer layer. In Fig. 12, a "potential on body surface" is a function representing a temporal change in potential of the body surface and is the waveform of the electrocardiogram. In Fig. 12, the horizontal axis represents time and the vertical axis represents a potential. Both the horizontal axis and the vertical axis are arbitrary units. Note that the length of time represented by an interval of one scale on each horizontal axis in Figs. 12 to 14 is the same. Furthermore, in each vertical axis in Figs. 12 to 14, 1 represents the maximum value of a cumulative Gaussian distribution.

The graph G5 represents an entire waveform of the electrocardiogram generated in one-time pulsation. The graph G6 illustrates an enlarged view of a T wave region that is a part of the graph G5. The T wave region is a region indicated as a region A1 in Fig. 12. The result G7 indicates statistics of two Gaussian distributions of a Gaussian distribution that is a cumulative source of the inner layer side cumulative distribution function and a Gaussian distribution that is a cumulative source of the outer layer side cumulative distribution function. Values of the result G7 represent the statistics of the two Gaussian distributions. Specifically, the statistics of the two Gaussian distributions are average values and standard deviations of Gaussian distributions that are cumulative sources of the inner layer side cumulative distribution function and the outer layer side cumulative distribution function.

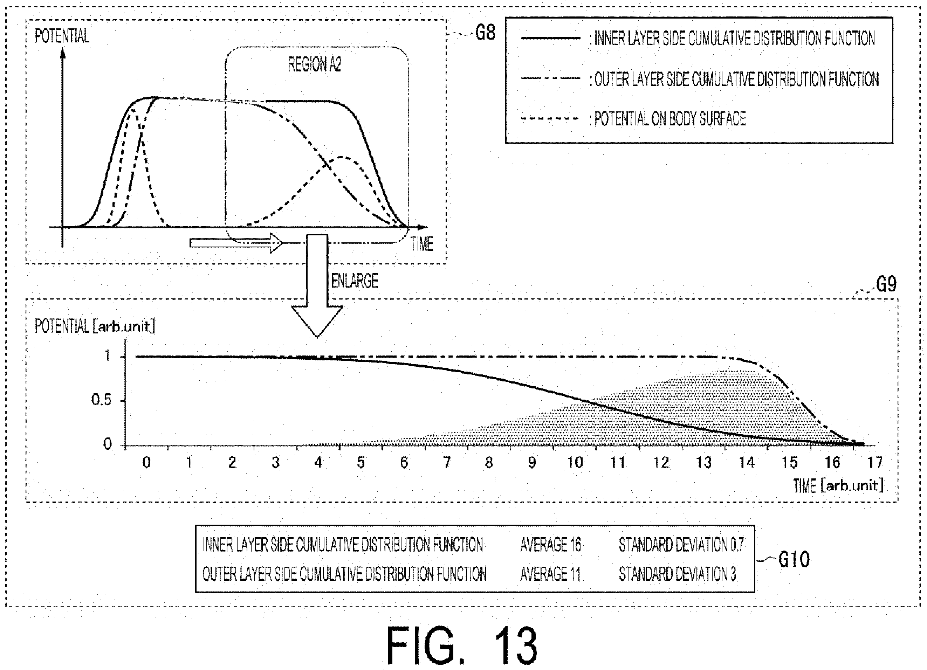

Fig. 13 is a third diagram illustrating an example of analysis results of the signal analysis device 1 according to the embodiment. More specifically, Fig. 13 is an example of analysis results by the signal analysis device 1 for a waveform of an electrocardiogram of a target heart in which the motion is long T type 3.

Fig. 13 illustrates three results of a graph G8, a graph G9, and a result G10. In Fig. 13, an "inner layer side cumulative distribution function" represents a fitting result of a function representing a collective channel activity timing distribution of channels existing in a myocardial inner layer. In Fig. 13, an "outer layer side cumulative distribution function" represents a fitting result of a function representing a collective channel activity timing distribution of channels existing in a myocardial outer layer. In Fig. 13, a "potential on body surface" is a function representing a temporal change in potential of the body surface and is the waveform of the electrocardiogram. In Fig. 13, the horizontal axis represents time and the vertical axis represents a potential. Both the horizontal axis and the vertical axis are arbitrary units.

The graph G8 represents an entire waveform of the electrocardiogram generated in one-time pulsation. The graph G9 illustrates an enlarged view of a T wave region that is a part of the graph G8. The T wave region is a region indicated as a region A2 in Fig. 13. The result G10 indicates statistics of two Gaussian distributions of a Gaussian distribution that is a cumulative source of the inner layer side cumulative distribution function and a Gaussian distribution that is a cumulative source of the outer layer side cumulative distribution function. Values of the result G10 represent the statistics of the two Gaussian distributions, that is, average values and standard deviations of Gaussian distributions that are cumulative sources of the inner layer side cumulative distribution function and the outer layer side cumulative distribution function.

The shapes of the inner layer side cumulative distribution function and the outer layer side cumulative distribution function in Fig. 13 substantially correspond to a result obtained by directly measuring a change in electromotive force generated by pulsation of the myocardial outer layer of the target heart in which the motion is long T type 3 by inserting a catheter electrode. This indicates that, according to the signal analysis device 1, information indicating the activity of the myocardium can be acquired only from the electrocardiogram without a catheter electrode being inserted.

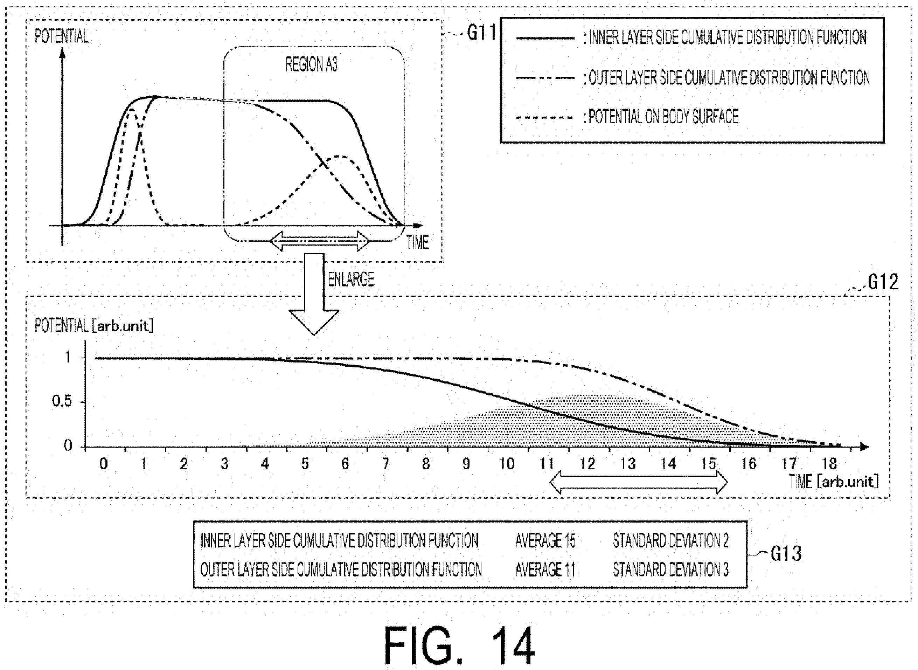

Fig. 14 is a fourth diagram illustrating an example of analysis results of the signal analysis device 1 according to the embodiment. More specifically, Fig. 14 is an example of analysis results by the signal analysis device 1 for a waveform of an electrocardiogram of a target heart in which the motion is long QT type 1.

Fig. 14 illustrates three results of a graph G11, a graph G12, and a result G13. In Fig. 14, an "inner layer side cumulative distribution function" represents a fitting result of a function representing a collective channel activity timing distribution of channels existing in a myocardial inner layer. In Fig. 14, an "outer layer side cumulative distribution function" represents a fitting result of a function representing a collective channel activity timing distribution of channels existing in a myocardial outer layer. In Fig. 14, a "potential on body surface" is a function representing a temporal change in potential of the body surface and is the waveform of the electrocardiogram. In Fig. 14, the horizontal axis represents time and the vertical axis represents a potential. Both the horizontal axis and the vertical axis are arbitrary units.

The graph G11 represents an entire waveform of the electrocardiogram generated in one-time pulsation. The graph G12 illustrates an enlarged view of a T wave region that is a part of the graph G11. The T wave region is a region indicated as a region A3 in Fig. 13. The result G13 indicates statistics of two Gaussian distributions of a Gaussian distribution that is a cumulative source of the inner layer side cumulative distribution function and a Gaussian distribution that is a cumulative source of the outer layer side cumulative distribution function. Values of the result G13 represent the statistics of the two Gaussian distributions. Specifically, the statistics of the two Gaussian distributions are average values and standard deviations of Gaussian distributions that are cumulative sources of the inner layer side cumulative distribution function and the outer layer side cumulative distribution function.

The shapes of the inner layer side cumulative distribution function and the outer layer side cumulative distribution function in Fig. 14 substantially correspond to a result obtained by directly measuring a change in electromotive force generated by pulsation of the myocardial outer layer of the target heart in which the motion is long QT type 3 by inserting a catheter electrode. This indicates that, according to the signal analysis device 1, information indicating the activity of the myocardium can be acquired only from the electrocardiogram without a catheter electrode being inserted.

Note that Fig. 14 is also an example of an estimation result of channel current characteristics related to sudden death by the signal analysis device 1.

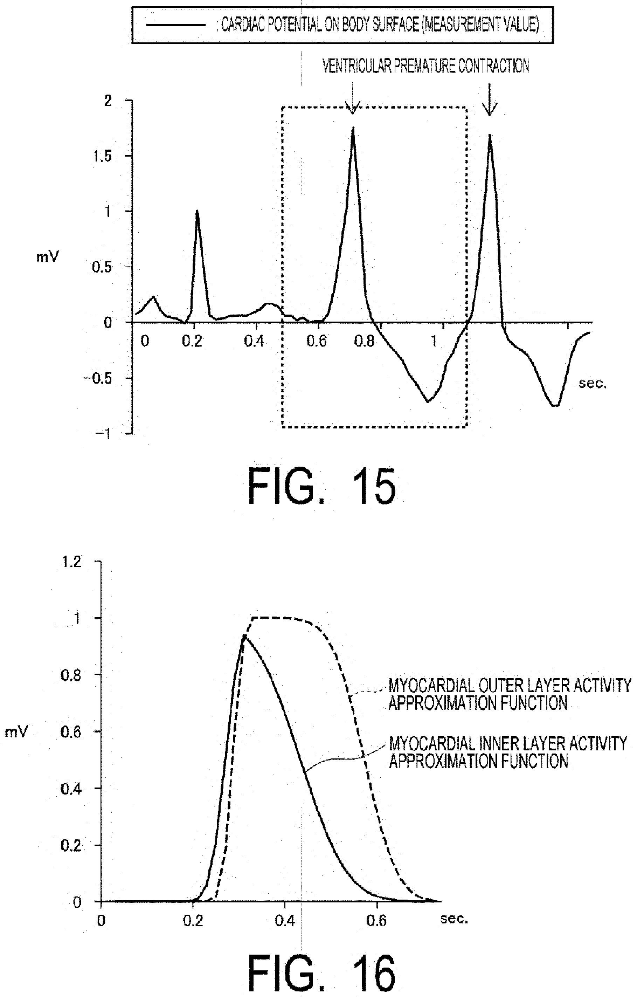

With reference to Figs. 15 to 17, it will be described that information indicating activity of a myocardium can be acquired only from an electrocardiogram by the signal analysis device 1 also for an electrocardiogram of a ventricular premature contraction. In Figs. 15 to 17, the horizontal axis represents time (second) and the vertical axis represents a potential (mV).

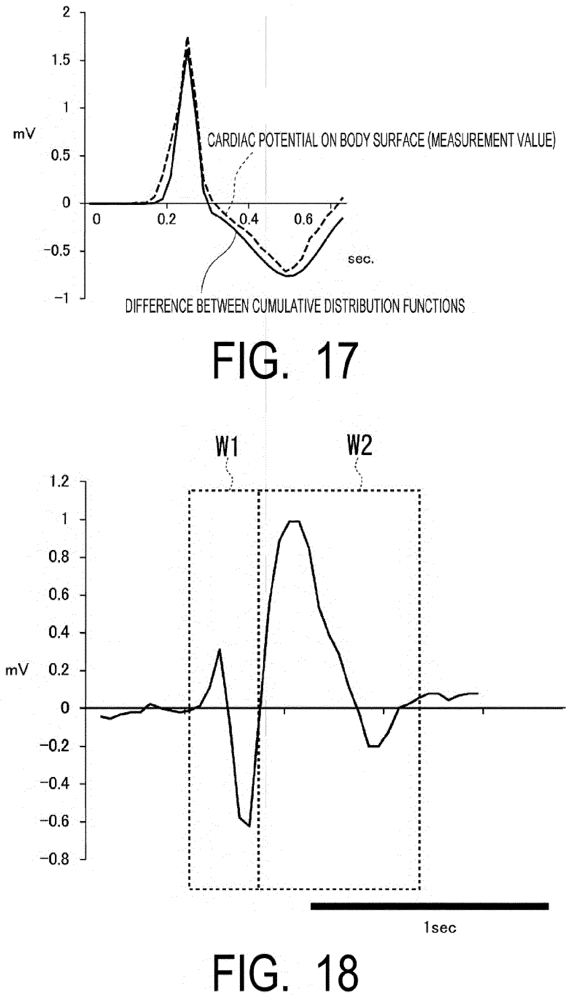

Fig. 15 is a first explanatory diagram for describing an example in which an electrocardiogram of a ventricular premature contraction is analyzed by the signal analysis device 1 of the embodiment. Fig. 16 is a second explanatory diagram of the example in which the electrocardiogram of the ventricular premature contraction is analyzed by the signal analysis device 1 of the embodiment. Fig. 17 is a third explanatory diagram of the example in which the electrocardiogram of the ventricular premature contraction is analyzed by the signal analysis device 1 of the embodiment.

More specifically, Fig. 15 illustrates a cardiac potential of the body surface. That is, Fig 15 illustrates normal one-time heartbeat recorded in the electrocardiogram and two consecutive ventricular premature contractions. More specifically, Fig. 16 illustrates a myocardial inner layer activity approximation function including an inner layer side cumulative distribution function of depolarization and an inner layer side cumulative distribution function in a repolarization phase of a ventricular premature contraction analyzed by the signal analysis device 1, and a myocardial outer layer activity approximation function including an outer layer side cumulative distribution function of depolarization and an outer layer side cumulative distribution function in a repolarization phase of the ventricular premature contraction analyzed by the signal analysis device 1. More specifically, Fig. 17 illustrates an example of a waveform of a ventricular premature contraction of an actually measured electrocardiogram.

Fig. 16 illustrates that the inner layer side cumulative distribution function of the depolarization precedes the outer layer side cumulative distribution function, and a standard deviation of the inner layer side cumulative distribution function and the outer layer side cumulative distribution function is larger than that of a normal heartbeat, and a spread of excitation is gentle. This analysis result corresponds to characteristics of a waveform of an R wave having a wide skirt.

Fig. 16 corresponds to a fact that an inner layer side cumulative distribution function starts deactivation earlier than an outer layer side cumulative distribution function in a repolarization phase, and illustrates an order of deactivation on the inner layer side and the outer layer side as a magnitude relationship between average values of two cumulative distribution functions in the repolarization phase. A function obtained by subtracting the outer layer side cumulative distribution function from the inner layer side cumulative distribution function in Fig. 16 corresponds to characteristics of a large negative T wave in the repolarization phase, and as illustrated in Fig. 17, a waveform obtained by subtracting the outer layer side cumulative distribution function from the inner layer side cumulative distribution function substantially corresponds to a waveform of the ventricular premature contraction of the actually measured electrocardiogram.

Note that results of Figs. 15 to 17 indicate that analysis by the signal analysis device 1 corresponds to an example in which a giant wave or a negative potential is generated due to modified conduction of excitation of the myocardium, early repolarization, or delay of repolarization. Note that, in Figs. 15 to 17, an average $\mu$ of the inner layer side cumulative distribution function in the depolarization phase is -1, and a standard deviation $\sigma$ is 0.32. Furthermore, in Figs. 15 to 17, an average $\mu$ of the outer layer side cumulative distribution function in the depolarization is -0.8, and a standard deviation $\sigma$ is 0.21. Furthermore, in Figs. 15 to 17, an average $\mu$ of the inner layer side cumulative distribution function in the repolarization phase is 1, and a standard deviation $\sigma$ is 1. Furthermore, in Figs. 15 to 17, an average $\mu$ of the outer layer side cumulative distribution function in the repolarization phase is 2.99, and a standard deviation $\sigma$ is 0.7.

With reference to Figs. 18 to 20, it will be described that information indicating activity of a myocardium can be acquired only from an electrocardiogram by the signal analysis device 1 also for an electrocardiogram of a target heart in a depolarization period of Brugada syndrome type 1. In Figs. 18 to 20, the vertical axis represents a potential in millivolts.

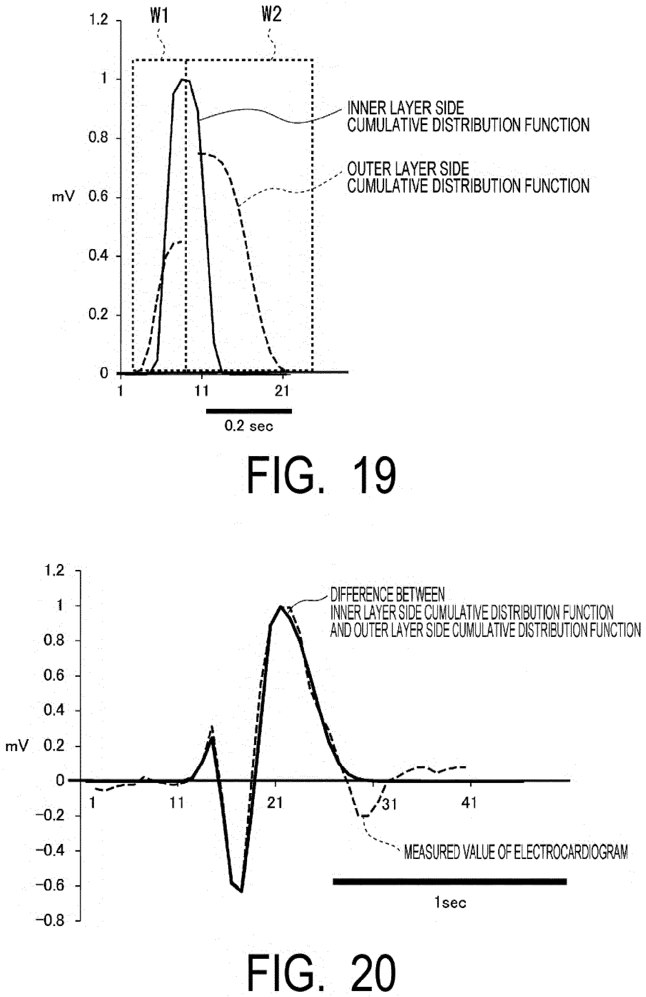

Fig. 18 is a first explanatory diagram for describing an example in which an electrocardiogram of a target heart in a depolarization period of Brugada syndrome type 1 is analyzed by the signal analysis device 1 of the embodiment. Fig. 19 is a second explanatory diagram of the example in which the electrocardiogram of the target heart in the depolarization period of Brugada syndrome type 1 is analyzed by the signal analysis device 1 of the embodiment. Fig. 20 is a third explanatory diagram of the example in which the electrocardiogram of the target heart in the depolarization period of Brugada syndrome type 1 is analyzed by the signal analysis device 1 of the embodiment.

More specifically, Fig. 18 illustrates the electrocardiogram of chest second lead of Brugada syndrome. In Fig. 18, an inner frame W1 indicates a depolarization phase, and an inner frame W2 indicates a repolarization phase. The same applies to Fig. 19. That is, also in Fig. 19, the inner frame W1 represents the depolarization phase, and the inner frame W2 represents the repolarization phase.

More specifically, Fig. 19 illustrates an inner layer side cumulative distribution function and an outer layer side cumulative distribution function in the depolarization and repolarization phase analyzed by the signal analysis device 1. In the example illustrated in Fig. 19, the repolarization phase of the inner layer side cumulative distribution function starts following the depolarization phase, indicating characteristics of early repolarization. On the other hand, in the example of Fig. 19, as for a potential amplitude of the outer layer side cumulative distribution function, a difference is observed between the depolarization phase and the repolarization phase. Furthermore, Fig. 19 illustrates a gap and anisotropy between the depolarization phase and the repolarization phase of the outer layer side cumulative distribution function. As described above, the signal analysis device 1 can express early repolarization and anisotropy between depolarization and repolarization that are characteristics of a waveform indicated by an electrocardiogram of a target heart of Brugada syndrome by averages and standard deviations of a depolarization phase and a repolarization phase of an inner layer side cumulative distribution function and an outer layer side cumulative distribution function, and a ratio of weight given to the outer layer side cumulative distribution function to weight given to the inner layer side cumulative distribution function (inner outer layer ratio).

Fig. 20 is a comparison between an analysis result and a measured value. More specifically, Fig. 20 illustrates a difference between the inner layer side cumulative distribution function and the outer layer side cumulative distribution function in the depolarization phase and the repolarization phase in Fig. 19. Fig. 20 also illustrates the measured value of the electrocardiogram. Except for the tail end, the analysis result and the measured value substantially correspond to each other. The tail end means a potential at a later time.

Note that, in Figs. 18 to 20, an average $\mu$ of the inner layer side cumulative distribution function in the depolarization phase is 15, and a standard deviation $\sigma$ is 0.15. Furthermore, in Figs. 18 to 20, an average $\mu$ of the outer layer side cumulative distribution function in the depolarization phase is 14, and a standard deviation $\sigma$ is 0.25. In Figs. 18 to 20, an inner outer layer ratio of the depolarization phase is 0.45. Furthermore, in Figs. 18 to 20, an average $\mu$ of the inner layer side cumulative distribution function in the repolarization phase is 25, and a standard deviation $\sigma$ is 0.25. Furthermore, in Figs. 18 to 20, an average $\mu$ of the outer layer side cumulative distribution function in the repolarization phase is 20, and a standard deviation $\sigma$ is 0.5.

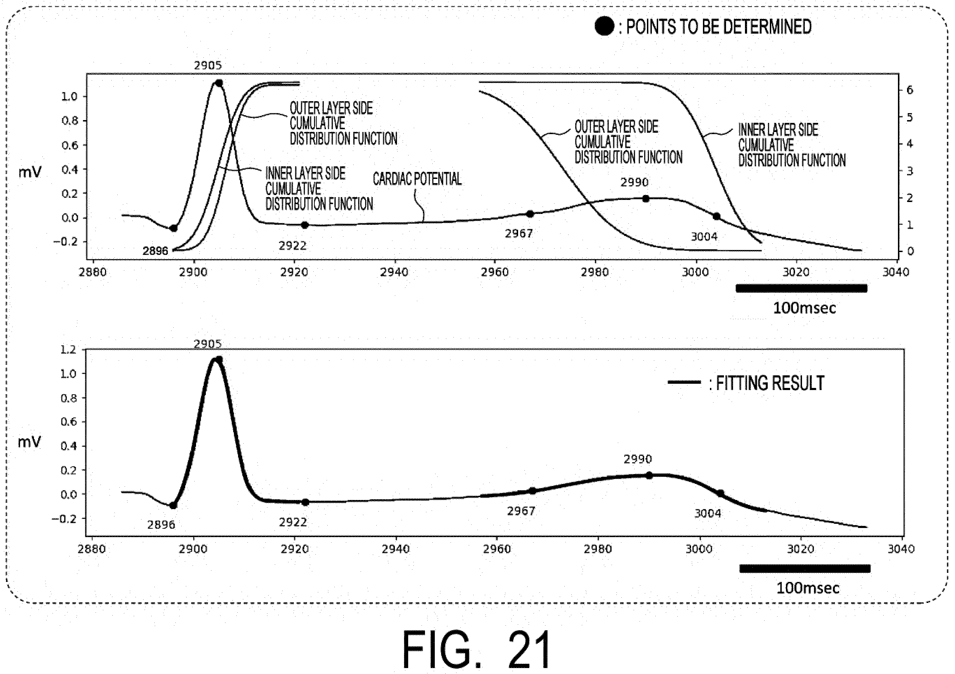

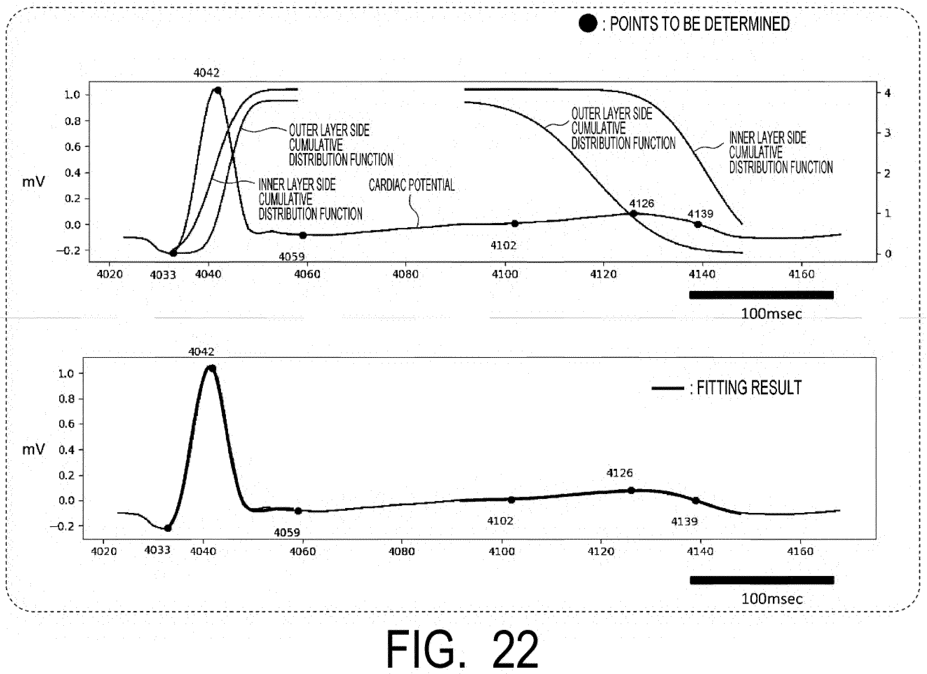

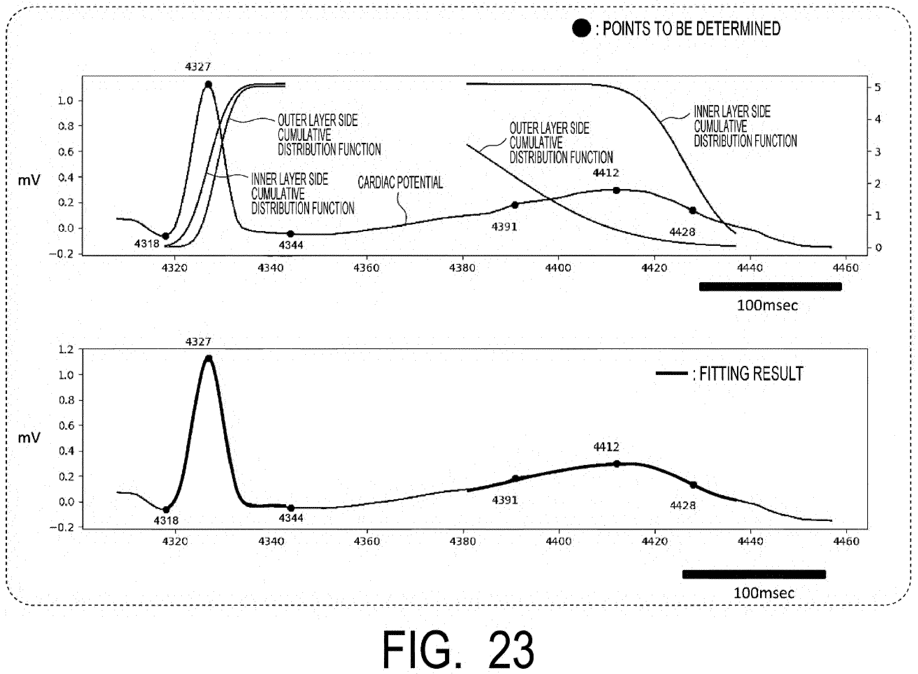

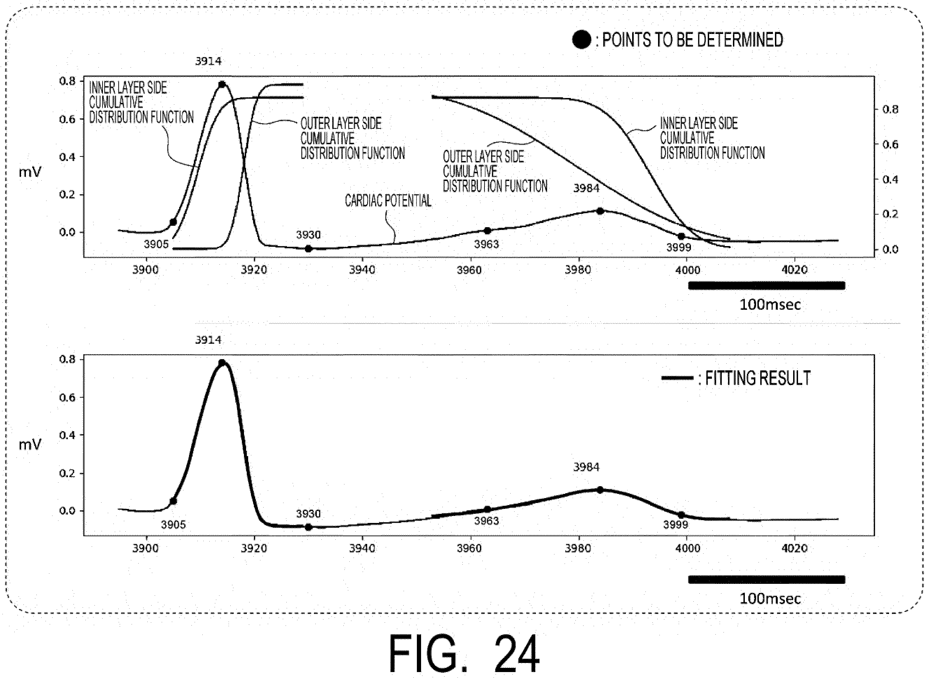

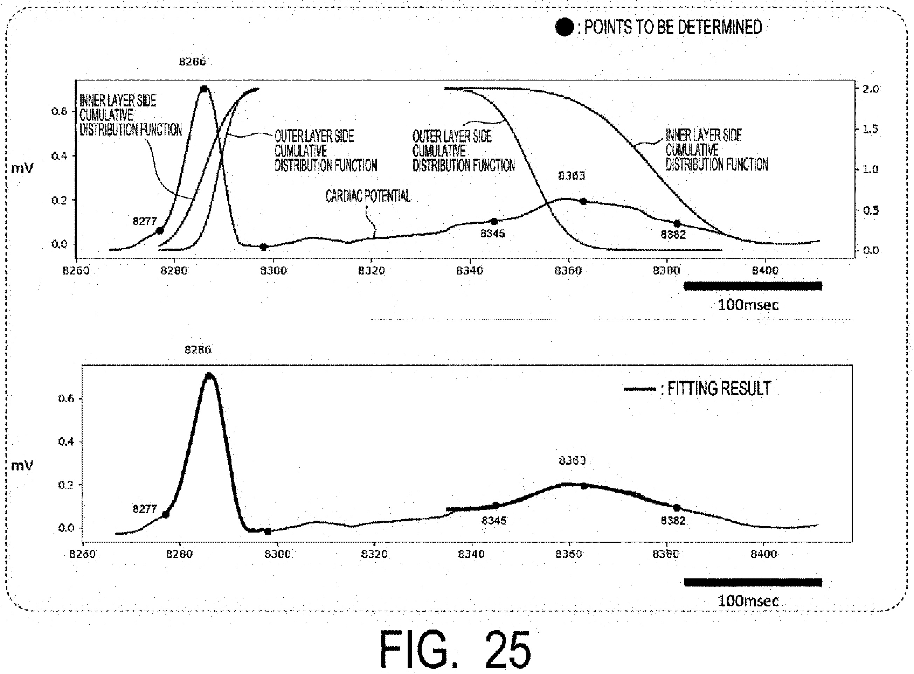

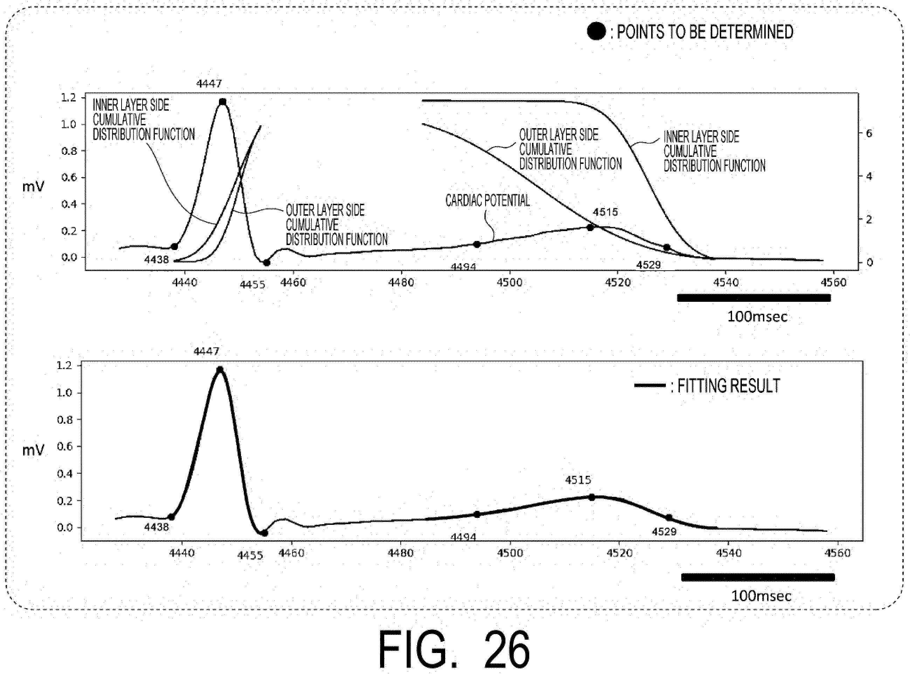

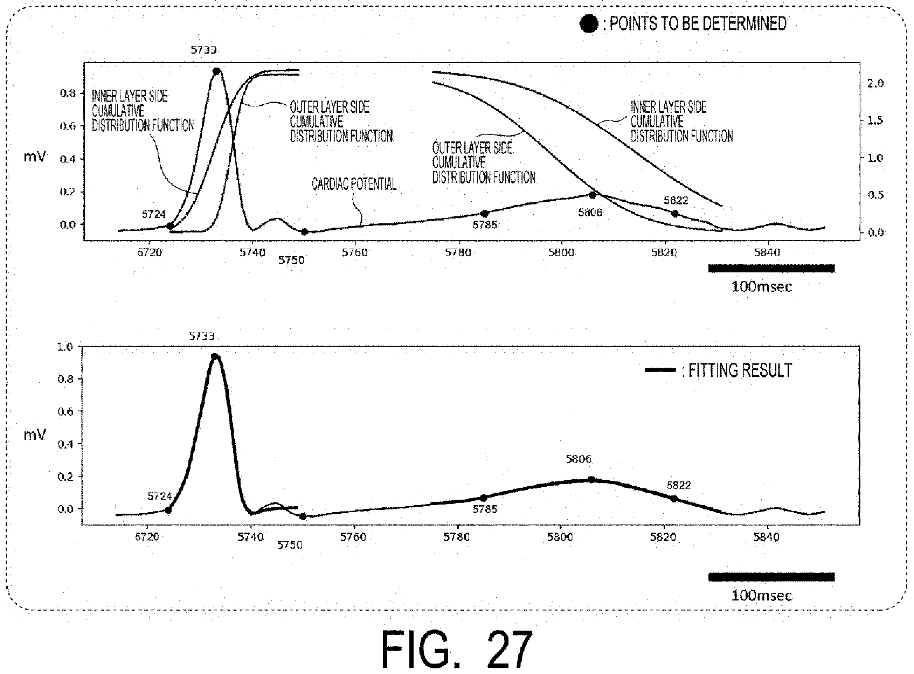

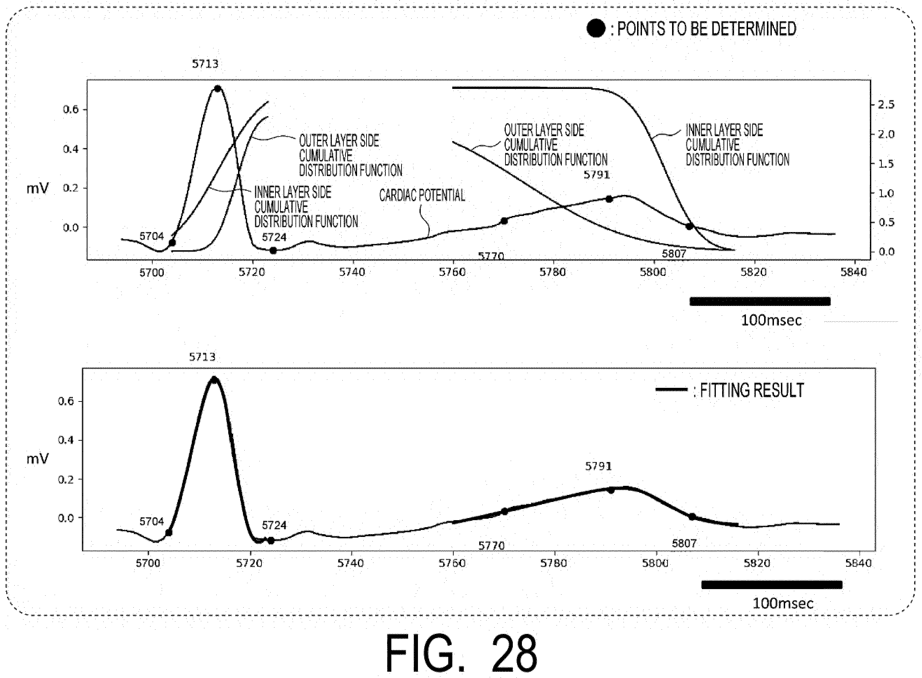

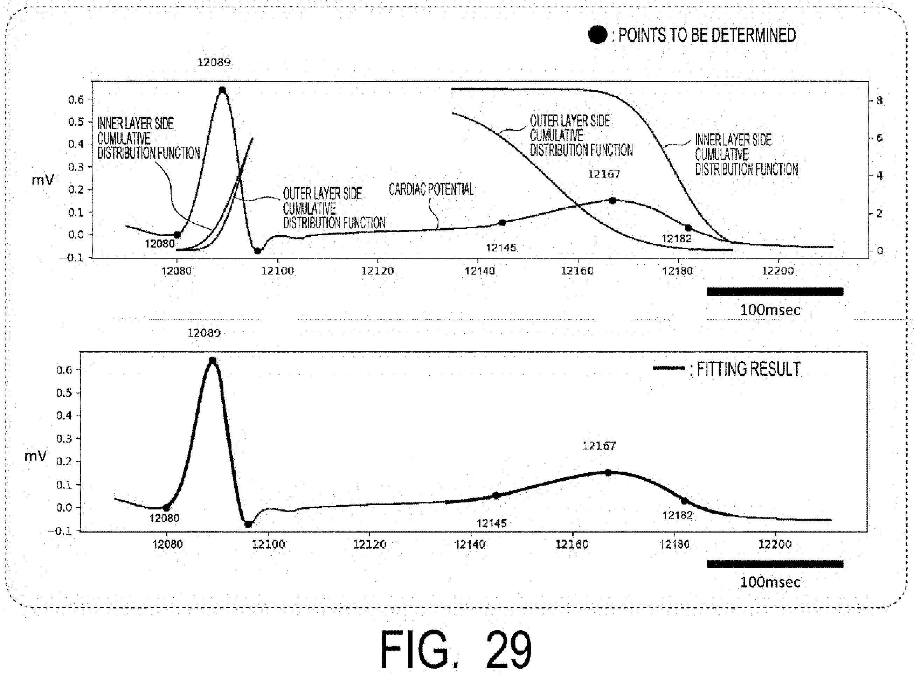

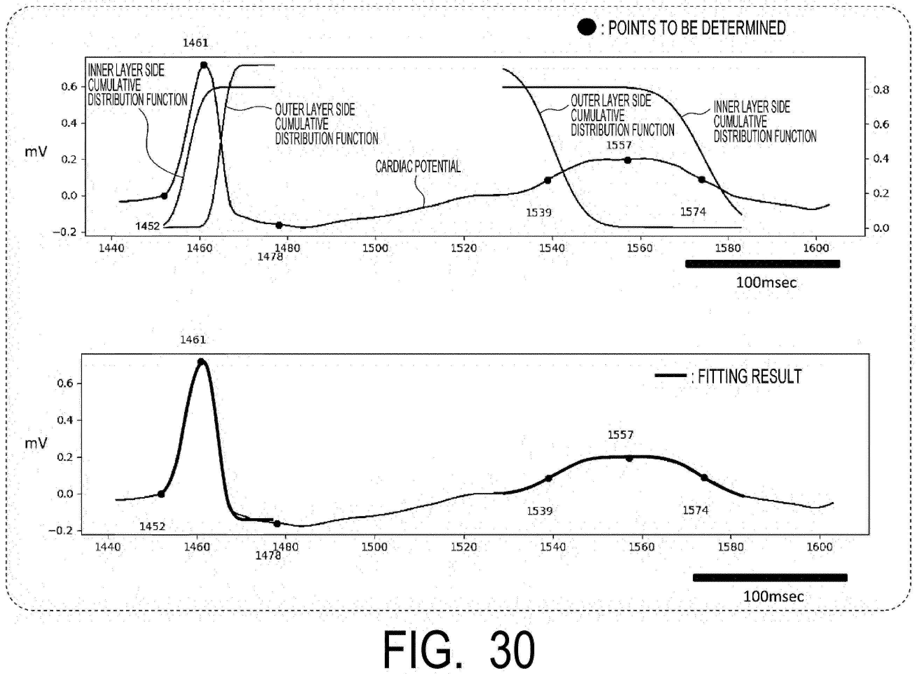

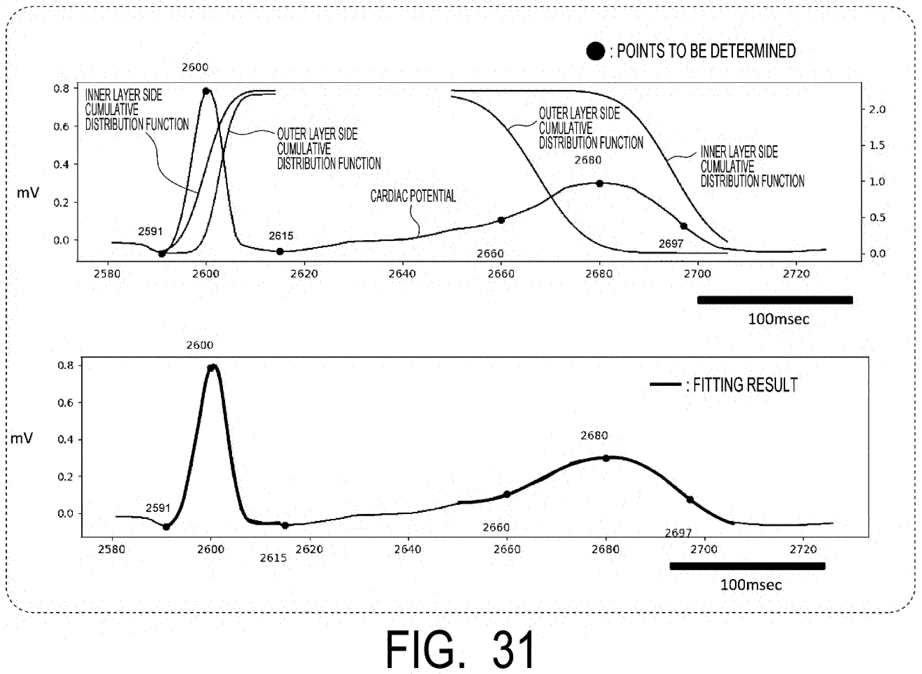

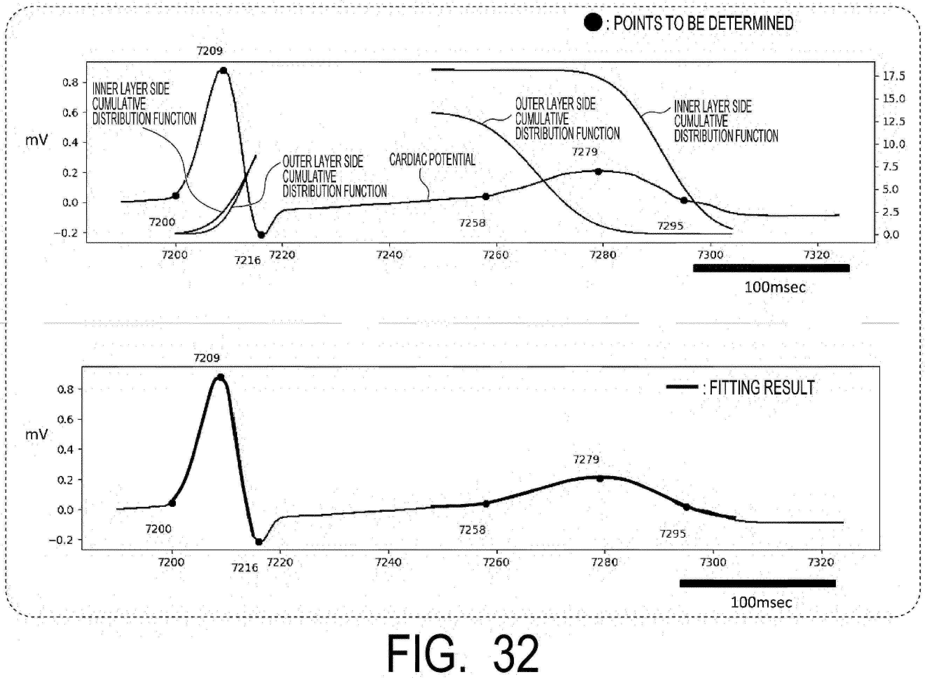

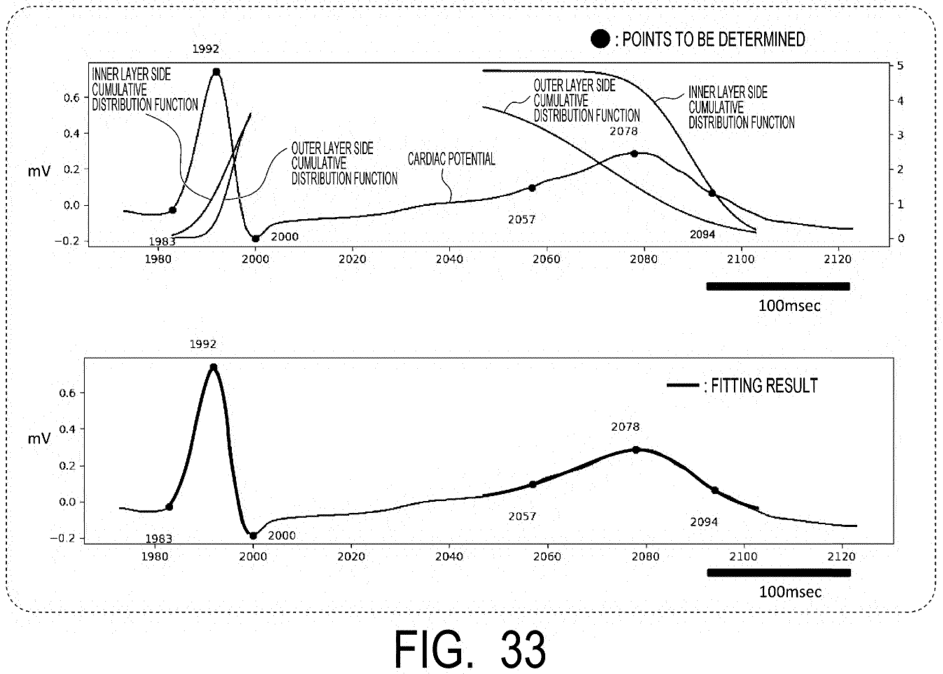

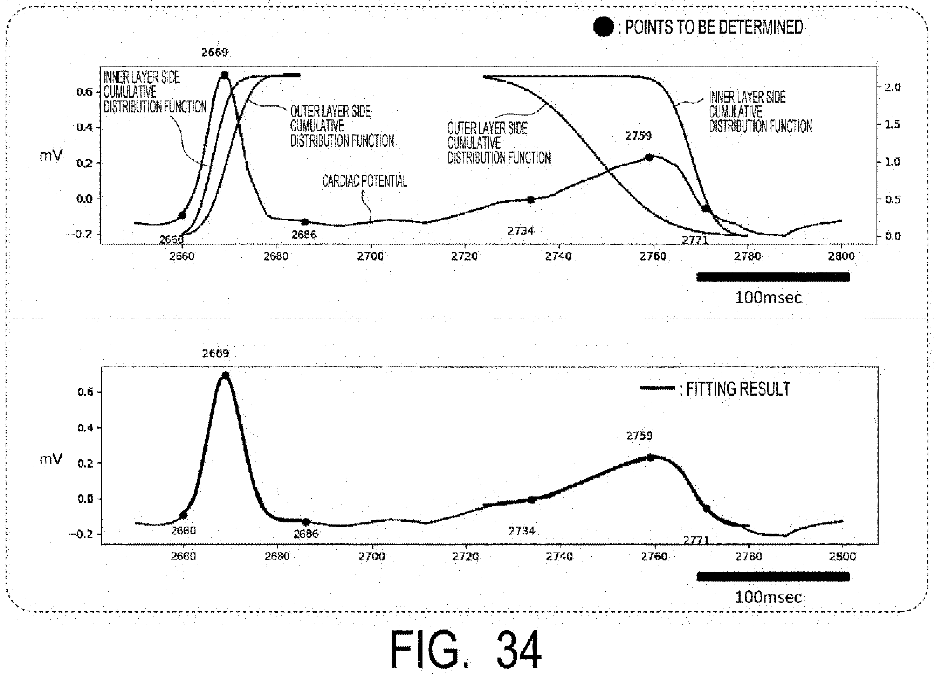

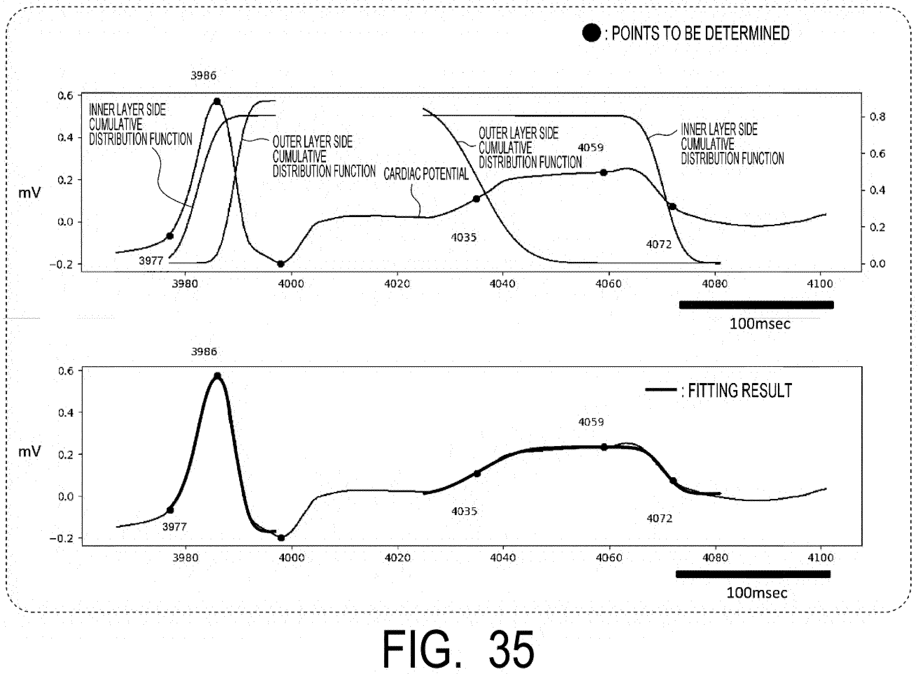

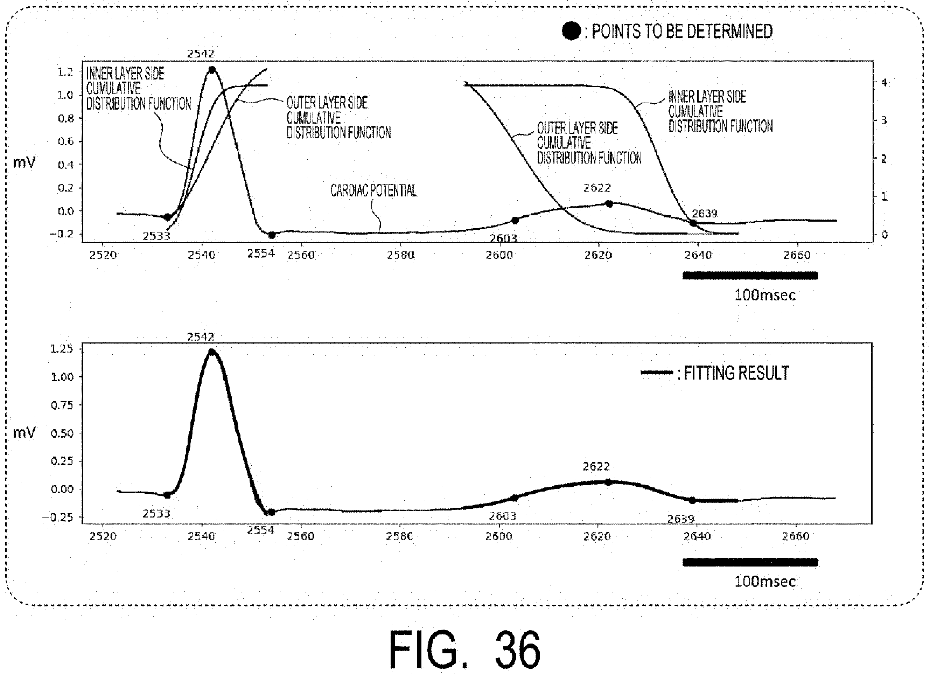

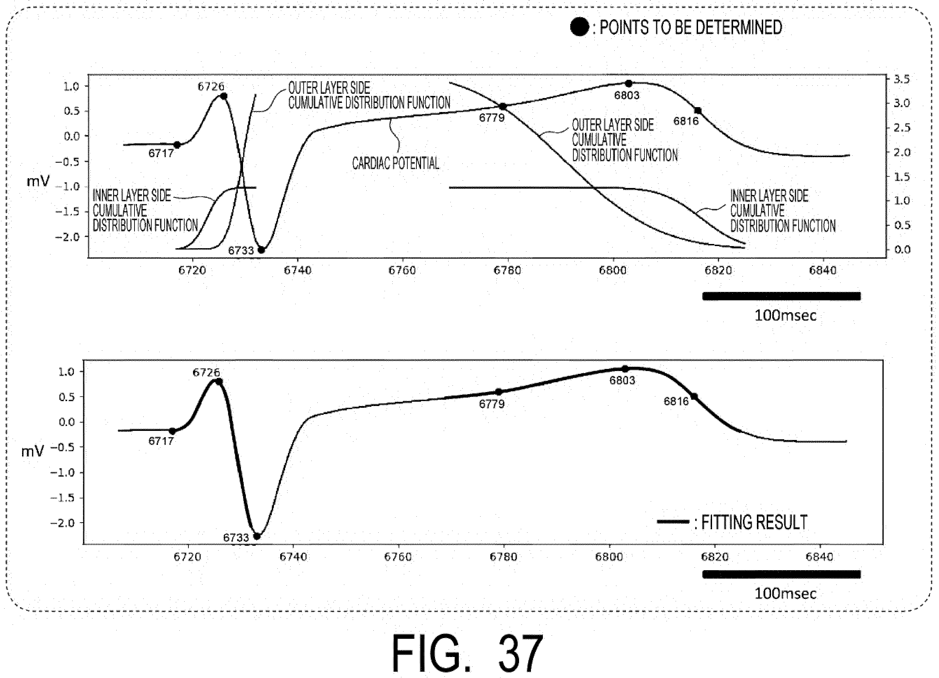

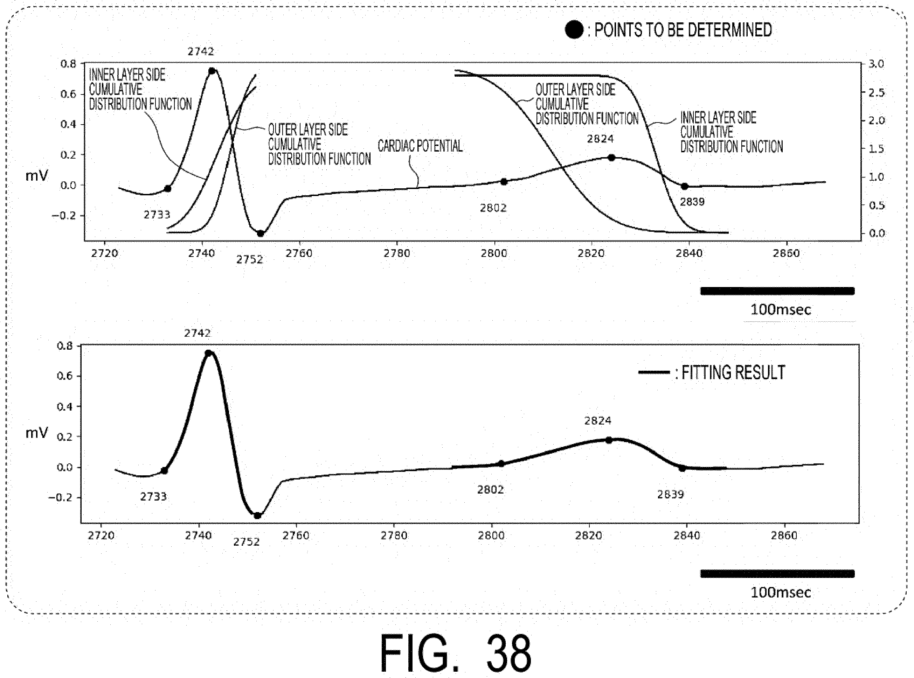

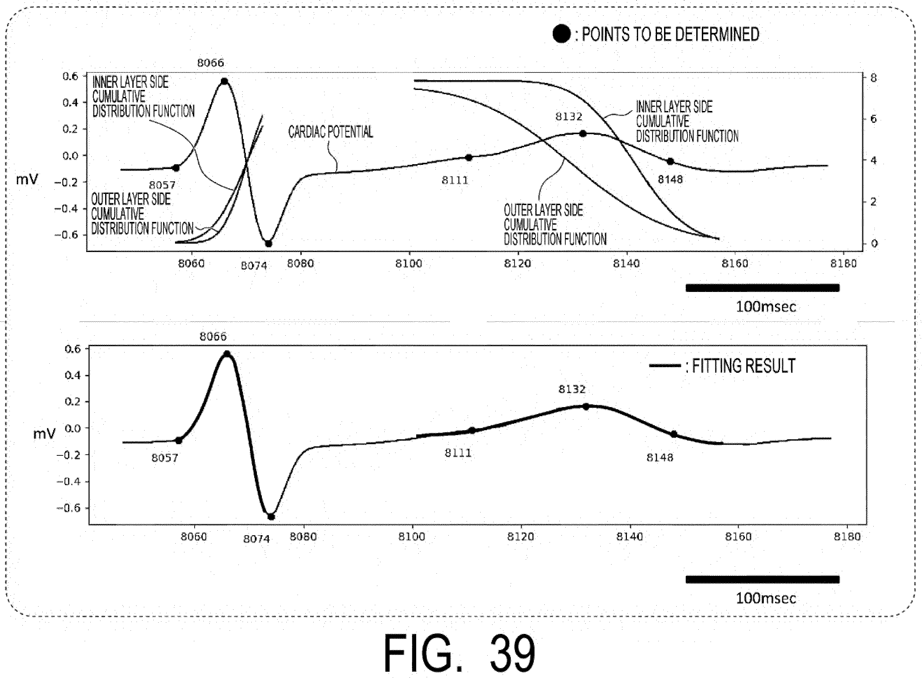

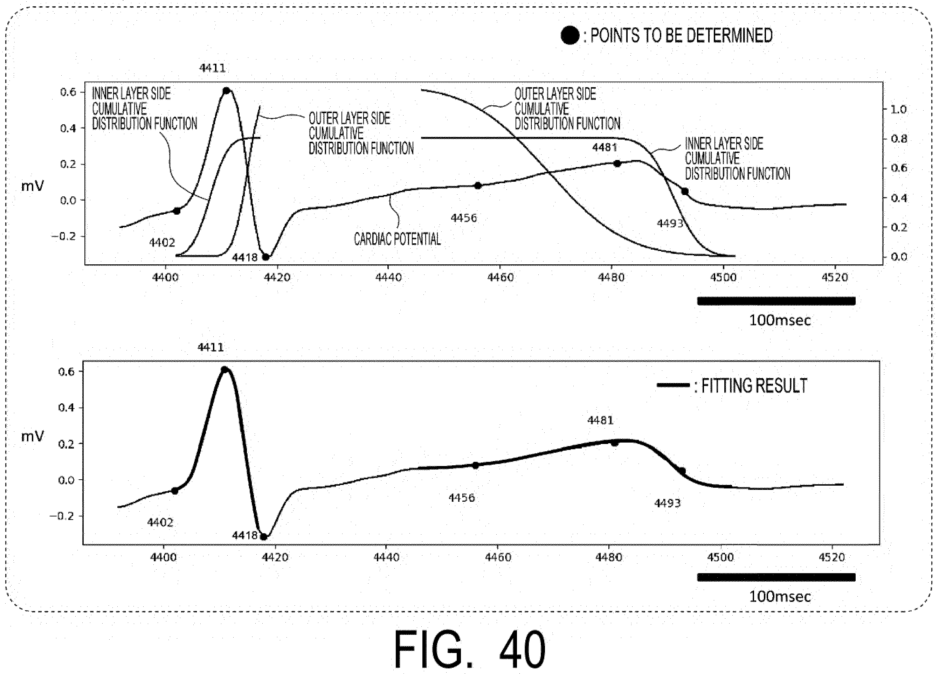

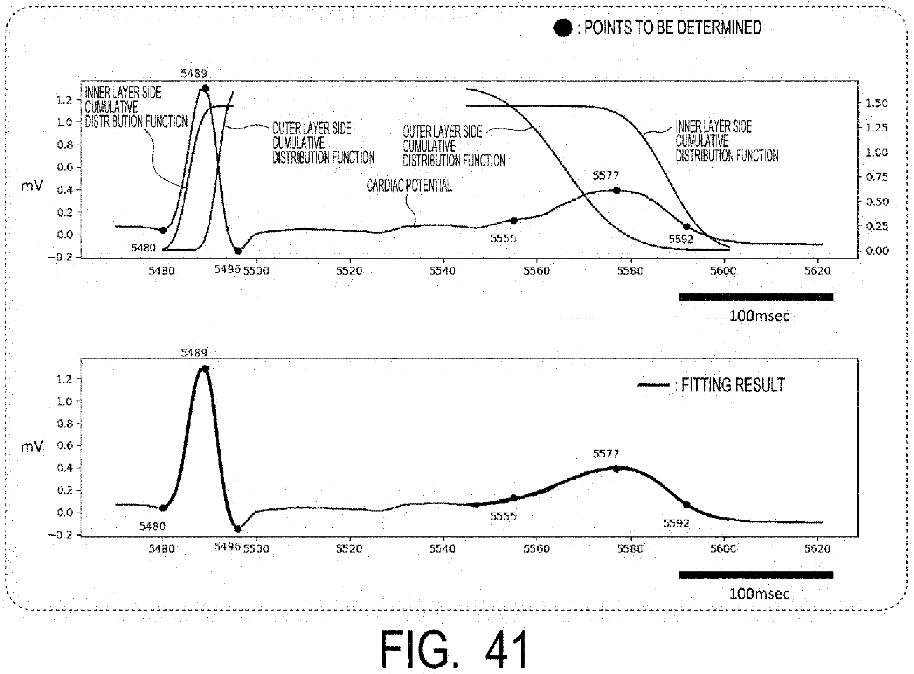

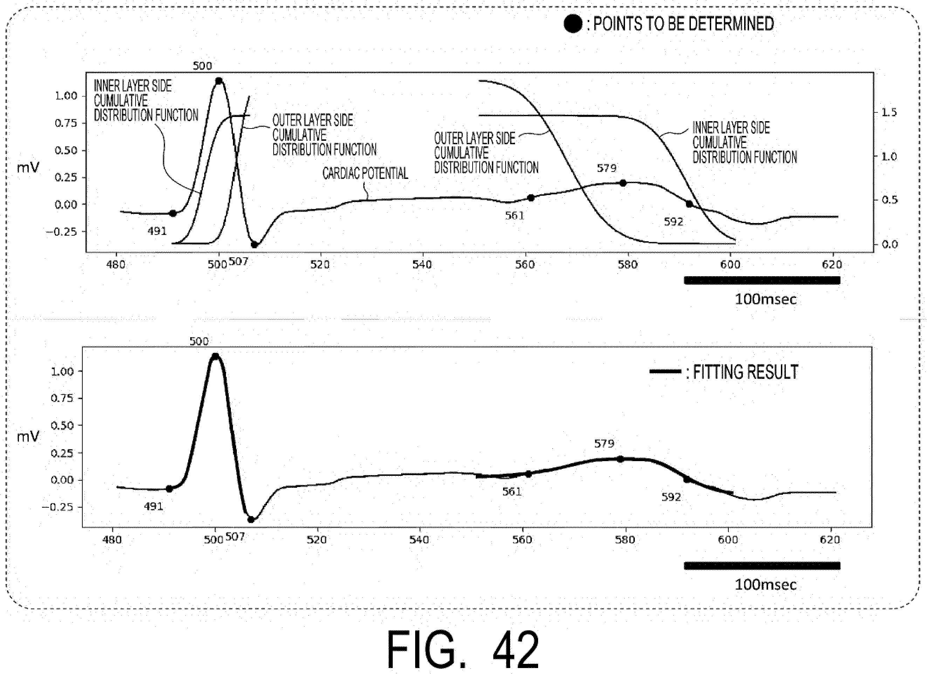

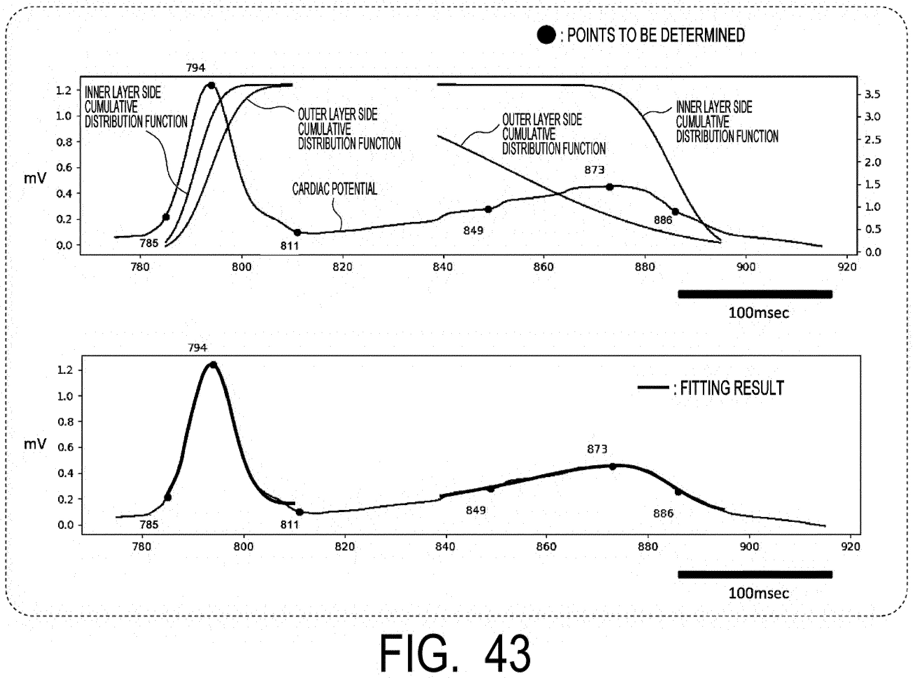

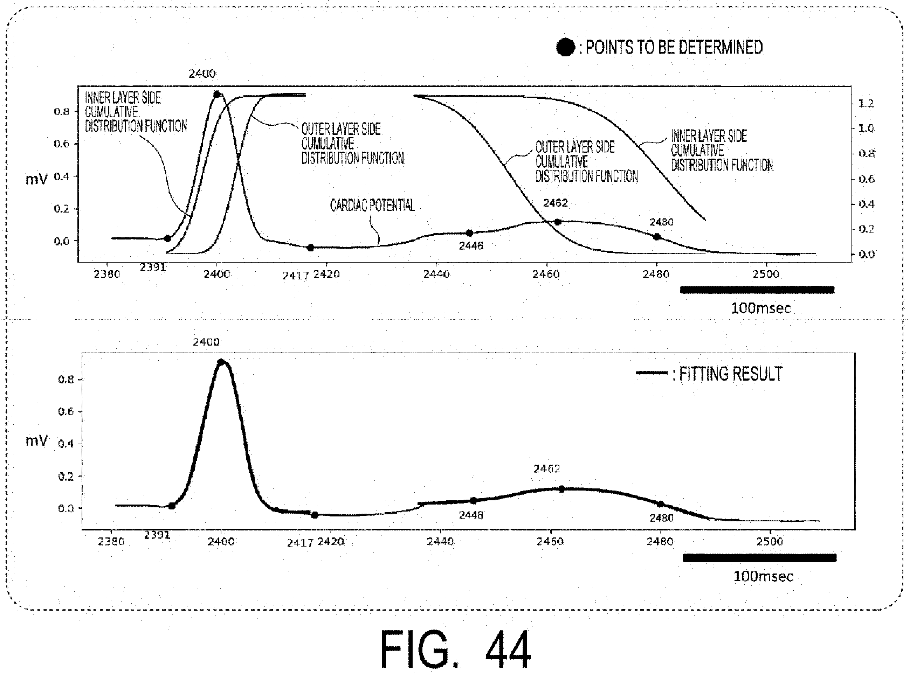

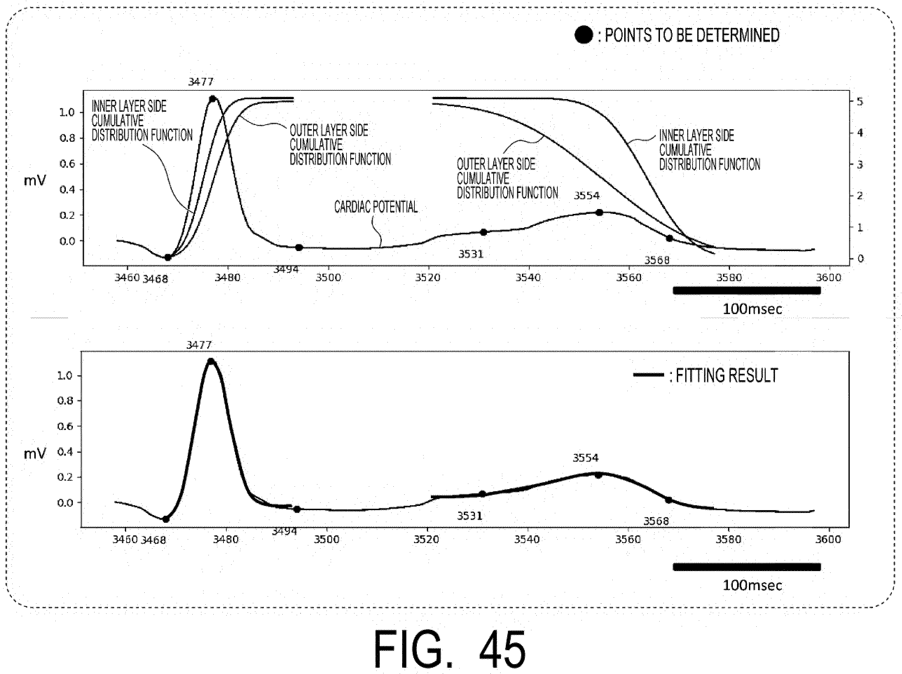

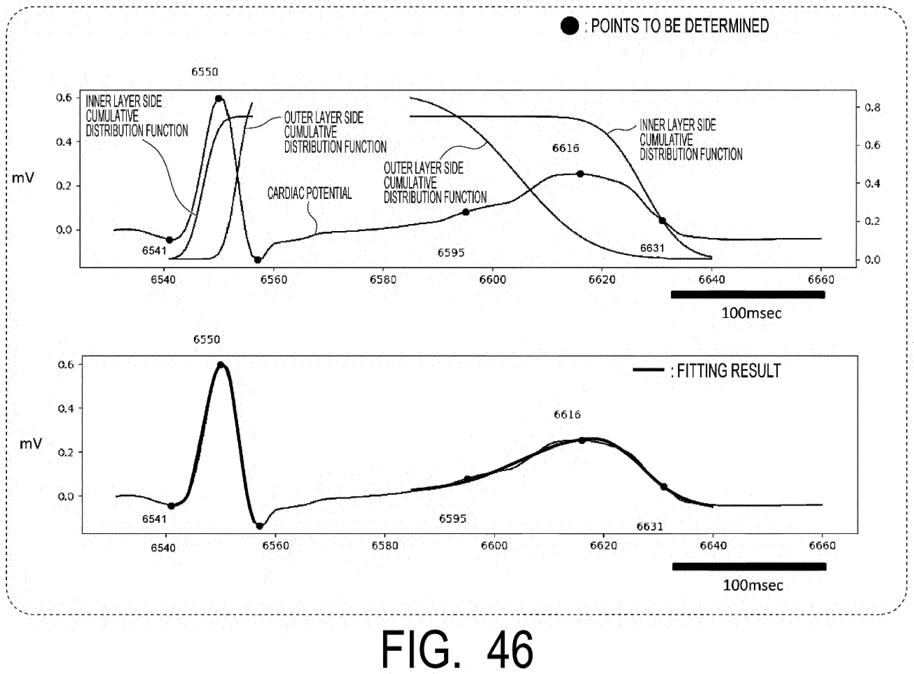

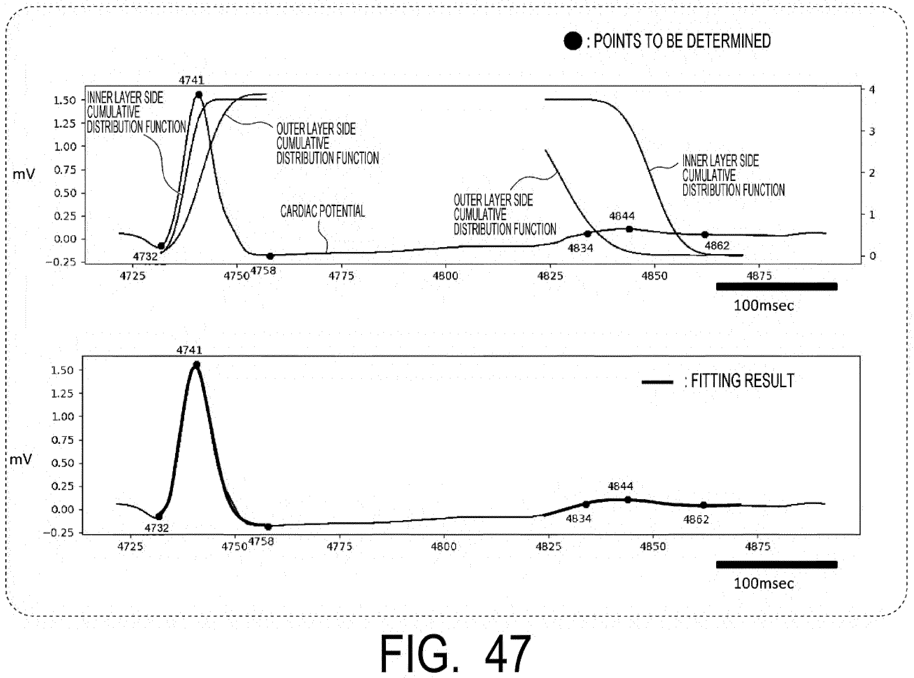

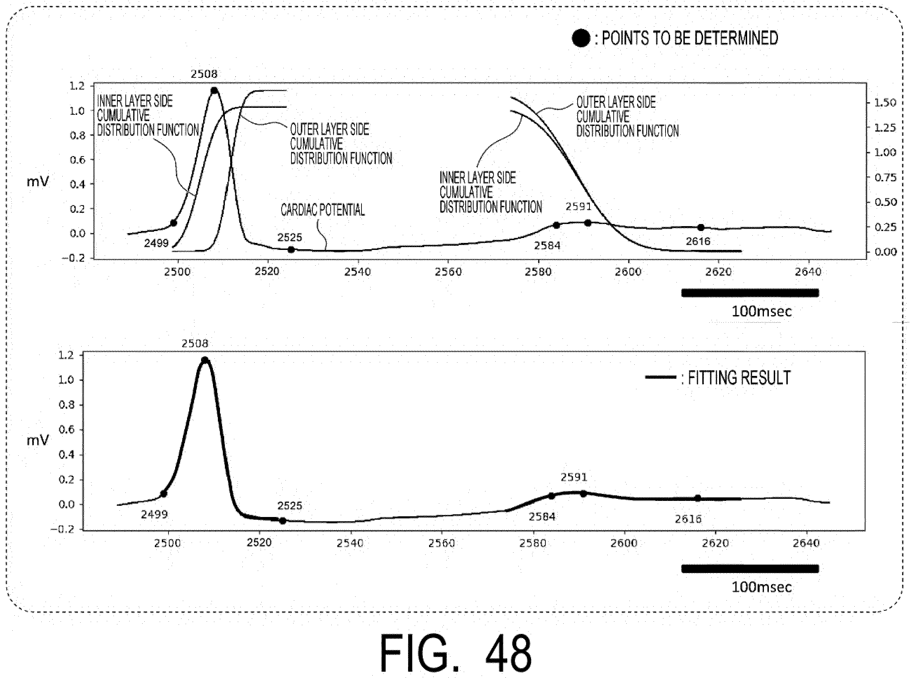

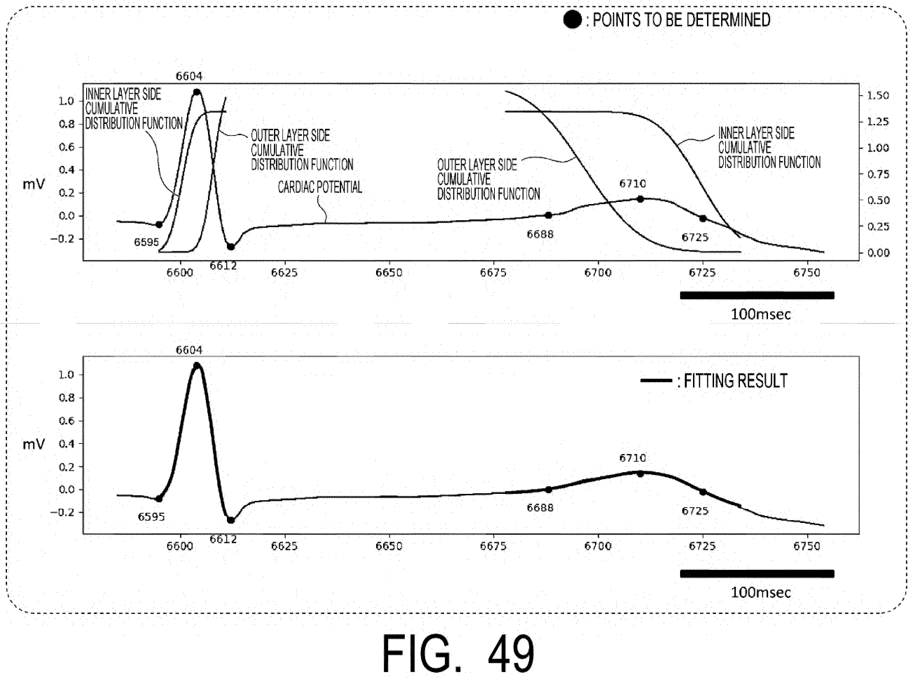

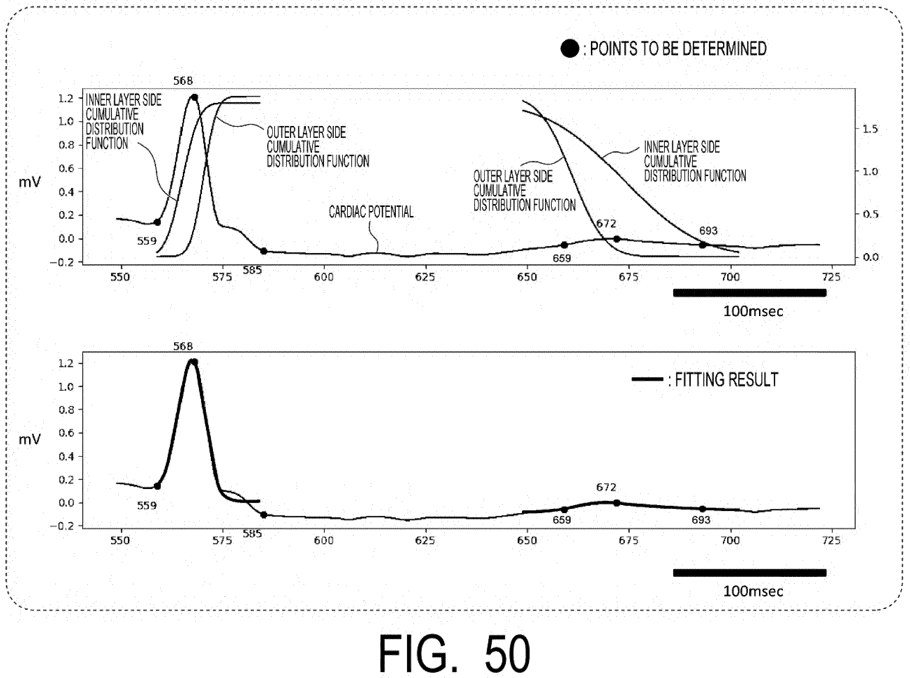

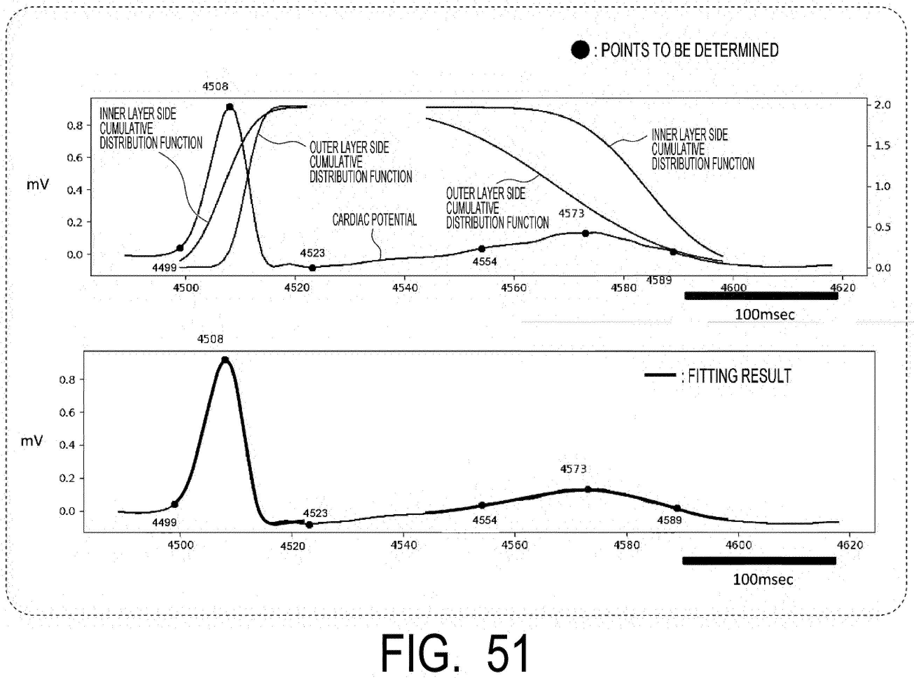

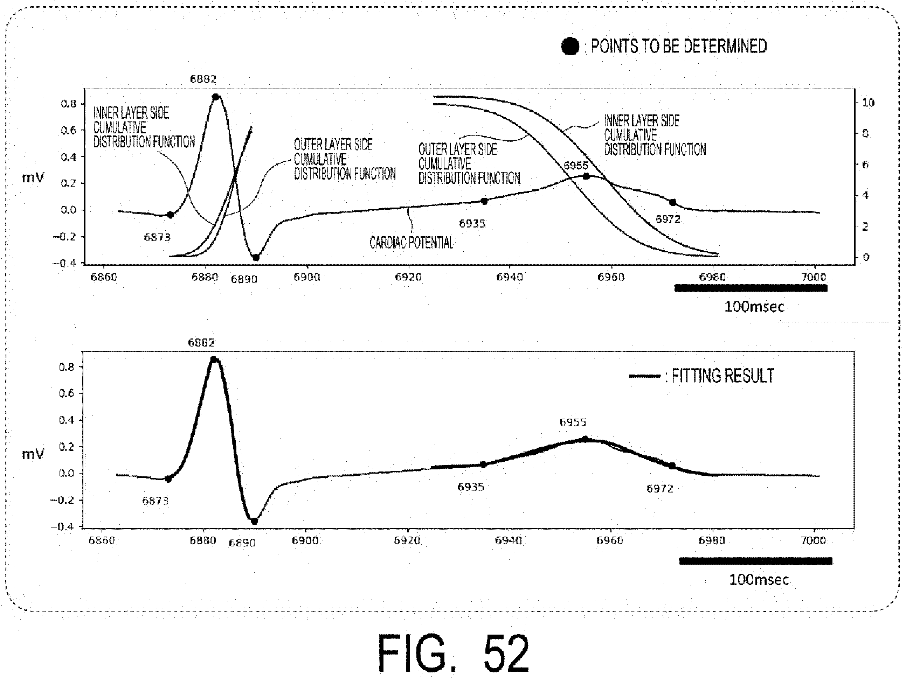

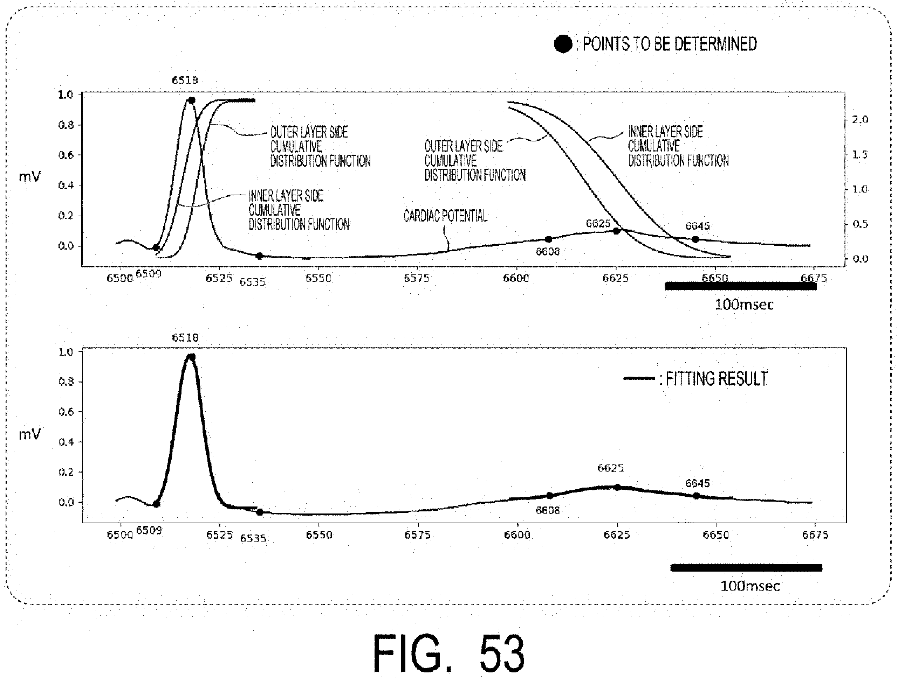

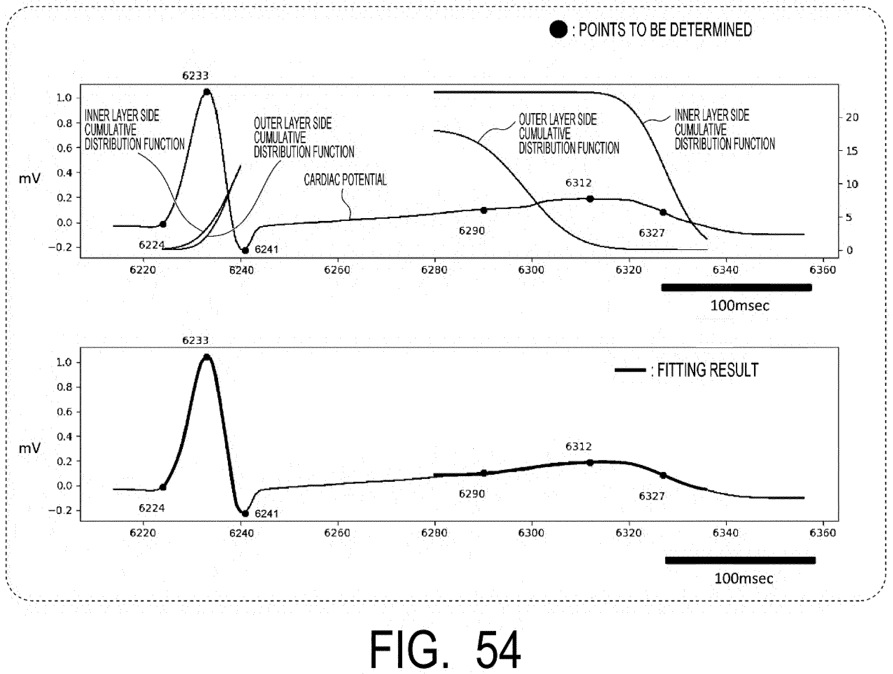

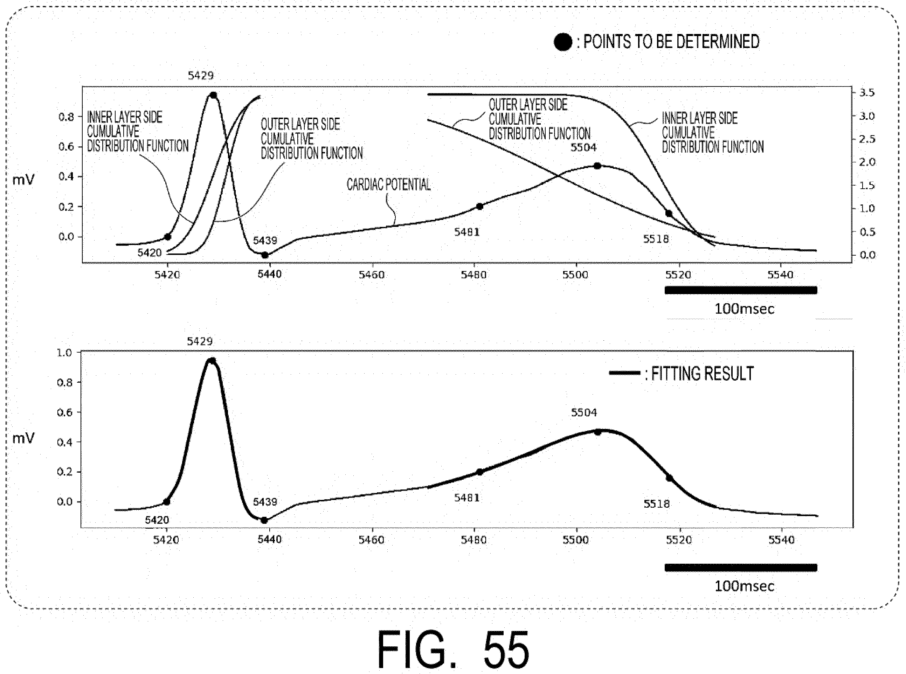

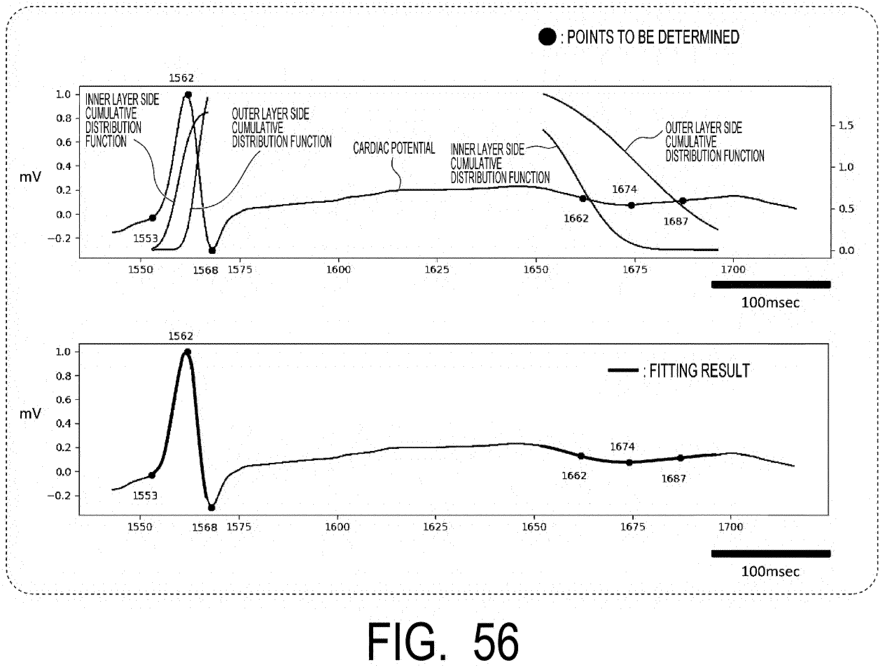

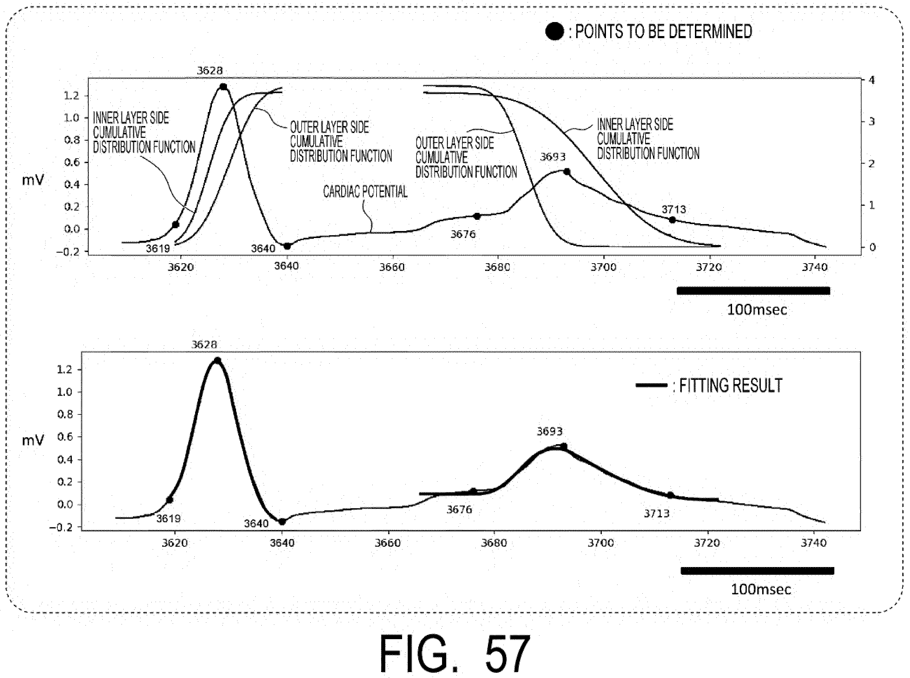

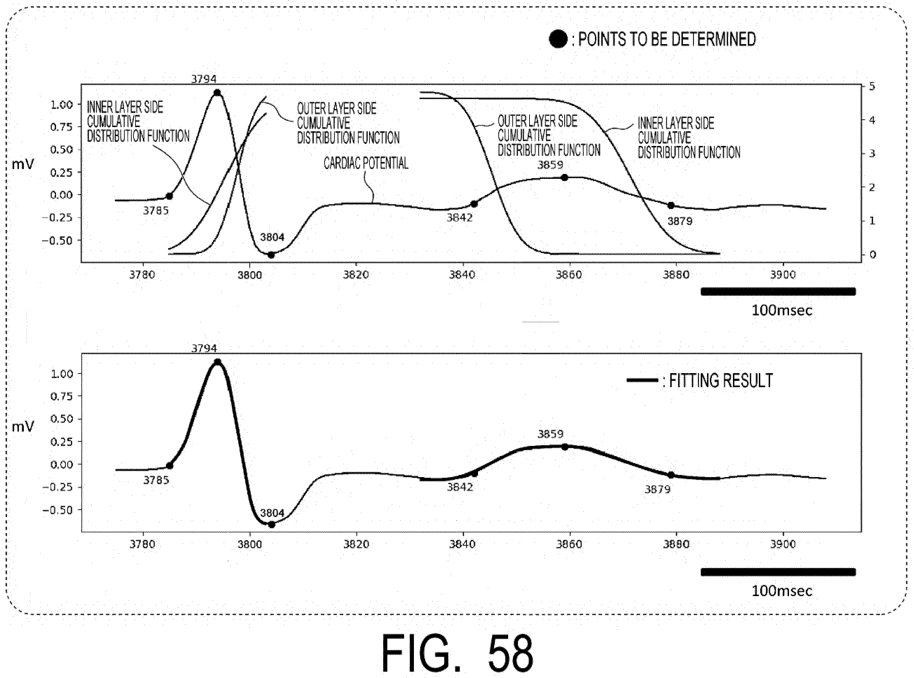

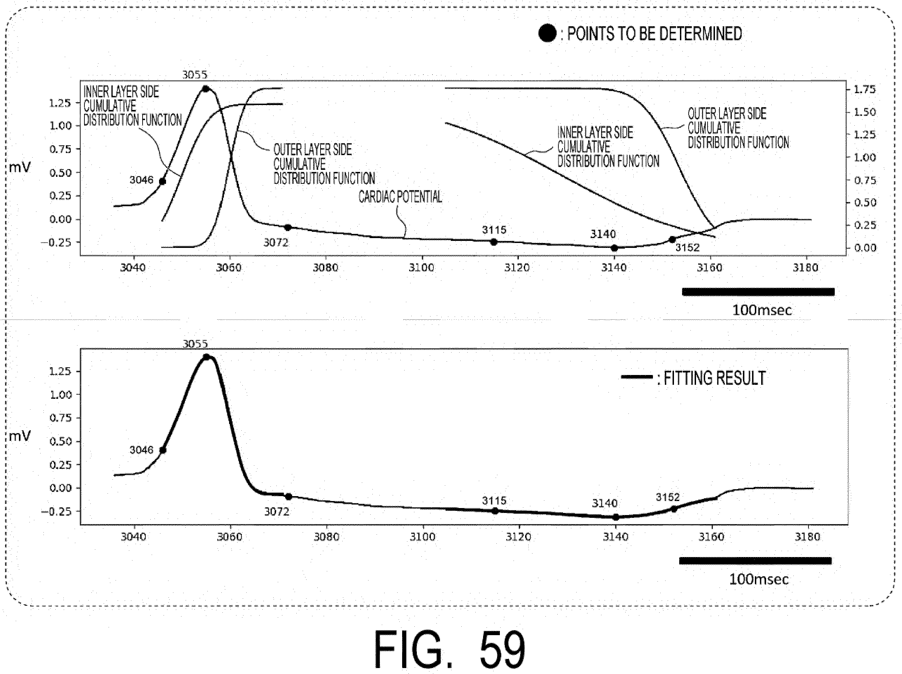

Figs. 21 to 59 illustrate results of analysis performed by the signal analysis device 1 using a public library of electrocardiogram data <https://physionet.org/about/database/>. Figs. 21 to 59 illustrate an inner layer side cumulative distribution function, an outer layer side cumulative distribution function, and a fitting result obtained as a result of performing analysis on a cardiac potential by the signal analysis device 1.

Each of Figs. 21 to 59 is a diagram illustrating an example in which an electrocardiogram is analyzed by the signal analysis device 1 according to the embodiment. Points to be determined illustrated in each diagram represent a Q point, an R point, an S point, a T start point, and a T end point of a cardiac potential in order from the left of the diagram. The points to be determined was determined by an inflection point detection and peak detection algorithm. Each diagram of Figs. 21 to 59 illustrates an inner layer side cumulative distribution function and an outer layer side cumulative distribution function in each section obtained for a section of a depolarization phase (QRS wave) and a section of a repolarization phase (T wave). Figs. 21 to 59 illustrate that, for various QRS waves and T waves, the signal analysis device 1 can express substantially the same shape by adjusting averages and standard deviations of the inner layer side cumulative distribution function and the outer layer side cumulative distribution function. Lower diagrams of Figs. 21 to 59 illustrate original waveforms of electrocardiograms and fitting results. Note that each result in Figs. 21 to 59 is a result of sampling at 300 Hz. Therefore, in the horizontal axis in each diagram of Figs. 21 to 59, the origin represents 0 second and a value 1 represents 3.33 milliseconds.

The signal analysis device 1 formed as described above, assuming a waveform of a time section of an R wave or a T wave included in a waveform for one cycle of a cardiac cycle of a target heart as a target time waveform, upon approximating the target time waveform by an approximate time waveform that is a time waveform obtained by a difference or a weighted difference between a first cumulative distribution function that is a cumulative distribution function of a first unimodal distribution and a second cumulative distribution function, acquires a parameter identifying the first unimodal distribution or a parameter identifying the first cumulative distribution function and a parameter identifying the second unimodal distribution or a parameter identifying the second cumulative distribution function as parameters representing characteristics of the target time waveform. The first cumulative distribution function is information indicating activity of a myocardial inner layer of the target heart, and the second cumulative distribution function is information indicating activity of a myocardial outer layer of the target heart. Therefore, a parameter representing the shape of the first cumulative distribution function is a parameter indicating the activity of the myocardial inner layer of the target heart (myocardial inner layer parameter), and a parameter representing the shape of the second cumulative distribution function is a parameter indicating the activity of the myocardial outer layer of the target heart (myocardial outer layer parameter). Information clearly indicating characteristics of the activity of the myocardial inner layer of the target heart and information clearly indicating characteristics of the activity of the myocardial outer layer of the target heart including these parameters cannot be obtained by conventional analysis of a waveform of an electrocardiogram. Therefore, according to the signal analysis device 1, useful information for grasping the state of a heart can be obtained from a waveform of an electrocardiogram.

[Acquisition of Only Some Parameters]

In a case where only characteristics of activity of a myocardial inner layer of a target heart is desired to be grasped, only a myocardial inner layer parameter may be acquired by the signal analysis device 1, and in a case where only characteristic of activity of a myocardial outer layer of the target heart is desired to be grasped, only a myocardial outer layer parameter may be acquired by the signal analysis device 1. Furthermore, only some parameters of parameters representing the shape of a cumulative distribution function may be acquired by the signal analysis device 1 as a myocardial inner layer parameter or a myocardial outer layer parameter.

For example, upon approximating a first target time waveform that is a waveform of a time section of an R wave of a target heart by a first approximate time waveform that is a time waveform obtained by a difference or a weighted difference between a first cumulative distribution function that is a cumulative distribution function of a first unimodal distribution and a second cumulative distribution function that is a cumulative distribution function of a second unimodal distribution, or by a first approximate time waveform that is a time waveform obtained by adding a level value to a difference or a weighted difference between a first cumulative distribution function that is a cumulative distribution function of a first unimodal distribution and a second cumulative distribution function that is a cumulative distribution function of a second unimodal distribution, the myocardial activity information parameter acquisition unit 132 of the analysis unit 130 (analyzer) of the signal analysis device 1 acquires at least one of at least some parameters of parameters identifying the first unimodal distribution, at least some parameters of parameters identifying the first cumulative distribution function, at least some parameters of parameters identifying the second unimodal distribution, or at least some parameters of parameters identifying the second cumulative distribution function as a myocardial activity parameter that is a parameter indicating activity of a myocardium of the target heart.

For example, in a case where both the first unimodal distribution and the second unimodal distribution are Gaussian distributions, the myocardial activity information parameter acquisition unit 132 needs to acquire at least one of an average value of the first unimodal distribution, a standard deviation or variance of the first unimodal distribution, an average value of the second unimodal distribution, or a standard deviation or variance of the second unimodal distribution as a myocardial activity parameter. Note that an average value of a Gaussian distribution is time at which the frequency value is the maximum value in a unimodal distribution, and is time at which the slope of a cumulative distribution function of the unimodal distribution is the maximum. Therefore, for example, regardless of whether the first unimodal distribution and the second unimodal distribution are Gaussian distributions, the myocardial activity information parameter acquisition unit 132 needs to acquire at least one of time corresponding to the maximum value of the first unimodal distribution, time corresponding to the maximum slope of a first cumulative distribution function, time corresponding to the maximum value of the second unimodal distribution, or time corresponding to the maximum slope of a second cumulative distribution function as a myocardial activity parameter.

For example, upon approximating a second target inverse time waveform that is a waveform obtained by reversing the time axis of a second target time waveform that is a waveform of a time section of a T wave of a target heart by a second approximate inverse time waveform that is a waveform obtained by a difference or a weighted difference between a third cumulative distribution function that is a cumulative distribution function of a third unimodal distribution and a fourth cumulative distribution function that is a cumulative distribution function of a fourth unimodal distribution, or by a second approximate inverse time waveform that is a waveform obtained by adding a level value to a difference or a weighted difference between a third cumulative distribution function that is a cumulative distribution function of a third unimodal distribution and a fourth cumulative distribution function that is a cumulative distribution function of a fourth unimodal distribution, the myocardial activity information parameter acquisition unit 132 of the analysis unit 130 (analyzer) of the signal analysis device 1 acquires at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a myocardial activity parameter that is a parameter indicating activity of a myocardium of the target heart.

For example, in a case where both the third unimodal distribution and the fourth unimodal distribution are Gaussian distributions, the myocardial activity information parameter acquisition unit 132 needs to acquire at least one of an average value of the third unimodal distribution, a standard deviation or variance of the third unimodal distribution, an average value of the fourth unimodal distribution, or a standard deviation or variance of the fourth unimodal distribution as a myocardial activity parameter. Furthermore, for example, regardless of whether the third unimodal distribution and the fourth unimodal distribution are Gaussian distributions, the myocardial activity information parameter acquisition unit 132 needs to acquire at least one of time corresponding to the maximum value of a distribution of the third unimodal distribution, time corresponding to the maximum slope of a third cumulative distribution function, time corresponding to the maximum value of the fourth unimodal distribution, or time corresponding to the maximum slope of a fourth cumulative distribution function as a myocardial activity parameter. Note that, in a case of acquiring time as a myocardial activity parameter, the myocardial activity information parameter acquisition unit 132 acquires time (value of x in the above-described examples) instead of information indicating time in the reverse direction (value of x' in the above-described examples) even in a case of approximating a waveform obtained by reversing the time axis.

For example, assuming a cumulative distribution function of a third unimodal distribution as a third cumulative distribution function, a cumulative distribution function of a fourth unimodal distribution as a fourth cumulative distribution function, a function obtained by subtracting the third cumulative distribution function from 1 as a third inverse cumulative distribution function, and a function obtained by subtracting the fourth cumulative distribution function from 1 as a fourth inverse cumulative distribution function, upon approximating a second target time waveform that is a waveform in a time section of a T wave of a target heart by a second approximate time waveform that is a waveform obtained by a difference or a weighted difference between the third inverse cumulative distribution function and the fourth inverse cumulative distribution function, or by a second approximate time waveform that is a waveform obtained by adding a level value to a difference or a weighted difference between the third inverse cumulative distribution function and the fourth inverse cumulative distribution function, the myocardial activity information parameter acquisition unit 132 of the analysis unit 130 (analyzer) of the signal analysis device 1 acquires at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a myocardial activity parameter that is a parameter indicating activity of a myocardium of the target heart.

For example, in a case where both the third unimodal distribution and the fourth unimodal distribution are Gaussian distributions, the myocardial activity information parameter acquisition unit 132 needs to acquire at least one of an average value of the third unimodal distribution, a standard deviation or variance of the third unimodal distribution, an average value of the fourth unimodal distribution, or a standard deviation or variance of the fourth unimodal distribution as a myocardial activity parameter. Furthermore, for example, regardless of whether the third unimodal distribution and the fourth unimodal distribution are Gaussian distributions, the myocardial activity information parameter acquisition unit 132 needs to acquire at least one of time corresponding to the maximum value of the third unimodal distribution, time corresponding to the maximum slope of the third cumulative distribution function, time corresponding to the maximum value of the fourth unimodal distribution, or time corresponding to the maximum slope of the fourth cumulative distribution function as a myocardial activity parameter.

Similarly, for example, in a case where the myocardial activity information parameter acquisition unit 132 of the analysis unit 130 of the signal analysis device 1 further approximates a residual time waveform that is a time waveform of a difference between a first target time waveform and a first approximate time waveform, a residual time waveform that is a time waveform of a difference between a second target time waveform and a second approximate time waveform, or a residual time waveform that is a time waveform of a difference between a second target time waveform and a second approximate time waveform that is a waveform obtained by reversing the time axis of a second approximate inverse time waveform by a fifth cumulative distribution function that is a cumulative distribution function of a fifth unimodal distribution or by an approximate residual time waveform that is a time waveform obtained by multiplying a fifth cumulative distribution function by weight, at least one of at least some parameters of parameters identifying the fifth unimodal distribution or at least some parameters of parameters identifying the fifth cumulative distribution function is also acquired as a myocardial activity parameter that is a parameter indicating activity of a myocardium of a target heart.

For example, in a case where the fifth unimodal distribution is a Gaussian distribution, the myocardial activity information parameter acquisition unit 132 needs to acquire at least one of an average value of the fifth unimodal distribution and a standard deviation or variance of the fifth unimodal distribution as a myocardial activity parameter. Furthermore, for example, regardless of whether the fifth unimodal distribution is a Gaussian distribution, the myocardial activity information parameter acquisition unit 132 needs to acquire at least one of time corresponding to the maximum value of the fifth unimodal distribution or time corresponding to the maximum slope of the fifth cumulative distribution function as a myocardial activity parameter.

Furthermore, for example, in a case where the myocardial activity information parameter acquisition unit 132 of the analysis unit 130 of the signal analysis device 1 further approximates a residual time waveform by an approximate residual time waveform that is a waveform obtained by a difference or a weighted difference between a fifth cumulative distribution function that is a cumulative distribution function of a fifth unimodal distribution and a sixth cumulative distribution function that is a cumulative distribution function of a sixth unimodal distribution, at least one of at least some parameters of parameters identifying the fifth unimodal distribution, at least some parameters of parameters identifying the fifth cumulative distribution function, at least some parameters of parameters identifying the sixth unimodal distribution, and at least some parameters of parameters identifying the sixth cumulative distribution function is acquired as a myocardial activity parameter that is a parameter indicating activity of a myocardium of a target heart.

For example, in a case where both the fifth unimodal distribution and the sixth unimodal distribution are Gaussian distributions, the myocardial activity information parameter acquisition unit 132 needs to acquire at least one of an average value of the fifth unimodal distribution, a standard deviation or variance of the fifth unimodal distribution, an average value of the sixth unimodal distribution, or a standard deviation or variance of the sixth unimodal distribution as a myocardial activity parameter. Furthermore, for example, regardless of whether the fifth unimodal distribution and the sixth unimodal distribution are Gaussian distributions, the myocardial activity information parameter acquisition unit 132 needs to acquire at least one of time corresponding to the maximum value of the fifth unimodal distribution, time corresponding to the maximum slope of the fifth cumulative distribution function, time corresponding to the maximum value of the sixth unimodal distribution, or time corresponding to the maximum slope of the sixth cumulative distribution function as a myocardial activity parameter.

[Acquisition of Myocardial Activity Parameter by Calculation of Parameters Obtained by Fitting]

The characteristics of activity of a myocardium of a target heart may clearly appear not only in parameters obtained by the fitting described above but also in a value obtained by calculation of the parameters obtained by the fitting. Therefore, a value obtained by calculation of the parameters obtained by the fitting may be acquired by the signal analysis device 1 as a myocardial activity parameter. A parameter obtained by the fitting is at least one of a parameter identifying a unimodal distribution or a parameter identifying a cumulative distribution function, weight, or a level value. The parameter identifying a unimodal distribution or the parameter identifying a cumulative distribution function is at least one of an average or a standard deviation (or variance) in a case where the unimodal distribution is a Gaussian distribution. Regardless of whether the unimodal distribution is a Gaussian distribution, time corresponding to the maximum value of the unimodal distribution is an example of a parameter identifying a unimodal distribution, and time corresponding to the maximum slope of a cumulative distribution function of the unimodal distribution is an example of a parameter identifying a cumulative distribution function.

For example, the myocardial activity information parameter acquisition unit 132 of the analysis unit 130 of the signal analysis device 1 may acquire a parameter indicating activity of a myocardium of a target heart (parameter different from each parameter described above) by calculating a myocardial activity parameter obtained by the above-described fitting for a waveform in a time section of an R wave included in a waveform for one cycle indicating a cardiac cycle of the target heart (that is, parameter indicating activity of the myocardium of the target heart in the time section of the R wave) and a myocardial activity parameter obtained by the above-described fitting for a waveform in a time section of a T wave included in the waveform for one cycle (that is, parameter indicating activity of the myocardium of the target heart in the time section of the T wave).

Furthermore, for example, the myocardial activity information parameter acquisition unit 132 of the analysis unit 130 of the signal analysis device 1 may acquire a parameter indicating activity of a myocardium of a target heart (parameter different from each parameter described above) by calculating a parameter indicating activity of an inner layer of the myocardium of the target heart obtained by the fitting described above for a waveform in a time section of an R wave included in a waveform for one cycle indicating a cardiac cycle of the target heart and a parameter indicating activity of an outer layer of the myocardium of the target heart obtained by the fitting.

Furthermore, for example, the myocardial activity information parameter acquisition unit 132 of the analysis unit 130 of the signal analysis device 1 may acquire a parameter indicating activity of a myocardium of a target heart (parameter different from each parameter described above) by calculating a parameter indicating activity of an inner layer of the myocardium of the target heart obtained by the fitting described above for a waveform in a time section of a T wave included in a waveform for one cycle indicating a cardiac cycle of the target heart and a parameter indicating activity of an outer layer of the myocardium of the target heart obtained by the fitting.

Hereinafter, examples will be described in which, in a case where all of first to fourth unimodal distributions are Gaussian distributions, values obtained by calculation of averages obtained by fitting are used as myocardial activity parameters. In a case where first to fourth unimodal distributions are not Gaussian distributions, "average" in the following examples needs to be read as "time at which the value is maximum in the unimodal distribution", that is, "time corresponding to the maximum value of the unimodal distribution", "time at which the slope is maximum in the cumulative distribution function", that is, "time corresponding to the maximum slope of the cumulative distribution function", or the like.

(1) Parameter Representing Time From Depolarization to Switching to Repolarization

It is known that sudden death may occur due to arrhythmia in a case where time from depolarization of an inner layer or an outer layer of a myocardium to switching to repolarization of the inner layer or the outer layer of the myocardium is extremely short or extremely long. That is, shortening or prolongation of the time from depolarization of a myocardium to switching to repolarization may indicate that some pathological state may have occurred in the myocardium. Therefore, the signal analysis device 1 preferably acquires the time from depolarization of an inner layer or an outer layer of a myocardium to switching to repolarization of the inner layer or the outer layer of the myocardium as a myocardial activity parameter, and specifically, preferably acquires at least one of the following four parameters (1A) to (1D) as a myocardial activity parameter.

(1A) Parameter Representing Time From Depolarization of Inner Layer of Myocardium to Switching to Repolarization of Inner Layer of Myocardium

The myocardial activity information parameter acquisition unit 132 of the analysis unit 130 of the signal analysis device 1 may acquire, as a myocardial activity parameter, a difference between an average of a first unimodal distribution obtained by the fitting described above for a first target time waveform that is a waveform in a time section of an R wave included in a waveform for one cycle indicating a cardiac cycle of a target heart and an average of a third unimodal distribution obtained by the fitting described above for a second target time waveform that is a waveform in a time section of a T wave included in the waveform for one cycle. For example, assuming the average of the first unimodal distribution is $\mu_a$ and the average of the third unimodal distribution is $\mu_c$ as in the example described above, the myocardial activity information parameter acquisition unit 132 may acquire $|\mu_a - \mu_c|$ as a myocardial activity parameter. Note that, since $\mu_c$ is temporally later than $\mu_a$, the myocardial activity information parameter acquisition unit 132 may acquire $\mu_c - \mu_a$ as a

myocardial activity parameter. This myocardial activity parameter is a parameter representing time from depolarization of an inner layer of a myocardium to switching to repolarization of the inner layer of the myocardium.

(1B) Parameter Representing Time From Depolarization of Outer Layer of Myocardium to Switching to Repolarization of Outer Layer of Myocardium

The myocardial activity information parameter acquisition unit 132 of the analysis unit 130 of the signal analysis device 1 may acquire, as a myocardial activity parameter, a difference between an average of a second unimodal distribution obtained by the fitting described above for a first target time waveform that is a waveform in a time section of an R wave included in a waveform for one cycle indicating a cardiac cycle of a target heart and an average of a fourth unimodal distribution obtained by the fitting described above for a second target time waveform that is a waveform in a time section of a T wave included in the waveform for one cycle. For example, assuming the average of the second unimodal distribution is $\mu_b$ and the average of the fourth unimodal distribution is $\mu_d$ as in the example described above, the myocardial activity information parameter acquisition unit 132 may acquire $|\mu_b - \mu_d|$ as a myocardial activity parameter. Note that, since $\mu_d$ is temporally later than $\mu_b$, the myocardial activity information parameter acquisition unit 132 may acquire $\mu_d - \mu_b$ as a myocardial activity parameter. This myocardial activity parameter is a parameter representing time from depolarization of an outer layer of a myocardium to switching to repolarization of the outer layer of the myocardium.

(1C) Parameter Representing Time From Depolarization of Outer Layer of Myocardium to Switching to Repolarization of Inner Layer of Myocardium

The myocardial activity information parameter acquisition unit 132 of the analysis unit 130 of the signal analysis device 1 may acquire, as a myocardial activity parameter, a difference between an average of a second unimodal distribution obtained by the fitting described above for a first target time waveform that is a waveform in a time section of an R wave included in a waveform for one cycle indicating a cardiac cycle of a target heart and an average of a third unimodal distribution obtained by the fitting described above for a second target time waveform that is a waveform in a time section of a T wave included in the waveform for one cycle. For example, assuming the average of the second unimodal distribution is $\mu_b$ and the average of the third unimodal distribution is $\mu_c$ as in the example described above, the myocardial activity information parameter acquisition unit 132 may acquire $|\mu_b - \mu_c|$ as a myocardial activity parameter. Note that, since $\mu_c$ is temporally later than $\mu_b$, the myocardial activity information parameter acquisition unit 132 may acquire $\mu_c - \mu_b$ as a myocardial activity parameter. This myocardial activity parameter is a parameter representing time from depolarization of an outer layer of a myocardium to switching to repolarization of an inner layer of the myocardium.

(1D) Parameter Representing Time From Depolarization of Inner Layer of Myocardium to Switching to Repolarization of Outer Layer of Myocardium

The myocardial activity information parameter acquisition unit 132 of the analysis unit 130 of the signal analysis device 1 may acquire, as a myocardial activity parameter, a difference between an average of a first unimodal distribution obtained by the fitting described above for a first target time waveform that is a waveform in a time section of an R wave included in a waveform for one cycle indicating a cardiac cycle of a target heart and an average of a fourth unimodal distribution obtained by the fitting described above for a second target time waveform that is a waveform in a time section of a T wave included in the waveform for one cycle. For example, assuming the average of the first unimodal distribution is $\mu_a$ and the average of the fourth unimodal distribution is $\mu_d$ as in the example described above, the myocardial activity information parameter acquisition unit 132 may acquire $|\mu_a - \mu_d|$ as a myocardial activity parameter. Note that, since $\mu_d$ is temporally later than $\mu_a$, the myocardial activity information parameter acquisition unit 132 may acquire $\mu_d - \mu_a$ as a myocardial activity parameter. This myocardial activity parameter is a parameter representing time from depolarization of an inner layer of a myocardium to switching to repolarization of an outer layer of the myocardium.

(2) Parameter Representing Time Difference And Order Between Inner Layer Activity And Outer Layer Activity in Depolarization

In a case where a time difference between activity of an inner layer and activity of an outer layer in depolarization is longer than normal, there is a possibility that a disorder such as delay or block occurs in conduction of excitation of a myocardium, or a place where excitation starts or the order in which excitation is transmitted are different from a normal pattern, and in particular, it is suggested that there is a disorder in a stimulation conduction system of the myocardium, ischemic condition of the myocardium, and the presence of premature contraction. Therefore, the signal analysis device 1 preferably acquires, as a myocardial activity parameter, a parameter representing a time difference and the order between activity of an inner layer and activity of an outer layer in depolarization. Specifically, the myocardial activity information parameter acquisition unit 132 of the analysis unit 130 of the signal analysis device 1 may acquire, as a myocardial activity parameter, a difference between an average of a first unimodal distribution and an average of a second unimodal distribution obtained by the fitting described above for a first target time waveform that is a waveform in a time section of an R wave included in a waveform for one cycle indicating a cardiac cycle of a target heart. For example, assuming the average of the first unimodal distribution is $\mu_a$ and the average of the second unimodal distribution is $\mu_b$ as in the example described above, the myocardial activity information parameter acquisition unit 132 may acquire $\mu_a$ - $\mu_b$ or $\mu_b$ - $\mu_a$ as a myocardial activity parameter.

(3) Parameter Representing Time Difference And Order Between Inner Layer Activity And Outer Layer Activity in Repolarization

In a case where a time difference between activity of an inner layer and activity of an outer layer in repolarization is prolonged or shortened, some abnormality may have occurred in a myocardium. Therefore, the signal analysis device 1 preferably acquires, as a myocardial activity parameter, a parameter representing a time difference and the order between activity of an inner layer and activity of an outer layer in repolarization. Specifically, the myocardial activity information parameter acquisition unit 132 of the analysis unit 130 of the signal analysis device 1 may acquire, as a myocardial activity parameter, a difference between an average of a third unimodal distribution and an average of a fourth unimodal distribution obtained by the fitting described above for a second target time waveform that is a waveform in a time section of a T wave included in a waveform for one cycle indicating a cardiac cycle of a target heart. For example, assuming the average of the third unimodal distribution is $\mu_c$ and the average of the fourth unimodal distribution is $\mu_d$ as in the example described above, the myocardial activity information parameter acquisition unit 132 may acquire $\mu_c$ - $\mu_d$ or $\mu_d$ - $\mu_c$ as a myocardial activity parameter.

(Correction of Myocardial Activity Parameter)

Note that a myocardial activity parameter related to the width in the time direction among the myocardial activity parameters described above acquired by the myocardial activity information parameter acquisition unit 132 is characterized in that influence of fluctuation due to a heartbeat rate and influence of individual differences of age, gender, and the like are recognized, similarly to a parameter related to the width in the time direction (for example, QT interval) among conventional parameters indicating activity of a heart. For a conventional parameter related to the width in the time direction, it is known that correction for reducing the influence of fluctuation due to a heartbeat rate and the influence of individual differences is performed, and a value after the correction is used as a parameter evaluating activity of a heart. Therefore, a myocardial activity parameter acquired by the myocardial activity information parameter acquisition unit 132 may also be corrected such that the influence of fluctuation due to a heartbeat rate and the influence of individual differences are reduced, and a value after the correction may be used as a parameter evaluating activity of a myocardium. For example, for the fluctuation due to a heartbeat rate, a myocardial activity parameter acquired by the fitting described above may be corrected by linear or nonlinear calculation on the basis of a time interval between adjacent R spine and R spine (hereinafter referred to as an "RR interval"), and a value after the correction may be used as a myocardial activity parameter. Furthermore, for the individual differences, a relationship between an RR interval and a myocardial activity parameter acquired by the fitting described above may be obtained from electrocardiogram data of each individual and the myocardial activity parameter described above is corrected, and a value after the correction may be used as a myocardial activity parameter. This correction may be performed by another device after the signal analysis device 1 outputs a myocardial activity parameter acquired by fitting, or may be performed by the signal analysis device 1. In a case where the signal analysis device 1 corrects a myocardial activity parameter, for example, the myocardial activity information parameter acquisition unit 132 needs to correct the myocardial activity parameter acquired by the fitting described above and output a value after the correction as a myocardial activity parameter. Note that, in a case where the correction is performed in the signal analysis device 1, a myocardial activity parameter acquired by the fitting described above is an intermediate parameter acquired in the device as a result, but is still a parameter representing activity of a myocardium as in a case of being output outside the device.

(Modifications)

Note that an electrocardiogram is preferably an electrocardiogram in which the induction is close to an electromotive force vector. The electrocardiogram in which the induction is close to the electromotive force vector is preferably, for example, an electrocardiogram capable of acquiring three-dimensional information of a cardiac potential such as II induction, V4 induction, and V5 induction. Since an information amount increases as the number of channels of the electrocardiogram increases, a larger number of channels of the electrocardiogram is desirably provided. That is, the signal analysis device 1 may analyze a waveform of each of the channels of the multi-channel electrocardiogram and obtain a myocardial activity parameter that is an analysis result for each of the channels.

Note that the signal analysis device 1 may be implemented by using a plurality of information processing devices communicably connected to each other via a network. In this case, the functional units included in the signal analysis device 1 may be implemented in a distributed manner in the plurality of information processing devices.

Note that the electrocardiogram acquisition unit 110 is an example of a biological information acquisition unit (biological information acquisitor).

Note that, all or some of the functions of the signal analysis device 1 may be implemented by using hardware such as an application specific integrated circuit (ASIC), a programmable logic device (PLD), or a field programmable gate array (FPGA). The program may be recorded on a computer-readable recording medium. The "computer-readable recording medium" refers to, for example, a portable medium such as a flexible disk, a magneto-optical disc, a read-only memory (ROM), or a compact disc read-only memory (CD-ROM), or a storage device such as a hard disk built in a computer system. The program may be transmitted via an electrical communication line.

Although the embodiments of the present disclosure have been described in detail with reference to the drawings, specific configurations are not limited to the described embodiments; the invention is defined by the appended claims.

**Reference** Signs List

| | |
|---|---|
| 1 | Signal analysis device |
| 11 | Control unit |
| 12 | Input unit |
| 13 | Communication unit |
| 14 | Storage unit |
| 15 | Output unit |
| 91 | Processor |
| 92 | Memory |
| 110 | Electrocardiogram acquisition unit |
| 120 | Fitting information acquisition unit |
| 130 | Analysis unit |
| 131 | Fitting unit |
| 132 | Myocardial activity information parameter acquisition unit |
| 140 | Recording unit |

**Claims**

1. A signal analysis device (1) comprising:

   a biological information acquisition unit (110) configured to acquire a waveform of a time section of an R wave included in a waveform for one cycle indicating a cardiac cycle of a heart of an analysis target as a target time waveform; and
   an analysis unit (130), upon approximating the target time waveform by an approximate time waveform that is a time waveform obtained by a difference or a weighted difference between a first cumulative distribution function that is a cumulative distribution function of a first unimodal distribution and a second cumulative distribution function that is a cumulative distribution function of a second unimodal distribution, or upon approximating the target time waveform by an approximate time waveform that is a time waveform obtained by adding a level value to a difference or a weighted difference between a first cumulative distribution function that is a cumulative distribution function of a first unimodal distribution and a second cumulative distribution function that is a cumulative distribution function of a second unimodal distribution, configured to acquire at least one of at least some parameters of parameters identifying the first unimodal distribution, at least some parameters of parameters identifying the first cumulative distribution function, at least some parameters of parameters identifying the second unimodal distribution, or at least some parameters of parameters identifying the second cumulative distribution function as a parameter indicating activity of a myocardium of the heart.

2. A signal analysis device (1) comprising:

   a biological information acquisition unit (110) configured to acquire a waveform of a time section of a T wave included in a waveform for one cycle indicating a cardiac cycle of a heart of an analysis target as a target time waveform; and

an analysis unit (130), assuming a waveform obtained by reversing a time axis of the target time waveform as a target inverse time waveform,

upon approximating the target inverse time waveform by a waveform obtained by a difference or a weighted difference between a third cumulative distribution function that is a cumulative distribution function of a third unimodal distribution and a fourth cumulative distribution function that is a cumulative distribution function of a fourth unimodal distribution, or upon approximating the target inverse time waveform by a waveform obtained by adding a level value to a difference or a weighted difference between a third cumulative distribution function that is a cumulative distribution function of a third unimodal distribution and a fourth cumulative distribution function that is a cumulative distribution function of a fourth unimodal distribution, configured to acquire at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

3. A signal analysis device (1) comprising:

a biological information acquisition unit (110) configured to acquire a waveform of a time section of a T wave included in a waveform for one cycle indicating a cardiac cycle of a heart of an analysis target as a target time waveform; and

an analysis unit (130), assuming a cumulative distribution function of a third unimodal distribution as a third cumulative distribution function, a cumulative distribution function of a fourth unimodal distribution as a fourth cumulative distribution function, a function obtained by subtracting the third cumulative distribution function from 1 as a third inverse cumulative distribution function, and a function obtained by subtracting the fourth cumulative distribution function from 1 as a fourth inverse cumulative distribution function,

upon approximating the target time waveform by an approximate time waveform that is a time waveform obtained by a difference or a weighted difference between the third inverse cumulative distribution function and the fourth inverse cumulative distribution function, or upon approximating the target time waveform by an approximate time waveform that is a time waveform obtained by adding a level value to a difference or a weighted difference between the third inverse cumulative distribution function and the fourth inverse cumulative distribution function, configured to acquire at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

4. The signal analysis device (1) according to claim 1 or 3,

wherein the analysis unit(130) is configured to,
upon approximating a residual time waveform that is a time waveform obtained by a difference between the target time waveform and the approximate time waveform by a fifth cumulative distribution function that is a cumulative distribution function of a fifth unimodal distribution or by an approximate residual time waveform that is a time waveform obtained by multiplying the fifth cumulative distribution function by weight, aquire at least one of at least some parameters of parameters identifying the fifth unimodal distribution or at least some parameters of parameters identifying the fifth cumulative distribution function as a parameter indicating activity of a myocardium of the heart,

or

upon approximating a residual time waveform that is a time waveform obtained by a difference between the target time waveform and the approximate time waveform by an approximate residual time waveform that is a time waveform obtained by a difference or a weighted difference between a fifth cumulative distribution function that is a cumulative distribution function of a fifth unimodal distribution and a sixth cumulative distribution function that is a cumulative distribution function of a sixth unimodal distribution, acquire at least one of at least some parameters of parameters identifying the fifth unimodal distribution, at least some parameters of parameters identifying the fifth cumulative distribution function, at least some parameters of parameters identifying the sixth unimodal distribution, or at least some parameters of parameters identifying the sixth cumulative distribution function as a parameter indicating activity of a myocardium of the heart.

5. The signal analysis device (1) according to claim 1,

wherein the analysis unit(130) is configured to
assume, in a case where the target time waveform is an R wave, a potential at a starting end of the target time waveform as the level value.

6. The signal analysis device (1) according to claim 2 or 3,

wherein the analysis unit (130) is configured to
assume, in a case where the target time waveform is a T wave, a potential at an end of the target time waveform as the level value.

7. The signal analysis device (1) according to any one of claims 1 to 6,
wherein each unimodal distribution is a Gaussian distribution.

8. A signal analysis method comprising:

a biological information acquisition step (S101) for acquiring a waveform of a time section of an R wave included in a waveform for one cycle indicating a cardiac cycle of a heart of an analysis target as a target time waveform; and
an analysis step (S103, S104) for, upon approximating the target time waveform by an approximate time waveform that is a time waveform obtained by a difference or a weighted difference between a first cumulative distribution function that is a cumulative distribution function of a first unimodal distribution and a second cumulative distribution function that is a cumulative distribution function of a second unimodal distribution, or upon approximating the target time waveform by an approximate time waveform that is a time waveform obtained by adding a level value to a difference or a weighted difference between a first cumulative distribution function that is a cumulative distribution function of a first unimodal distribution and a second cumulative distribution function that is a cumulative distribution function of a second unimodal distribution, acquiring at least one of at least some parameters of parameters identifying the first unimodal distribution, at least some parameters of parameters identifying the first cumulative distribution function, at least some parameters of parameters identifying the second unimodal distribution, or at least some parameters of parameters identifying the second cumulative distribution function as a parameter indicating activity of a myocardium of the heart.

9. A signal analysis method comprising:

a biological information acquisition step (S101) for acquiring a waveform of a time section of a T wave included in a waveform for one cycle indicating a cardiac cycle of a heart of an analysis target as a target time waveform; and
an analysis step (S103, S104) for, assuming a waveform obtained by reversing a time axis of the target time waveform as a target inverse time waveform,
upon approximating the target inverse time waveform by a waveform obtained by a difference or a weighted difference between a third cumulative distribution function that is a cumulative distribution function of a third unimodal distribution and a fourth cumulative distribution function that is a cumulative distribution function of a fourth unimodal distribution, or upon approximating the target inverse time waveform by a waveform obtained by adding a level value to a difference or a weighted difference between a third cumulative distribution function that is a cumulative distribution function of a third unimodal distribution and a fourth cumulative distribution function that is a cumulative distribution function of a fourth unimodal distribution, acquiring at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

10. A signal analysis method comprising:

a biological information acquisition step (S101) for acquiring a waveform of a time section of a T wave included in a waveform for one cycle indicating a cardiac cycle of a heart of an analysis target as a target time waveform; and
an analysis step (S103, S104) for, assuming a cumulative distribution function of a third unimodal distribution as a third cumulative distribution function, a cumulative distribution function of a fourth unimodal distribution as a fourth cumulative distribution function, a function obtained by subtracting the third cumulative distribution function from 1 as a third inverse cumulative distribution function, and a function obtained by subtracting the fourth cumulative distribution function from 1 as a fourth inverse cumulative distribution function,
upon approximating the target time waveform by an approximate time waveform that is a time waveform obtained by a difference or a weighted difference between the third inverse cumulative distribution function and the fourth inverse cumulative distribution function, or upon approximating the target time waveform by an approximate time waveform that is a time waveform obtained by adding a level value to a difference or a weighted difference between the third inverse cumulative distribution function and the fourth inverse cumulative distribution function, acquiring at least one of at least some parameters of parameters identifying the third unimodal distribution, at least some parameters of parameters identifying the third cumulative distribution function, at least some parameters of parameters identifying the fourth unimodal distribution, or at least some parameters of parameters identifying the fourth cumulative distribution function as a parameter indicating activity of a myocardium of the heart.

11. A computer program comprising instructions which, when the computer program is executed by a computer, cause the computer to carry out the signal analysis method according to any one of claims 8 to 10.

**Patentansprüche**

1. Signalanalysevorrichtung (1), umfassend:

   eine Einheit (110) zur Erfassung biologischer Informationen, die so konfiguriert ist, dass sie eine Wellenform eines Zeitabschnitts einer R-Welle, die in einer Wellenform für einen Zyklus enthalten ist, der einen Herzzyklus eines Herzens eines Analyseziels anzeigt, als Zielzeitwellenform erfasst; und
   eine Analyseeinheit (130), die so konfiguriert ist, dass sie bei Annäherung der Zielzeitwellenform durch eine annähernde Zeitwellenform, die eine Zeitwellenform ist, die durch eine Differenz oder eine gewichtete Differenz zwischen einer ersten kumulativen Verteilungsfunktion, die eine kumulative Verteilungsfunktion einer ersten unimodalen Verteilung ist, und einer zweiten kumulativen Verteilungsfunktion, die eine kumulative Verteilungsfunktion einer zweiten unimodalen Verteilung ist, erhalten wird, oder bei Annäherung der Zielzeitwellenform durch eine angenäherte Zeitwellenform, die eine Zeitwellenform ist, die durch Addition eines Pegelwerts zu einer Differenz oder einer gewichteten Differenz zwischen einer ersten kumulativen Verteilungsfunktion, die eine kumulative Verteilungsfunktion einer ersten unimodalen Verteilung ist, und einer zweiten kumulativen Verteilungsfunktion, die eine kumulative Verteilungsfunktion einer zweiten unimodalen Verteilung ist, erhalten wird, mindestens einen von mindestens einigen Parametern von Parametern, die die erste unimodale Verteilung identifizieren, mindestens einige Parameter von Parametern, die die erste kumulative Verteilungsfunktion identifizieren, mindestens einige Parameter von Parametern, die die zweite unimodale Verteilung identifizieren, oder mindestens einige Parameter von Parametern, die die zweite kumulative Verteilungsfunktion identifizieren, als einen Parameter erfasst, der die Aktivität eines Myokards des Herzens anzeigt.

2. Signalanalysevorrichtung (1), umfassend:

   eine Einheit (110) zur Erfassung biologischer Informationen, die so konfiguriert ist, dass sie eine Wellenform eines Zeitabschnitts einer T-Welle, die in einer Wellenform für einen Zyklus enthalten ist, der einen Herzzyklus eines Herzens eines Analyseziels anzeigt, als Zielzeitwellenform erfasst; und
   eine Analyseeinheit (130), die eine Wellenform, die durch Umkehren einer Zeitachse der Zielzeitwellenform erhalten wird, als eine inverse Zielzeitwellenform annimmt und so konfiguriert ist, dass sie
   bei Annäherung der inversen Zielzeitwellenform durch eine Wellenform, die durch eine Differenz oder eine gewichtete Differenz zwischen einer dritten kumulativen Verteilungsfunktion, die eine kumulative Verteilungsfunktion einer dritten unimodalen Verteilung ist, und einer vierten kumulativen Verteilungsfunktion, die eine kumulative Verteilungsfunktion einer vierten unimodalen Verteilung ist, erhalten wird, oder bei Annäherung der inversen Zielzeitwellenform durch eine Wellenform, die durch Addition eines Pegelwerts zu einer Differenz oder einer gewichteten Differenz zwischen einer dritten kumulativen Verteilungsfunktion, die eine kumulative Verteilungsfunktion einer dritten unimodalen Verteilung ist, und einer vierten kumulativen Verteilungsfunktion, die eine kumulative Verteilungsfunktion einer vierten unimodalen Verteilung ist, erhalten wird, mindestens einen von mindestens einigen Parametern von Parametern, die die dritte unimodale Verteilung identifizieren, mindestens einige Parameter von Parametern, die die dritte kumulative Verteilungsfunktion identifizieren, mindestens einige Parameter von Parametern, die die vierte unimodale Verteilung identifizieren, oder mindestens einige Parameter von Parametern, die die vierte kumulative Verteilungsfunktion identifizieren, als einen Parameter erfasst, der die Aktivität eines Myokards des Herzens anzeigt.

3. Signalanalysevorrichtung (1), umfassend:

eine Einheit (110) zur Erfassung biologischer Informationen, die so konfiguriert ist, dass sie eine Wellenform eines Zeitabschnitts einer T-Welle, die in einer Wellenform für einen Zyklus enthalten ist, der einen Herzzyklus eines Herzens eines Analyseziels anzeigt, als Zielzeitwellenform erfasst; und
eine Analyseeinheit (130), die eine kumulative Verteilungsfunktion einer dritten unimodalen Verteilung als eine dritte kumulative Verteilungsfunktion, eine kumulative Verteilungsfunktion einer vierten unimodalen Verteilung als eine vierte kumulative Verteilungsfunktion, eine Funktion, die durch Subtraktion der dritten kumulativen Verteilungsfunktion von 1 erhalten wird, als eine dritte inverse kumulative Verteilungsfunktion und eine Funktion, die durch Subtraktion der vierten kumulativen Verteilungsfunktion von 1 erhalten wird, als eine vierte inverse kumulative Verteilungsfunktion annimmt und so konfiguriert ist, dass sie
bei Annäherung der Zielzeitwellenform durch eine annähernde Zeitwellenform, die eine Zeitwellenform ist, die durch eine Differenz oder eine gewichtete Differenz zwischen der dritten inversen kumulativen Verteilungsfunktion und der vierten inversen kumulativen Verteilungsfunktion erhalten wird, oder bei Annäherung der Zielzeitwellenform durch eine angenäherte Zeitwellenform, die eine Zeitwellenform ist, die durch Addition eines Pegelwerts zu einer Differenz oder einer gewichteten Differenz zwischen der dritten inversen kumulativen Verteilungsfunktion und der vierten inversen kumulativen Verteilungsfunktion erhalten wird, mindestens einen von mindestens einigen Parametern von Parametern, die die dritte unimodale Verteilung identifizieren, mindestens einige Parameter von Parametern, die die dritte kumulative Verteilungsfunktion identifizieren, mindestens einige Parameter von Parametern, die die vierte unimodale Verteilung identifizieren, oder mindestens einige Parameter von Parametern, die die vierte kumulative Verteilungsfunktion identifizieren, als einen Parameter erfasst, der die Aktivität eines Myokards des Herzens anzeigt.

4. Signalanalysevorrichtung (1) nach Anspruch 1 oder 3, wobei die Analyseeinheit (130) so konfiguriert ist, dass sie

bei Annäherung einer Restzeitwellenform, die eine Zeitwellenform ist, die durch eine Differenz zwischen der Zielzeitwellenform und der angenäherten Zeitwellenform erhalten wird, durch eine fünfte kumulative Verteilungsfunktion, die eine kumulative Verteilungsfunktion einer fünften unimodalen Verteilung ist, oder durch eine angenäherte Restzeitwellenform, die eine Zeitwellenform ist, die durch Multiplikation der fünften kumulativen Verteilungsfunktion mit einem Gewicht erhalten wird, mindestens einen von mindestens einigen Parametern von Parametern, die die fünfte unimodale Verteilung identifizieren, oder mindestens einige Parameter von Parametern, die die fünfte kumulative Verteilungsfunktion identifizieren, als einen Parameter erfasst, der die Aktivität eines Myokards des Herzens anzeigt,
oder
bei Annäherung einer Restzeitwellenform, die eine Zeitwellenform ist, die durch eine Differenz zwischen der Zielzeitwellenform und der angenäherten Zeitwellenform erhalten wird, durch eine angenäherte Restzeitwellenform, die eine Zeitwellenform ist, die durch eine Differenz oder eine gewichtete Differenz zwischen einer fünften kumulativen Verteilungsfunktion, die eine kumulative Verteilungsfunktion einer fünften unimodalen Verteilung ist, und einer sechsten kumulativen Verteilungsfunktion, die eine kumulative Verteilungsfunktion einer sechsten unimodalen Verteilung ist, erhalten wird, mindestens einen von mindestens einigen Parametern von Parametern, die die fünfte unimodale Verteilung identifizieren, mindestens einige Parameter von Parametern, die die fünfte kumulative Verteilungsfunktion identifizieren, mindestens einige Parameter von Parametern, die die sechste unimodale Verteilung identifizieren, oder mindestens einige Parameter von Parametern, die die sechste kumulative Verteilungsfunktion identifizieren, als einen Parameter erfasst, der die Aktivität eines Myokards des Herzens anzeigt.

5. Signalanalysevorrichtung (1) nach Anspruch 1,

wobei die Analyseeinheit (130) so konfiguriert ist, dass sie
in einem Fall, in dem die Zielzeitwellenform eine R-Welle ist, ein Potenzial an einem Anfangsende der Zielzeitwellenform als den Pegelwert annimmt.

6. Signalanalysevorrichtung (1) nach Anspruch 2 oder 3,

wobei die Analyseeinheit (130) so konfiguriert ist, dass sie
in einem Fall, in dem die Zielzeitwellenform eine T-Welle ist, ein Potenzial an einem Ende der Zielzeitwellenform als den Pegelwert annimmt.

7.  Signalanalysevorrichtung (1) nach einem der Ansprüche 1 bis 6,
    wobei jede unimodale Verteilung eine Gaußsche Verteilung ist.

8.  Signalanalyseverfahren, umfassend:

    einen Schritt (S101) zur Erfassung biologischer Informationen, um eine Wellenform eines Zeitabschnitts einer R-Welle zu erfassen, die in einer Wellenform für einen Zyklus enthalten ist, der einen Herzzyklus eines Herzens eines Analyseziels anzeigt, als eine Zielzeitwellenform; und
    einen Analyseschritt (S103, S104), um bei Annäherung der Zielzeitwellenform durch eine annähernde Zeitwellenform, die eine Zeitwellenform ist, die durch eine Differenz oder eine gewichtete Differenz zwischen einer ersten kumulativen Verteilungsfunktion, die eine kumulative Verteilungsfunktion einer ersten unimodalen Verteilung ist, und einer zweiten kumulativen Verteilungsfunktion, die eine kumulative Verteilungsfunktion einer zweiten unimodalen Verteilung ist, erhalten wird, oder bei Annäherung der Zielzeitwellenform durch eine angenäherte Zeitwellenform, die eine Zeitwellenform ist, die durch Addition eines Pegelwerts zu einer Differenz oder einer gewichteten Differenz zwischen einer ersten kumulativen Verteilungsfunktion, die eine kumulative Verteilungsfunktion einer ersten unimodalen Verteilung ist, und einer zweiten kumulativen Verteilungsfunktion, die eine kumulative Verteilungsfunktion einer zweiten unimodalen Verteilung ist, erhalten wird, mindestens einen von mindestens einigen Parametern von Parametern, die die erste unimodale Verteilung identifizieren, mindestens einige Parameter von Parametern, die die erste kumulative Verteilungsfunktion identifizieren, mindestens einige Parameter von Parametern, die die zweite unimodale Verteilung identifizieren, oder mindestens einige Parameter von Parametern, die die zweite kumulative Verteilungsfunktion identifizieren, als einen Parameter zu erfassen, der die Aktivität eines Myokards des Herzens anzeigt.

9.  Signalanalyseverfahren, umfassend:

    einen Schritt (S101) zur Erfassung biologischer Informationen, um eine Wellenform eines Zeitabschnitts einer T-Welle zu erfassen, die in einer Wellenform für einen Zyklus enthalten ist, der einen Herzzyklus eines Herzens eines Analyseziels anzeigt, als eine Zielzeitwellenform; und
    einen Analyseschritt (S103, S104), um eine Wellenform, die durch Umkehren einer Zeitachse der Zielzeitwellenform erhalten wird, als eine inverse Zielzeitwellenform anzunehmen und
    bei Annäherung der inversen Zielzeitwellenform durch eine Wellenform, die durch eine Differenz oder eine gewichtete Differenz zwischen einer dritten kumulativen Verteilungsfunktion, die eine kumulative Verteilungsfunktion einer dritten unimodalen Verteilung ist, und einer vierten kumulativen Verteilungsfunktion, die eine kumulative Verteilungsfunktion einer vierten unimodalen Verteilung ist, erhalten wird, oder bei Annäherung der inversen Zielzeitwellenform durch eine Wellenform, die durch Addition eines Pegelwerts zu einer Differenz oder einer gewichteten Differenz zwischen einer dritten kumulativen Verteilungsfunktion, die eine kumulative Verteilungsfunktion einer dritten unimodalen Verteilung ist, und einer vierten kumulativen Verteilungsfunktion, die eine kumulative Verteilungsfunktion einer vierten unimodalen Verteilung ist, erhalten wird, mindestens einen von mindestens einigen Parametern von Parametern, die die dritte unimodale Verteilung identifizieren, mindestens einige Parameter von Parametern, die die dritte kumulative Verteilungsfunktion identifizieren, mindestens einige Parameter von Parametern, die die vierte unimodale Verteilung identifizieren, oder mindestens einige Parameter von Parametern, die die vierte kumulative Verteilungsfunktion identifizieren, als einen Parameter zu erfassen, der die Aktivität eines Myokards des Herzens anzeigt.

10. Signalanalyseverfahren, umfassend:

    einen Schritt (S101) zur Erfassung biologischer Informationen, um eine Wellenform eines Zeitabschnitts einer T-Welle zu erfassen, die in einer Wellenform für einen Zyklus enthalten ist, der einen Herzzyklus eines Herzens eines Analyseziels anzeigt, als eine Zielzeitwellenform; und
    einen Analyseschritt (S103, S104), um eine kumulative Verteilungsfunktion einer dritten unimodalen Verteilung als eine dritte kumulative Verteilungsfunktion, eine kumulative Verteilungsfunktion einer vierten unimodalen Verteilung als eine vierte kumulative Verteilungsfunktion, eine Funktion, die durch Subtraktion der dritten kumulativen Verteilungsfunktion von 1 erhalten wird, als eine dritte inverse kumulative Verteilungsfunktion und eine Funktion, die durch Subtraktion der vierten kumulativen Verteilungsfunktion von 1 erhalten wird, als eine vierte inverse kumulative Verteilungsfunktion anzunehmen und
    bei Annäherung der Zielzeitwellenform durch eine annähernde Zeitwellenform, die eine Zeitwellenform ist, die durch eine Differenz oder eine gewichtete Differenz zwischen der dritten inversen kumulativen Verteilungsfunktion und der vierten inversen kumulativen Verteilungsfunktion erhalten wird, oder bei Annäherung der Zielzeitwellenform durch eine angenäherte Zeitwellenform, die eine Zeitwellenform ist, die durch Addition eines Pegelwerts zu einer Differenz oder einer gewichteten Differenz zwischen der dritten inversen kumulativen Verteilungsfunktion und der vierten inversen kumulativen Verteilungsfunktion erhalten wird, mindestens einen von mindestens einigen Parametern von Parametern, die die dritte unimodale Verteilung identifizieren, mindestens einige Parameter von Parametern, die die dritte kumulative Verteilungsfunktion identifizieren, mindestens einige Parameter von Parametern, die die vierte unimodale Verteilung identifizieren, oder mindestens einige Parameter von Parametern, die die vierte kumulative Verteilungsfunktion identifizieren, als einen Parameter zu erfassen, der die Aktivität eines Myokards des Herzens anzeigt.

11. Computerprogramm, das Anweisungen umfasst, die, wenn das Computerprogramm von einem Computer ausgeführt wird, den Computer dazu veranlassen, das Signalanalyseverfahren nach einem der Ansprüche 8 bis 10 durchzuführen.

**Revendications**

1. Dispositif d'analyse de signal (1) comportant :

une unité d'acquisition d'informations biologiques (110) configurée pour acquérir une forme d'onde d'une section temporelle d'une onde R incluse dans une forme d'onde pour un cycle indiquant un cycle cardiaque d'un cœur d'une cible d'analyse en tant que forme d'onde temporelle cible ; et
une unité d'analyse (130), lors de l'approximation de la forme d'onde temporelle cible par une forme d'onde temporelle approximative qui est une forme d'onde temporelle obtenue par une différence ou une différence pondérée entre une première fonction de distribution cumulative qui est une fonction de distribution cumulative d'une première distribution unimodale et une deuxième fonction de distribution cumulative qui est une fonction de distribution cumulative d'une deuxième distribution unimodale, ou lors de l'approximation de la forme d'onde temporelle cible par une forme d'onde temporelle approximative qui est une forme d'onde temporelle obtenue par ajout d'une valeur de niveau à une différence ou à une différence pondérée entre une première fonction de distribution cumulative qui est une fonction de distribution cumulative d'une première distribution unimodale et une deuxième fonction de distribution cumulative qui est une fonction de distribution cumulative d'une deuxième distribution unimodale, configurée pour acquérir au moins un parmi au moins certains paramètres de paramètres identifiant la première distribution unimodale, au moins certains paramètres de paramètres identifiant la première fonction de distribution cumulative, au moins certains paramètres de paramètres identifiant la deuxième distribution unimodale ou au moins certains paramètres de paramètres identifiant la deuxième fonction de distribution cumulative en tant que paramètre indiquant l'activité d'un myocarde du cœur.

2. Dispositif d'analyse de signal (1) comportant :

une unité d'acquisition d'informations biologiques (110) configurée pour acquérir une forme d'onde d'une section temporelle d'une onde T incluse dans une forme d'onde pour un cycle indiquant un cycle cardiaque d'un cœur d'une cible d'analyse en tant que forme d'onde temporelle cible ; et
une unité d'analyse (130), en supposant qu'une forme d'onde obtenue par inversion d'un axe temporel de la forme d'onde temporelle cible est une forme d'onde temporelle inverse cible,
lors de l'approximation de la forme d'onde temporelle inverse cible par une forme d'onde obtenue par une différence ou une différence pondérée entre une troisième fonction de distribution cumulative qui est une fonction de distribution cumulative d'une troisième distribution unimodale et une quatrième fonction de distribution cumulative qui est une fonction de distribution cumulative d'une quatrième distribution unimodale, ou lors de l'approximation de la forme d'onde temporelle inverse cible par une forme d'onde obtenue par ajout d'une valeur de niveau à une différence ou à une différence pondérée entre une troisième fonction de distribution cumulative qui est une fonction de distribution cumulative d'une troisième distribution unimodale et une quatrième fonction de distribution cumulative qui est une fonction de distribution cumulative d'une quatrième distribution unimodale, configurée pour acquérir au moins un parmi au moins certains paramètres de paramètres identifiant la troisième distribution unimodale, au moins certains paramètres de paramètres identifiant la troisième fonction de distribution cumulative, au moins certains paramètres de paramètres identifiant la quatrième distribution unimodale ou au moins certains paramètres de paramètres identifiant la quatrième fonction de distribution cumulative en tant que paramètre indiquant l'activité d'un myocarde du cœur.

3. Dispositif d'analyse de signal (1) comportant :

une unité d'acquisition d'informations biologiques (110) configurée pour acquérir une forme d'onde d'une section temporelle d'une onde T incluse dans une forme d'onde pour un cycle indiquant un cycle cardiaque d'un cœur d'une cible d'analyse en tant que forme d'onde temporelle cible ; et

une unité d'analyse (130), en supposant qu'une fonction de distribution cumulative d'une troisième distribution unimodale est une troisième fonction de distribution cumulative, qu'une fonction de distribution cumulative d'une quatrième distribution unimodale est une quatrième fonction de distribution cumulative, qu'une fonction obtenue par soustraction de la troisième fonction de distribution cumulative à 1 est une troisième fonction de distribution cumulative inverse et qu'une fonction obtenue par soustraction de la quatrième fonction de distribution cumulative à 1 est une quatrième fonction de distribution cumulative inverse,

lors de l'approximation de la forme d'onde temporelle cible par une forme d'onde temporelle approximative qui est une forme d'onde temporelle obtenue par une différence ou une différence pondérée entre la troisième fonction de distribution cumulative inverse et la quatrième fonction de distribution cumulative inverse, ou lors de l'approximation de la forme d'onde temporelle cible par une forme d'onde temporelle approximative qui est une forme d'onde temporelle obtenue par ajout d'une valeur de niveau à une différence ou à une différence pondérée entre la troisième fonction de distribution cumulative inverse et la quatrième fonction de distribution cumulative inverse, configurée pour acquérir au moins un parmi au moins certains paramètres de paramètres identifiant la troisième distribution unimodale, au moins certains paramètres de paramètres identifiant la troisième fonction de distribution cumulative, au moins certains paramètres de paramètres identifiant la quatrième distribution unimodale ou au moins certains paramètres de paramètres identifiant la quatrième fonction de distribution cumulative en tant que paramètre indiquant l'activité d'un myocarde du cœur.

4. Dispositif d'analyse de signal (1) selon la revendication 1 ou 3, dans lequel l'unité d'analyse (130) est configurée pour,

lors de l'approximation d'une forme d'onde temporelle résiduelle qui est une forme d'onde temporelle obtenue par une différence entre la forme d'onde temporelle cible et la forme d'onde temporelle approximative par une cinquième fonction de distribution cumulative qui est une fonction de distribution cumulative d'une cinquième distribution unimodale ou par une forme d'onde temporelle résiduelle approximative qui est une forme d'onde temporelle obtenue par multiplication de la cinquième fonction de distribution cumulative par une pondération, acquérir au moins un parmi au moins certains paramètres de paramètres identifiant la cinquième distribution unimodale ou au moins certains paramètres de paramètres identifiant la cinquième fonction de distribution cumulative en tant que paramètre indiquant l'activité d'un myocarde du cœur,

ou

lors de l'approximation d'une forme d'onde temporelle résiduelle qui est une forme d'onde temporelle obtenue par une différence entre la forme d'onde temporelle cible et la forme d'onde temporelle approximative par une forme d'onde temporelle résiduelle approximative qui est une forme d'onde temporelle obtenue par une différence ou une différence pondérée entre une cinquième fonction de distribution cumulative qui est une fonction de distribution cumulative d'une cinquième distribution unimodale et une sixième fonction de distribution cumulative qui est une fonction de distribution cumulative d'une sixième distribution unimodale, acquérir au moins un parmi au moins certains paramètres de paramètres identifiant la cinquième distribution unimodale, au moins certains paramètres de paramètres identifiant la cinquième fonction de distribution cumulative, au moins certains paramètres de paramètres identifiant la sixième distribution unimodale ou au moins certains paramètres de paramètres identifiant la sixième fonction de distribution cumulative en tant que paramètre indiquant l'activité d'un myocarde du cœur.

5. Dispositif d'analyse de signal (1) selon la revendication 1,

dans lequel l'unité d'analyse (130) est configurée pour
supposer, dans un cas où la forme d'onde temporelle cible est une onde R, qu'un potentiel à une extrémité de départ de la forme d'onde temporelle cible est la valeur de niveau.

6. Dispositif d'analyse de signal (1) selon la revendication 2 ou 3,

dans lequel l'unité d'analyse (130) est configurée pour
supposer, dans un cas où la forme d'onde temporelle cible est une onde T, qu'un potentiel à une extrémité de la forme d'onde temporelle cible est la valeur de niveau.

7. Dispositif d'analyse de signal (1) selon l'une quelconque des revendications 1 à 6,
dans lequel chaque distribution unimodale est une distribution gaussienne.

8.  Procédé d'analyse de signal comportant :

    une étape d'acquisition d'informations biologiques (S101) pour acquérir une forme d'onde d'une section temporelle d'une onde R incluse dans une forme d'onde pour un cycle indiquant un cycle cardiaque d'un cœur d'une cible d'analyse en tant que forme d'onde temporelle cible ; et
    une étape d'analyse (S103, S104) pour, lors de l'approximation de la forme d'onde temporelle cible par une forme d'onde temporelle approximative qui est une forme d'onde temporelle obtenue par une différence ou une différence pondérée entre une première fonction de distribution cumulative qui est une fonction de distribution cumulative d'une première distribution unimodale et une deuxième fonction de distribution cumulative qui est une fonction de distribution cumulative d'une deuxième distribution unimodale, ou lors de l'approximation de la forme d'onde temporelle cible par une forme d'onde temporelle approximative qui est une forme d'onde temporelle obtenue par ajout d'une valeur de niveau à une différence ou à une différence pondérée entre une première fonction de distribution cumulative qui est une fonction de distribution cumulative d'une première distribution unimodale et une deuxième fonction de distribution cumulative qui est une fonction de distribution cumulative d'une deuxième distribution unimodale, acquérir au moins un parmi au moins certains paramètres de paramètres identifiant la première distribution unimodale, au moins certains paramètres de paramètres identifiant la première fonction de distribution cumulative, au moins certains paramètres de paramètres identifiant la deuxième distribution unimodale ou au moins certains paramètres de paramètres identifiant la deuxième fonction de distribution cumulative en tant que paramètre indiquant l'activité d'un myocarde du cœur.

9.  Procédé d'analyse de signal comportant :

    une étape d'acquisition d'informations biologiques (S101) pour acquérir une forme d'onde d'une section temporelle d'une onde T incluse dans une forme d'onde pour un cycle indiquant un cycle cardiaque d'un cœur d'une cible d'analyse en tant que forme d'onde temporelle cible ; et
    une étape d'analyse (S103, S104) pour, en supposant qu'une forme d'onde obtenue par inversion d'un axe temporel de la forme d'onde temporelle cible est une forme d'onde temporelle inverse cible,
    lors de l'approximation de la forme d'onde temporelle inverse cible par une forme d'onde obtenue par une différence ou une différence pondérée entre une troisième fonction de distribution cumulative qui est une fonction de distribution cumulative d'une troisième distribution unimodale et une quatrième fonction de distribution cumulative qui est une fonction de distribution cumulative d'une quatrième distribution unimodale, ou lors de l'approximation de la forme d'onde temporelle inverse cible par une forme d'onde obtenue par ajout d'une valeur de niveau à une différence ou à une différence pondérée entre une troisième fonction de distribution cumulative qui est une fonction de distribution cumulative d'une troisième distribution unimodale et une quatrième fonction de distribution cumulative qui est une fonction de distribution cumulative d'une quatrième distribution unimodale, acquérir au moins un parmi au moins certains paramètres de paramètres identifiant la troisième distribution unimodale, au moins certains paramètres de paramètres identifiant la troisième fonction de distribution cumulative, au moins certains paramètres de paramètres identifiant la quatrième distribution unimodale ou au moins certains paramètres de paramètres identifiant la quatrième fonction de distribution cumulative en tant que paramètre indiquant l'activité d'un myocarde du cœur.

10. Procédé d'analyse de signal comportant :

    une étape d'acquisition d'informations biologiques (S101) pour acquérir une forme d'onde d'une section temporelle d'une onde T incluse dans une forme d'onde pour un cycle indiquant un cycle cardiaque d'un cœur d'une cible d'analyse en tant que forme d'onde temporelle cible ; et
    une étape d'analyse (S103, S104) pour, en supposant qu'une fonction de distribution cumulative d'une troisième distribution unimodale est une troisième fonction de distribution cumulative, qu'une fonction de distribution cumulative d'une quatrième distribution unimodale est une quatrième fonction de distribution cumulative, qu'une fonction obtenue par soustraction de la troisième fonction de distribution cumulative à 1 est une troisième fonction de distribution cumulative inverse et qu'une fonction obtenue par soustraction de la quatrième fonction de distribution cumulative à 1 est une quatrième fonction de distribution cumulative inverse,
    lors de l'approximation de la forme d'onde temporelle cible par une forme d'onde temporelle approximative qui est une forme d'onde temporelle obtenue par une différence ou une différence pondérée entre la troisième fonction de distribution cumulative inverse et la quatrième fonction de distribution cumulative inverse, ou lors de l'approximation de la forme d'onde temporelle cible par une forme d'onde temporelle approximative qui est une forme d'onde temporelle obtenue par ajout d'une valeur de niveau à une différence ou à une différence pondérée entre la troisième fonction de distribution cumulative inverse et la quatrième fonction de distribution cumulative inverse, acquérir au moins un parmi au moins certains paramètres de paramètres identifiant la troisième distribution unimodale, au moins certains paramètres de paramètres identifiant la troisième fonction de distribution cumulative, au moins certains paramètres de paramètres identifiant la quatrième distribution unimodale ou au moins certains paramètres de paramètres identifiant la quatrième fonction de distribution cumulative en tant que paramètre indiquant l'activité d'un myocarde du cœur.

11. Programme informatique comportant des instructions qui, lorsque le programme informatique est exécuté par un ordinateur, amènent l'ordinateur à réaliser le procédé d'analyse de signal selon l'une quelconque des revendications 8 à 10.

## FIG. 1

FIG. 2

FIG. 3

FIG. 4

FIG. 5

$k_a f_a(x) + \beta_R$

$k_b f_b(x) + \beta_R$

$k_a f_a(x) - k_b f_b(x) + \beta_R$

$\mu_a$ $\mu_b$

$k_a$ $k_b$

$\beta_R$

$\sigma_a$

$\sigma_b$

x

## FIG. 6

$k_c f'_c(x) + \beta_T$

$k_d f'_d(x) + \beta_T$

$\mu_d$ $\mu_c$

$k_c f'_c(x) - k_d f'_d(x) + \beta_T$

$k_c$

$k_d$

$\beta_T$

$\sigma_d$

$\sigma_c$

x

## FIG. 7

FIG. 8

FIG. 9

50

START

ACQUIRE ELECTROCARDIOGRAM ⌐S101

ACQUIRE DISTRIBUTION SHAPE
DESIGNATION INFORMATION ⌐S102

PERFORM FITTING USING
CUMULATIVE DISTRIBUTION FUNCTIONS ⌐S103

ACQUIRE MYOCARDIAL ACTIVITY PARAMETERS
ON BASIS OF FITTING RESULTS ⌐S104

OUTPUT RESULTS ⌐S105

END

# FIG. 10

FIG. 11

FIG. 12

FIG. 13

FIG. 14

: CARDIAC POTENTIAL ON BODY SURFACE (MEASUREMENT VALUE)

VENTRICULAR PREMATURE CONTRACTION

FIG. 15

MYOCARDIAL OUTER LAYER ACTIVITY
APPROXIMATION FUNCTION

MYOCARDIAL INNER LAYER ACTIVITY
APPROXIMATION FUNCTION

FIG. 16

CARDIAC POTENTIAL ON BODY SURFACE (MEASUREMENT VALUE)

DIFFERENCE BETWEEN CUMULATIVE DISTRIBUTION FUNCTIONS

FIG. 17

FIG. 18

FIG. 19

FIG. 20

FIG. 21

FIG. 22

FIG. 23

FIG. 24

FIG. 25

FIG. 26

FIG. 27

FIG. 28

EP 4 238 498 B1

FIG. 29

FIG. 30

FIG. 31

EP 4 238 498 B1

FIG. 32

FIG. 33

FIG. 34

FIG. 35

FIG. 36

EP 4 238 498 B1

FIG. 37

FIG. 38

FIG. 39

FIG. 40

FIG. 41

FIG. 42

FIG. 43

FIG. 44

FIG. 45

FIG. 46

FIG. 47

FIG. 48

EP 4 238 498 B1

**FIG. 49**

FIG. 50

FIG. 51

FIG. 52

FIG. 53

FIG. 54

EP 4 238 498 B1

FIG. 55

FIG. 56

FIG. 57

● : POINTS TO BE DETERMINED

FIG. 58

FIG. 59

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021000209 W
- JP 2021033138 W

- US 2012302903 A

**Non-patent literature cited in the description**

- **HIROSHI TANAKA**. On the Inverse Solution of Electrocardiology. *Medical Electronics and Biological Engineering*, 1985, vol. 23 (3), 147-158

- **YOSHIFUMI TANAKA**. Understand from the constitution, electrocardiogram waveform, reading activity potential of myocardium. Gakken Medical Shujunsha Co., Ltd., 2012